(19) Europäisches Patentamt
European Patent Office
Office européen des brevets

(11) Veröffentlichungsnummer : **0 288 431 B1**

# (12) EUROPÄISCHE PATENTSCHRIFT

(45) Veröffentlichungstag der Patentschrift :
**19.08.92 Patentblatt 92/34**

(51) Int. Cl.$^5$ : **C07D 487/04,** A61K 31/505, // (C07D487/04, 249:00, 239:00)

(21) Anmeldenummer : **88810212.6**

(22) Anmeldetag : **30.03.88**

(54) **3H-1,2,3-Triazolo[4,5-d]pyrimidine.**

(30) Priorität : **07.04.87 CH 1333/87**

(43) Veröffentlichungstag der Anmeldung :
**26.10.88 Patentblatt 88/43**

(45) Bekanntmachung des Hinweises auf die Patenterteilung :
**19.08.92 Patentblatt 92/34**

(84) Benannte Vertragsstaaten :
**AT BE CH DE ES FR GB GR IT LI LU NL SE**

(56) Entgegenhaltungen :
**DE-A- 2 162 096**
**DE-A- 2 714 254**
**LIEBIGS "Annalen der Chemie", Nr, 10, Oktober 1984, Seiten 1848-1859**

(73) Patentinhaber : **CIBA-GEIGY AG**
**Klybeckstrasse 141**
**CH-4002 Basel (CH)**

(72) Erfinder : **Meier, René**
**Rickenbacherstrasse 13**
**CH-4463 Buus (CH)**

EP 0 288 431 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 3H-1,2,3-Triazolo[4,5-d]pyrimidinderivate, insbesondere substituierte 3-Benzyl-3H-1,2,3-triazolo-[4,5-d]pyrimidine der allgemeinen Formel

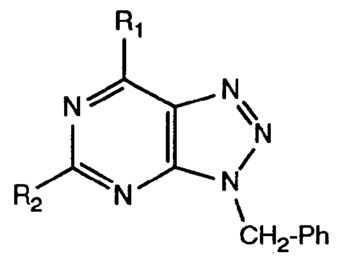

(I),

worin Ph einen durch Halogen mit einer Atomnummer bis und mit 35, $C_1$-$C_7$-Alkyl, Trifluormethyl und/oder Cyano substituierten Phenylrest bedeutet, $R_1$ Amino, N-Mono-$C_1$-$C_7$-alkylamino, N,N-Di-$C_1$-$C_7$-alkylamino, worin die beiden N-Alkylgruppen gleich oder verschieden sein können, N-($C_1$-$C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$alkyl-amino, N-Mono($C_3$-$C_8$-Cycloalkyl-$C_1$-$C_7$-alkyl)amino, N,N-Di($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkylamino darstellt und $R_2$ für Wasserstoff, $C_1$-$C_7$-Alkyl, Amino, N-Mono-$C_1$-$C_7$-alkyl-amino, N,N-Di-$C_1$-$C_7$-alkylamino, worin die beiden N-Alkylgruppen gleich oder verschieden sein können, N-($C_1$-$C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$-alkyl-amino, N-Mono($C_3$-$C_8$-Cycloalkyl-$C_1$-$C_7$-alkyl)amino, N,N-Di ($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkyl-amino steht, in freier Form oder in Salzform, mit der Massgabe, dass in einer Verbindung der Formel I in freier Form, worin $R_1$ N,N-Di-$C_1$-$C_6$-alkylamino, worin die beiden N-$C_1$-$C_6$-Alkylgruppen gleich oder verschieden sind, N-Mono-$C_1$-$C_6$-alkylamino oder Amino bedeutet, $R_2$ von Wasserstoff und von $C_1$-$C_6$-Alkyl verschieden ist, wenn Ph durch Halogen mit einer Atomnummer bis und mit 35 oder durch Trifluormethyl einfach substituiertes Phenyl darstellt, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist, Verfahren zur Herstellung solcher Verbindungen, diese enthaltende pharmazeutische Präparate und ihre Verwendung als Arzneimittelwirkstoffe.

Der Phenylrest Ph kann bis und mit 3, vorzugsweise 1 oder 2, der genannten Substituenten aufweisen, wobei im Falle von mehrfacher Substitution die Substituenten gleich oder verschieden sein können. Die Substituenten sind jeweils vorzugsweise in einer ortho-Stellung oder in zweiter Linie in einer meta-Stellung gebunden, können jedoch auch in para-Stellung gebunden sein. Als Beispiele seien genannt: o- und m-, ferner p-Halogenphenyl, 2,6-Dihalogenphenyl, ferner

2,3- und 2,5-Dihalogenphenyl sowie 2,3,6- und 2,5,6-Trihalogenphenyl, o-Niederalkylphenyl, ferner m- und p-Niederalkylphenyl, m-Trifluormethylphenyl, ferner o- und p-Trifluormethylphenyl, und o- und m-Cyanophenyl, ferner p-Cyanophenyl.

Der Phenylrest Ph kann beispielsweise bis und mit 3, vorzugsweise 1 oder 2, Halogensubstituenten aufweisen, die jeweils vorzugsweise in einer ortho-Stellung oder in zweiter Linie in einer meta-Stellung gebunden sind. Als Beispiele seien genannt: o-Halogenphenyl und 2,6-Dihalogenphenyl, ferner 2,3- und 2,5-Dihalogenphenyl sowie 2,3,6- und 2,5,6-Trihalogenphenyl.

Vor- und nachstehend sind unter mit "Nieder" bezeichneten organischen Gruppen und Verbindungen, sofern nicht abweichend definiert, solche zu verstehen, die bis und mit 7, insbesondere bis und mit 4, Kohlenstoffatome (C-Atome) enthalten.

Halogen ist beispielsweise Halogen mit einer Atomnummer bis und mit 35, wie Fluor, Chlor oder in zweiter Linie Brom.

$C_1$-$C_7$-Alkyl ist beispielsweise $C_1$-$C_4$-Alkyl, wie Methyl, Aethyl, n-Propyl, Isopropyl oder n-Butyl, ferner Sekundärbutyl, Isobutyl oder Tertiärbutyl, kann aber auch eine $C_5$-$C_7$-Alkylgruppe, d.h. eine Pentyl-, Hexyl- oder Heptylgruppe, sein.

M-Mono-$C_1$-$C_7$-alkylamino ist insbesondere N-$C_1$-$C_4$-Alkylamino, wie N-Methyl- oder N-Aethylamino.

N,N-Di-$C_1$-$C_7$-alkylamino ist insbesondere N,N-Di-$C_1$-$C_4$-alkylamino, wobei jeweils die beiden N-Alkylgruppen gleich oder verschieden sein können, wie N,N-Dimethyl-, N,N-Diäthyl-, N,N-Diisopropyl- oder N-Butyl-N-methylamino.

N-$C_1$-$C_7$-Alkoxy-$C_1$-$C_7$alkyl)amino ist beispielsweise N-($C_1$-$C_4$-Alkoxy-, wie Methoxy- oder Aethoxy-, $C_1$-$C_7$-alkyl)-, insbesondere N-($C_1$-$C_4$-Alkoxy-, wie Methoxy- oder Aethoxy-, $C_1$-$C_4$-alkyl)amino, wie N-(Methoxymethyl)amino oder N-(1-Methoxyäthyl)amino.

N-(Hydroxy-$C_1$-$C_7$alkyl)amino ist insbesondere N-(Hydroxy-$C_1$-$C_4$-alkyl)amino, wie N-(Hydroxymethyl)amino oder N-(1-Hydroxyäthyl)amino.

N-(Hydroxy-$C_1$-$C_7$-alkyl)-N-$C_1$-$C_7$-alkyl-amino ist insbesondere N-(Hydroxy-$C_1$-$C_4$-alkyl)-N-$C_1$-$C_4$-alkyl-amino, wie N-(1-Hydroxyäthyl)-N-methyl-amino oder N-Hydroxymethyl)-N-äthyl-amino.

N-$C_3$-$C_8$-Cycloalkylamino ist insbesondere N-$C_3$-$C_6$-Cycloalkylamino, wie N-Cyclopropyl- oder N-Cyclohexylamino.

N,N-Di-$C_3$-$C_8$-cycloalkylamino, ist insbesondere N,N-Di-$C_3$-$C_6$-Cycloalkylamino, wobei jeweils die beiden N-Cycloalkylgruppen gleich oder verschieden sein können, wie N,N-Dicyclohexyl- oder N-Cyclohexyl-N-cyclopropylamino.

N-$C_3$-$C_8$-Cycloalkyl-N-$C_1$-$C_7$-alkyl-amino ist insbesondere N-$C_3$-$C_6$-Cycloalkyl-N-$C_1$-$C_4$-alkyl-amino, wie N-Cyclopropyl-N-methyl-oder N-Cyclohexyl-N-äthyl-amino.

N-($C_3$-$C_8$-Cycloalkyl-$C_1$-$C_7$-alkyl)amino, ist insbesondere N-($C_3$-$C_6$-Cycloalkyl-$C_1$-$C_4$-alkyl)amino, wie N-(Cyclopropylmethyl)amino oder N-(1-Cyclohexyläthyl)amino.

N,N-Di($C_3$-$C_8$-cycloalkyl-$C_1$-$C_7$alkyl)amino, ist insbesondere N,N-Di($C_3$-$C_6$-cycloalkyl-$C_1$-$C_4$-alkyl)amino, wobei jeweils die beiden N-(Cycloalkylalkyl)gruppen gleich oder verschieden sein können, wie N,N-Di(cyclopropylmethyl)amino oder N-(Cyclopropylmethyl)-N-(1-cyclohexyläthyl)-amino.

N-($C_3$-$C_8$-Cycloalkyl-$C_1$-$C_7$-alkyl)-N-$C_1$-$C_7$-alkyl-amino ist insbesondere N-($C_3$-$C_6$-Cycloalkyl-$C_1$-$C_4$-alkyl)-N-$C_1$-$C_4$-alkyl-amino, wie N-(Cyclopropylmethyl)-N-äthyl-amino oder N-(1-Cyclohexyläthyl)-N-methylamino.

$C_2$-$C_5$-Alkanoyl ist beispielsweise Acetyl Propionyl, Butyryl, Isobutyryl oder Pivaloyl.

N-$C_2$-$C_5$-Alkanoylamino ist z.B. N-Acetyl-, N-Propionyl-, N-Butyryl- oder N-Pivaloylamino.

N-$C_2$-$C_5$-Alkanoyl-N-$C_1$-$C_7$-alkyl-amino ist insbesondere N-$C_2$-$C_5$-Alkanoyl-N-$C_1$-$C_4$-alkyl-amino, wie N-Acetyl-N-propyl-amino oder N-Butyryl-N-metlylamino.

Die Verbindungen I können über ihre basischen Zentren Salze bilden. Salze von Verbindungen I sind daher insbesondere entsprechende Säureadditionssalze, vorzugsweise pharmazeutisch verwendbare Säureadditionssalze. Diese werden beispielsweise mit starken anorganischen Protonensäuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet.

Umfasst sind ferner für pharmazeutische Verwendungen ungeeignete Salze, da diese beispielsweise für die Isolierung bzw. Reinigung freier Verbindungen I sowie deren pharmazeutisch verwendbarer Salze verwendet werden können.

Die neuen Verbindungen I und ihre pharmazeutisch verwendbaren Salze besitzen wertvolle pharmakologische Eigenschaften, insbesondere eine ausgeprägte antikonvulsive Wirksamkeit, die sich beispielsweise an der Maus anhand eines deutlichen Metrazol-Antagonismus im Dosisbereich ab etwa 10 mg/kg p.o. sowie an Maus und Ratte anhand einer ausgeprägten Schutzwirkung gegen durch Elektroschock ausgelöste Konvulsionen im Dosisbereich ab etwa 3 mg/kg p.o. zeigen lässt.

Die Verbindungen I und ihre pharmazeutisch verwendbaren Salze sind dementsprechend vorzüglich geeignet zur Behandlung von Konvulsionen verschiedenartiger Genese, beispielsweise zur Behandlung der Epilepsie. Sie können dementsprechend als antikonvulsive, beispielsweise antiepileptische, Arzneimittelwirkstoffe verwendet werden. Dabei kann auch die gewerbsmässige Herrichtung der Wirksubstanzen eingeschlossen sein. Aus Liebigs Ann. Chem. (1984, 1848) ist 7-Amino-3-(4-chlorbenzyl)-5-methyl-3H-1,2,3-triazolo[4,5-d]pyrimidin bereits bekannt, wobei eine pharmakologische Wirksamkeit für diese Verbindung nicht erwähnt wird. Durch die in Anspruch 1 enthaltene Massgabe wird diese Verbindung aus dem Umfang der vorliegenden Erfindung ausgekammert, ebenso wie ein Reihe von substituierten 3H-1,2,3-Triazolo[4,5-d]pyrimidinen mit Anti-Psoriasis-Wirksamkeit, die in der deutschen Offenlegungsschrift Nr. 27 14 254 generisch offenbart sind.

Die Erfindung betrifft insbesondere eine Verbindung der Formel I, worin, unter Berücksichtigung der obigen Massgabe, Ph einen durch mindestens ein Halogenatom mit einer Atomnummer bis und mit 35 substituierten Phenylrest bedeutet, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

Die Erfindung betrifft insbesondere eine Verbindung der Formel I, worin, unter Berücksichtigung der obigen Massgabe, Ph 2-, 3- oder 4-Halogenphenyl, 2,3-, 2,5- oder 2,6-Dihalogenphenyl, 2-, 3- oder 4-$C_1$-$C_4$-Alkylphenyl, 2-, 3- oder 4-Trifluormethylphenyl oder 2-, 3- oder 4-Cyanophenyl bedeutet, wobei Halogen jeweils Halogen mit einer Atomnummer bis und mit 35 sein kann, $R_1$ Amino, N-Mono-$C_1$-$C_4$-alkylamino, N,N-Di-$C_1$-$C_4$-alkylamino, N-$C_3$-$C_6$-Cycloalkylamino oder N-$C_2$-$C_5$-Alkanoylamino darstellt und $R_2$ für Wasserstoff, $C_1$-$C_4$-Alkyl, Amino, N-Mono-$C_1$-$C_4$-alkylamino, N,N-Di-$C_1$-$C_4$-alkylamino, N-$C_3$-$C_6$-Cycloalkylamino oder N-$C_2$-$C_5$-Alkanoyl amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist.

Die Erfindung betrifft insbesondere eine Verbindung der Formel I, worin, unter Berücksichtigung der obigen Massgabe, Ph 2-Halogenphenyl, das zusätzlich in 3-, 5- oder 6-Position durch Halogen substituiert sein kann, bedeutet, wobei Halogen jeweils Halogen mit einer Atomnummer bis und mit 35 sein kann, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

Die Erfindung betrifft insbesondere eine Verbindung der Formel I, worin, unter Berücksichtigung der obigen Massgabe, Ph 2- oder 3-Fluorphenyl, 2-Chlorphenyl, 2,6-Difluor phenyl oder 2-$C_1$-$C_4$-Alkylphenyl bedeutet, $R_1$ N-Mono-$C_1$-$C_4$-alkylamino, N,N-Di-$C_1$-$C_4$-alkylamino oder N-$C_3$-$C_6$-Cycloalkylamino darstellt und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino ist und $R_2$ Wasserstoff oder Methyl ist oder worin Ph o-Fluorphenyl oder o-Chlorphenyl ist, $R_1$ N,N-Dimethylamino ist und $R_2$ Wasserstoff ist oder worin Ph m-Fluorphenyl oder o-Chlorphenyl ist, $R_1$ N-Monomethylamino ist und $R_2$ Wasserstoff ist.

Die Erfindung betrifft insbesondere eine Verbindung der Formel I, worin, unter Berücksichtigung der obigen Massgabe, Ph 2-Fluorphenyl, das zusätzlich in 5- oder 6-Position durch Halogen mit einer Atomnummer bis und mit 35 substituiert sein kann, bedeutet, $R_1$ Amino, N-Mono-$C_1$-$C_4$-alkylamino, N,N-Di-$C_1$-$C_4$-alkylamino oder N-$C_3$-$C_6$-Cycloalkylamino darstellt und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

Die Erfindung betrifft insbesondere eine Verbindung der Formel I, worin, unter Berücksichtigung der obigen Massgabe, Ph 2-Fluor- oder 2,6-Difluorphenyl bedeutet, $R_1$ Amino, N-Mono-$C_1$-$C_4$-alkylamino oder N,N-Di-$C_1$-$C_4$-alkylamino darstellt und $R_2$ für Wasserstoff oder $C_1$-$C_4$-Alkyl steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

Die Erfindung betrifft insbesondere eine Verbindung der Formel I, worin, unter Berücksichtigung der obigen Massgabe, Ph 2-Fluorphenyl bedeutet, $R_1$ N-Mono-$C_1$-$C_4$-alkylamino oder N,N-Di-$C_1$-$C_4$-alkylamino darstellt und $R_2$ für Wasserstoff oder Amino steht, in freier Form oder in Salzform, sowie eine Verbindung der Formel I in freier Form, worin entweder Ph o-Fluorphenyl ist, $R_1$ N-Monomethylamino ist und $R_2$ Wasserstoff ist oder worin Ph o-Fluorphenyl ist, $R_1$ N,N-Dimethylamino ist und $R_2$ Wasserstoff ist.

Die Erfindung betrifft namentlich die in den Beispielen genannten neuen Verbindungen der Formel I und ihre Salze.

Ebenfalls Gegenstand der vorliegenden Erfindung ist ein Verfahren zur Herstellung von Verbindungen I und ihren Salzen, das auf an sich bekannten Verfahrensweisen beruht und dadurch gekennzeichnet ist, dass man

a) in einer Verbindung der Formel

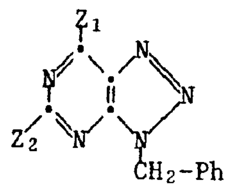

(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder einem Tautomeren und/oder Salz davon $X_1$ durch Umsetzung mit einer Verbindung der Formel H-$R_1$ (IIb) unter Abspaltung einer Verbindung $X_1$-H in $R_1$ und/oder $X_2$ durch Umsetzung mit einer Verbindung der Formel H-$R_2$ (IIj) under Abspaltung einer Verbindung $X_2$-H in $R_2$ überführt oder

b) aus einer Verbindung der Formel

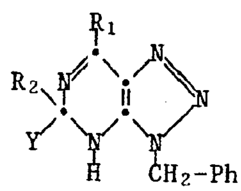

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) zur Herstellung von Verbindungen I, worin $R_2$ für Amino steht, und ihren Salzen eine Verbindung der Formel

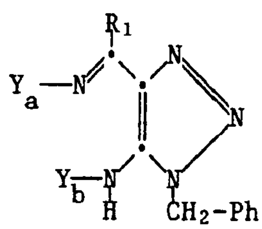

(IV),

worin einer der Reste $Y_a$ und $Y_b$ Cyano und der andere Wasserstoff bedeutet, oder ein Tautomeres und/oder Salz davon cyclisiert oder

d) ein Salz der Formel

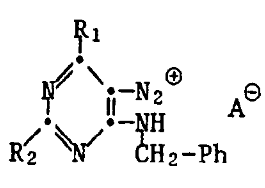

(V),

worin A für das Anion einer Protonensäure steht, cyclisiert oder

e) eine Verbindung der Formel

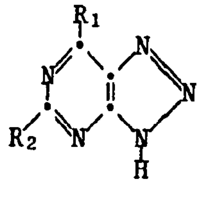

(VI)

oder ein Salz davon mit einer Verbindung der Formel $X_1$-$CH_2$-Ph (VII), worin $X_1$ eine nucleofuge Abgangsgruppe darstellt, umsetzt und gewünschtenfalls jeweils ein gegebenenfalls verfahrensgemäss erhältliches Isomerengemisch in die Komponenten auftrennt und das Isomere der Formel I isoliert, eine verfahrensgemäss oder auf andere Weise erhältliche Verbindung I in eine andere Verbindung I überführt, eine verfahrensgemäss erhältliche freie Verbindung I in ein Salz überführt, ein verfahrensgemäss erhältliches Salz einer Verbindung I in die freie Verbindung I oder in ein anderes Salz der Verbindung I umwandelt und/oder ein gegebenenfalls verfahrensgemäss erhältliches Stereoisomerengemisch in die Stereoisomeren auftrennt und das gewünschte Stereoisomere isoliert.

Die vor- und nachstehend beschriebenen verfahrensgemässen Reaktionen sowie die Herstellung neuer Ausgangsstoffe bzw. Zwischenprodukte erfolgen in an sich bekannter Weise. Dabei wird in Analogie zur Reaktions- und Bildungsweise bekannter Ausgangsstoffe bzw. Zwischenprodukte, auch wenn nicht ausdrücklich erwähnt, unter den jeweils üblichen Reaktionsbedingungen, z.B. je nach Bedarf unter Kühlen, bei Raumtemperatur oder unter Erwärmen, beispielsweise in einem Temperaturbereich von etwa -10°C bis etwa +250°C, vorzugsweise von etwa 20°C bis etwa 200°C, unter Verwendung der jeweils üblichen Hilfsmittel, wie Katalysatoren, Kondensations- sowie Solvolysemittel, in Ab- oder üblicherweise in Anwesenheit eines geeigneten Lösungs- bzw. Verdünnungsmittels oder eines Gemisches derselben, gegebenenfalls in einem geschlossenen Gefäss, in einer Inertgasatmosphäre und/oder unter wasserfreien Bedingungen, gearbeitet.

Das vor- und nachstehend aufgeführte Ausgangsmaterial der Formeln II, III, IV, V, VI und VII, das für die Herstellung der Verbindungen I und ihrer Salze verwendet wird, ist entweder bekannt oder kann ebenfalls nach an sich bekannten Methoden hergestellt werden. Ausgangsmaterial mit basischen Zentren kann auch in Form von Salzen, wie Säureadditionssalzen, beispielsweise mit den vorstehend aufgeführten Säuren, vorliegen.

Salze von Verbindungen II, III oder IV bzw. gegebenenfalls ihrer jeweiligen Tautomeren sind insbesondere Säureadditionssalze, vorzugsweise mit starken anorganischen oder organischen Säuren, z.B. analog der vorstehend für Säureadditionssalze von Verbindungen I genannten Art.

Tautomere von in der Verfahrensvariante a) verwendeten Ausgangsverbindungen II bzw. deren Salzen können beispielsweise dann auftreten, wenn in Verbindungen II bzw. deren Salzen die Gruppen $Z_1$ und/oder $Z_2$ Hydroxy oder Mercapto und/oder eine der Gruppen $Z_1$ und $Z_2$ gegebenenfalls monosubstituiertes Amino darstellen. Demnach können z.B. Verbindungen II bzw. deren Salze mit Enol-, Enthiol- und/oder Enamin-Teilstrukturen auch in protomerer Form, d.h. als entsprechende Oxo-, Thioxo- und/oder Iminotautomere vorliegen und/oder mit diesen in einem dynamischen Gleichgewicht stehen.

Nucleofuge Abgangsgruppen $X_1$ bzw. $X_2$ in Verbindungen II sind beispielsweise gegebenenfalls verätherte oder veresterte Hydroxy- oder Mercaptogruppen, Sulfinyl- und Sulfonylgruppen, ferner Sulfoniumgruppen. Veräthertes Hydroxy ist beispielsweise Niederalkoxy, wie Methoxy, oder gegebenenfalls substituiertes Phenylniederalkoxy, wie gegebenenfalls substituiertes Benzyloxy. Verestertes Hydroxy bedeutet insbesondere mit einer Mineralsäure oder einer organischen Sulfonsäure verestertes Hydroxy, vor allem Halogen, wie Chlor, Brom oder Iod, Sulfonyloxy, wie gegebenenfalls durch Halogen substituiertes Niederalkansulfonyloxy, z.B. Methan- oder Trifluormethansulfonyloxy, Cycloalkansulfonyloxy, z.B. Cyclohexansulfonyloxy, oder gegebenenfalls durch Niederalkyl oder Halogen substituiertes Benzolsulfonyloxy, z.B. Benzol-, p-Bromphenyl- oder p-Toluolsulfonyloxy, ferner Niederalkanoyloxy, z.B. Acetoxy. Veräthertes Mercapto ist z.B. Niederalkylthio, wie Methylthio, gegebenenfalls substituiertes Arylthio, wie gegebenenfalls substituiertes Phenyl-oder Naphthylthio, beispielsweise Phenyl-, p-Tolyl- oder Naphthylthio, oder gegebenenfalls substituiertes Arylniederalkylthio, wie gegebenenfalls substituiertes Benzyl- oder Naphthylmethylthio, z.B. Benzyl-, p-Brombenzyl- oder Naphthylmethylthio. Veresterte Mercaptogruppen sind beispielsweise Niederalkanoylthiogruppen, wie Acetylthio. Sulfinylgruppen sind beispielsweise Niederalkansulfinylgruppen, wie Methansulfinyl, gegebenenfalls substituierte Arylsulfinylgruppen, wie gegebenenfalls substituiertes Benzol- oder Naphthylsulfinyl, beispielsweise p-Toluol-oder Naphthylsulfinyl, oder gegebenenfalls substituiertes Benzylsulfinyl, wie Benzyl- oder p-Chlorbenzyl sulfinyl. Sulfonylgruppen sind beispielsweise Niederalkansulfonylgruppen, wie Methansulfonyl, gegebenenfalls substituierte Arylsulfonylgruppen, wie gegebenenfalls substituiertes Benzol- oder Naphthylsulfonyl, beispielsweise Benzol- oder Naphthylsulfonyl, oder gegebenenfalls substituiertes Benzylsulfonyl, wie Benzyl- oder p-Methylbenzylsulfonyl. Sulfoniumgruppen sind z.B. Diniederalkylsulfoniumgruppen, wie Dimethylsulfonium.

Die Ueberführung von $X_1$ und/oder $X_2$ in Verbindungen II, deren Tautomeren und jeweils ihren Salzen in Reste $R_1$ und/oder $R_2$ erfolgt durch Umsetzung mit Verbindungen der Formel H-$R_1$ (IIb) und/oder H-$R_2$ (IIj) oder jeweils einem Salz davon unter Abspaltung von Verbindungen $X_1$-H und/oder $X_2$-H.

Die Umsetzung von Verbindungen II, deren Tautomeren und jeweils ihren Salzen mit Verbindungen IIb und/oder IIj bzw. jeweils deren Salzen erfolgt in üblicher Weise, beispielsweise unter Kühlen, bei Raumtemperatur oder unter Erwärmen, z.B. im Temperaturbereich von etwa -20 bis etwa +250°C, vorzugsweise von etwa -10 bis etwa +200°C, gegebenenfalls in Gegenwart eines inerten Lösungs- oder Verdünnungsmittels oder

eines Gemisches derselben, gegebenenfalls in Gegenwart eines wasserbindenden Mittels, gegebenenfalls in Gegenwart eines basischen Mittels und/oder unter Inertgas, wie Stickstoff.

Als inerte Lösungs- oder Verdünnungsmittel sind beispielsweise Wasser, cyclische Aether, aromatische Kohlenwasserstoffe, N,N-Diniederalkylniederalkansäureamide, Phosphorsäureniederalkylamide, Diniederalkylsulfoxide, cyclische Amine und insbesondere, gegebenenfalls in Form von Gemischen mit Wasser, Niederalkanole, wie Tetrahydrofuran, Dioxan, Benzol, Toluol, Xylol, N,N-Dimethylformamid, Hexamethylphosphorsäuretriamid, Dimethylsulfoxid, N-Methylmorpholin und insbesondere, gegebenenfalls in Form von Gemischen mit Wasser, Methanol und Aethanol zu nennen. Ebenso können zur Herstellung von Verbindungen I, worin mindestens einer der Reste $R_1$ und $R_2$ eine wie angegeben substituierte Aminogruppe darstellt, bzw. ihren Salzen auch entsprechende Amine H-$R_1$ (IIb; $R_1$ = wie angegeben substituiertes Amino) und/oder H-$R_2$ (IIj; $R_2$ = wie angegeben substituiertes Amino), die bei den Reaktionstemperaturen in flüssiger Form handhabbar sind, im Ueberschuss eingesetzt und als Lösungs- oder Verdünnungsmittel und/oder Cosolvens, gegebenenfalls auch in gelöster Form, z.B. als wässrige Lösung, verwendet werden.

Wasserbindende Mittel sind z.B. Oxide des Phosphors, wie Phosphorpentoxid, Sulfate von Alkali- oder Erdalkalimetallen, wie Natrium- oder Calciumsulfat, Halogenide von Erdalkalimetallen, wie Calciumchlorid, oder Carbodiimide, wie N,N'-Dicyclohexylcarbodiimid.

Basische Mittel sind beispielsweise Alkali- oder Erdalkalimetallhydroxide, -hydride, -amide, -niederalkanolate, -carbonate, -diniederalkylamide oder -niederalkylsilylamide, Niederalkylamine, gegebenenfalls N-niederalkylierte Cycloalkylamine, basische Heterocyclen, Ammoniumhydroxide sowie carbocyclische Amine. Beispielhaft seien Natriumhydroxid, -hydrid, -amid, -methanolat, Kalium-tert.-butanolat, -carbonat, Lithiumdiisopropylamid, Kalium-bis(trimethylsilyl)-amid, Calciumhydrid, Triäthylamin, Cyclohexylamin, N-Cyclohexyl-N,N-dimethylamin, Pyridin, Benzyl-trimethyl-ammoniumhydroxid sowie 1,5-Diazabicyclo[5.4.0]undec-5-en (DBU) genannt. Vorzugsweise können anstelle eines zusätzlichen basischen Mittels bei der Herstellung von Verbindungen I, worin mindestens einer der Reste $R_1$ und $R_2$ eine wie angegeben substituierte Aminogruppe darstellt, bzw. ihren Salzen auch entsprechende Amine H-$R_1$ (IIb; $R_1$ = wie angegeben substituiertes Amino) und/oder H-$R_2$ (IIj; $R_2$ = wie angegeben substituiertes Amino) Verwendung finden, die dann vorteilhaft im Ueberschuss eingesetzt werden.

In einer bevorzugten Ausführungsform der Verfahrensvariante a) wird eine Verbindung der Formel

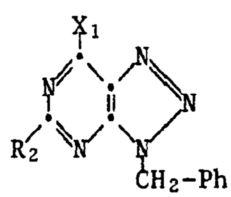

(IIa),

worin $X_1$ eine nucleofuge Abgangsgruppe, vorzugsweise Hydroxy oder Halogen, wie Chlor oder Brom, darstellt, oder ein Tautomeres, z.B. eine entsprechende 6H-7-Oxo-Verbindung, und/oder Salz davon mit Ammoniak oder einem Amin der Formel H-$R_1$ (IIb) oder einem Salz davon unter Abspaltung einer Verbindung $X_1$-H umgesetzt.

Die Ueberführung von $X_1$ in Verbindungen IIa, deren Tautomeren und jeweils ihren Salzen in einen Rest $R_1$ (Abspaltung von $X_1$-H) erfolgt in üblicher Weise, beispielsweise bei Raumtemperatur oder unter Erwärmen, z.B. im Temperaturbereich von etwa 20 bis etwa 250°C, vorzugsweise von etwa 20 bis etwa 200°C, gegebenenfalls in Gegenwart eines inerten Lösungs- oder Verdünnungsmittels, z.B. der zuvor angegebenen Art, oder eines Gemisches derselben, gegebenenfalls in Gegenwart eines basischen Mittels, beispielsweise der vorstehend erwähnten Art, gegebenfalls in Gegenwart eines wasserbindenden Mittels, z.B. der zuvor angegebenen Art, und/oder unter Inertgas, wie Stickstoff.

In einer weiteren bevorzugten Ausführungsform der Verfahrensvariante a) wird zur Herstellung von Verbindungen I, worin $R_2$ von Wasserstoff verschieden ist, bzw. deren Salzen eine Verbindung der Formel

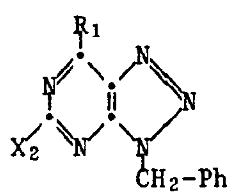

(IIc),

worin $X_2$ eine nucleofuge Abgangsgruppe, vorzugsweise Hydroxy oder Halogen, wie Chlor oder Brom, darstellt, oder ein Tautomeres und/oder Salz davon mit einer Verbindung der Formel H-$R_1$ (IIj; $R_2 \neq$ Wasserstoff), oder einem Salz davon unter Abspaltung einer Verbindung $X_2$-H umgesetzt.

Die Ueberführung von $X_2$ in Verbindungen IIc, deren Tautomeren und jeweils ihren Salzen in eine von Wasserstoff verschiedene Gruppe $R_2$ (Abspaltung von $X_2$-H) erfolgt in üblicher Weise, zum Beispiel wie vorstehend für die Ueberführung von Verbindungen IIa, deren Tautomeren und jeweils ihren Salzen in Verbindungen I bzw. deren Salze erläutert.

In einer weiteren bevorzugten Ausführungsform der Verfahrensvariante a) wird zur Herstellung von Verbindungen I, worin $R_2$ für Wasserstoff steht, bzw. deren Salzen eine Verbindung IIc, worin $X_2$ einem nucleofuge Abgangsgruppe, vorzugsweise Halogen, wie Chlor oder Brom, darstellt, oder ein Tautomeres und/oder Salz davon reduziert.

Als Reduktionsmittel kommen beispielsweise Raney-Nickel oder Wasserstoff in Betracht. Die Reduktion mit Raney-Nickel erfolgt in übliger Weise, z.b. durch Umsetzung mit einer Lösung des Metalls in einem Niederalkanol, wie Methanol, unter Erwärmen, beispielsweise im Temperaturbereich von etwa 20 bis etwa 140°C, vorzugsweise von etwa 50 bis etwa 100°C. Die Reduktion mit Wasserstoff erfolgt vorteilhaft in Gegenwart eines Hydrierungskatalysators. Als solcher kommt z.B. ein Element der VIII. Nebengruppe des Periodensystems der Elemente oder ein Derivat davon in Betracht, wie Palladium, Platin, Platinoxid, Ruthenium, Rhodium, ein Tris(triphenylphosphan)-rhodium(I)-halogenid, z.B. -chlorid, oder Raney-Nickel, wobei der Katalysator gegebenenfalls auf einem Trägermaterial, wie Aktivkohle, Alkalimetallcarbonat oder -sulfat oder einem Kieselge, aufgezogen sein kann. Die katalytische Hydrierung erfolgt vorzugsweise in einem polaren Lösungs- oder Verdünnungsmittel, insbesondere in einem Niederalkanol, wie Methanol, oder in einer starken anorganischen Säure, wie Halogenwasserstoffsäure, z.B. Chlorwasserstoffsäure, oder starken organischen Carbonsäure, insbesondere wässriger Essigsäure oder Eisessig, wobei unter Kühlung oder Erwärmen, vorzugsweise in einem Temperaturbereich von etwa -10 bis etwa +120°C, insbesondere von etwa 0 bis etwa 100°C, besonders vorteilhaft bei Raumtemperatur, gearbeitet wird.

In einer weiteren bevorzugten Ausführungsform der Verfahrensvariante a) können, ausgehend von einer Verbindung der Formel

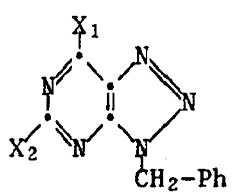

(IId),

worin $X_1$ und $X_2$ jeweils für eine nucleofuge Abgangsgruppe der vorstehend beschriebenen Art, vorzugsweise Hydroxy oder Halogen, wie Chlor oder Brom, stehen, oder einem Tautomeren und/oder Salz davon nacheinander die Gruppe $X_1$ durch einen Rest $R_1$ und die Gruppe $X_2$ durch einen Rest $R_2$ ersetzt werden. So kann z.B. vorzugsweise eine Verbindung IId oder ein Tautomeres und/oder Salz davon zunächst mit Ammoniak oder einem Amin der Formel H-$R_1$ (IIb) oder einem Salz davon unter Abspaltung einer Verbindung $X_1$-H umgesetzt und das gebildete Zwischenprodukt IIc oder ein Tautomeres und/oder Salz davon anschliessend durch Umsetzung mit Ammoniak oder einem Amin der Formel H-$R_2$ (IIj; $R_2$ = freies oder wie angegeben substituiertes Amino) oder einem Salz davon unter Abspaltung einer Verbindung $X_2$-H oder durch Reduktion, beispielsweise mittels Einwirkung von Raney-Nickel oder durch katalytische Hydrierung der zuvor beschriebenen Art, in eine Verbindung der Formel I, worin $R_2$ freies oder wie angegeben substituiertes Amino oder Wasserstoff bedeutet, bzw. ein Salz davon überführt werden.

Die Ueberführung von $X_1$ bzw. $X_2$ in Verbindungen IId, deren Tautomeren und jeweils ihren Salzen in $R_1$ bzw. $R_2$ erfolgt in üblicher Weise, beispielsweise wie vorstehend für die entsprechende Ueberführung von Verbindungen IIa bzw. IIc, deren Tautomeren und jeweils ihren Salzen in Verbindungen I bzw. deren Salze beschrieben, wobei gegebenenfalls das zunächst aus IId entstehende Zwischenprodukt IIc nicht isoliert zu werden braucht, sondern vorteilhaft in situ ohne zusätzliche Reinigung zu einer Verbindung I bzw. deren Salz weiterumgesetzt wird.

Die Verbindungen IIb und IIj bzw. jeweils deren Salze sind bekannt. Die Verbindungen IIa, IIc und IId, deren Tautomere und jeweils ihre Salze lassen sich in Analogie zu bekannten Methoden herstellen, beispielsweise durch Umsetzung einer Verbindung der Formel

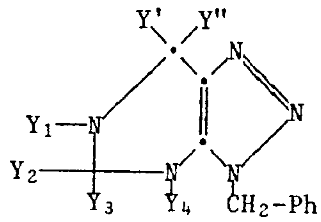

(IIe),

worin entweder Y′ und Y″ gemeinsam für gegebenenfalls funktionell abgewandeltes Oxo $X_3$ stehen, $Y_1$ Wasserstoff bedeutet und entweder $Y_2$ für $R_2$ und $Y_3$ und $Y_4$ gemeinsam für eine zusätzliche Bindung stehen oder $Y_2$ und $Y_3$ gemeinsam gegebenenfalls funktionell abgewandeltes Oxo $X_3$ und $Y_4$ Wasserstoff darstellen, oder worin Y″ für $R_1$ steht, Y′ und $Y_1$ gemeinsam für eine zusätzliche Bindung stehen, $Y_2$ und $Y_3$ gemeinsam gegebenenfalls funktionell abgewandeltes Oxo $X_3$ darstellen und $Y_4$ Wasserstoff bedeutet, oder eines Tautomeren und/oder Salzes davon mit einem Halogenierungsmittel und gegebenenfalls in üblicher Weise erfolgender Weiterumsetzung einer erhaltenen Verbindung IIa, IIc oder IId, worin $X_1$ und $X_2$ jeweils für Halogen stehen, oder eines Tautomeren und/oder Salzes davon mit einer Verbindung der Formel $X_1$-H und/oder $X_2$-H, worin $X_1$ und $X_2$ unabhängig voneinander jeweils für eine der vorstehend beschriebenen nucleofugen Abgangsgruppen, die von Halogen verschieden sind, stehen.

Gegebenenfalls funktionell abgewandeltes Oxo $X_3$ in Verbindungen IIe, deren Tautomeren und jeweils ihren Salzen ist beispielsweise Thioxo oder gegebenenfalls substituiertes Imino, vorzugweise aber Oxo. Gegebenenfalls substituiertes Imino ist beispielsweise eine Gruppe =N-R′, in der R′ dieselbe Bedeutung hat wie in der Teilstruktur -NHR′, die für einen Rest $R_1$ und/oder $R_2$ steht, wie Imino, N-Niederalkylimino, N-Cycloalkylimino oder N-Niederalkanoylimino.

Für Salze und/oder Tautomere von Verbindungen IIe gilt in analoger Weise das vorstehend für Salze und/oder Tautomere von Verbindungen II Gesagte. So sind insbesondere entsprechende Keto/Enol-, Thioketo/Enthiol- und/oder Imin/Enamin-Tautomerien möglich.

Halogenierungsmittel sind beispielsweise Halogenide von Phosphor oder Schwefel, wie Phosphortrihalogenide, Phosphorpentahalogenide, Phosphoroxytrihalogenide, Thionylhalogenide oder Sulfuryhalogenide, z.B. Phosphortrichlorid, -bromid, Phosphorpentachlorid, Phosphoroxytrichlorid, Thionylchlorid oder Sulfurylchlorid, können aber beispielsweise auch Säurehalogenide, wie Säurechloride, der Kohlensäure, beispielsweise Phosgen, sein.

Die Umsetzung einer Verbindung IIe bzw. eines Tautomeren und/oder Salzes davon mit einem Halogenierungsmittel erfolgt unter den üblichen Reaktionsbedingungen, beispielsweise unter Erwärmen, z.B. im Temperaturbereich von etwa 20 bis etwa 200°C, und in einem inerten Lösungsmittel, wie einem Halogennierderalkan, z.B. Tetrachlormethan, vorzugsweise jedoch in Form einer Lösung oder Suspension der Verbindung IIe bzw. eines Tautomeren und/oder Salzes davon in einem Ueberschuss des Halogenierungsmittels.

So erhält man beispielsweise eine Verbindung IIa, worin $X_1$ Halogen, wie Chlor, ist, oder ein Tautomeres und/oder Salz davon durch Umsetzung einer Verbindung IIe, worin Y′ und Y″ gemeinsam eine Gruppe $X_3$, vorzugsweise Oxo, $Y_1$ Wasserstoff, $Y_2$ eine Gruppe $R_2$ und $Y_3$ und $Y_4$ gemeinsame eine zusätzliche Bindung darstellen, oder eines Tautomeren und/oder Salzes davon mit einem Halogenierungsmittel, beispielsweise Phosphoroxytrichlorid, wobei die entsprechende Verbindung IIe bzw. ein Tautomeres und/oder Salz davon beispielsweise durch Umsetzung einer Verbindung der Formel

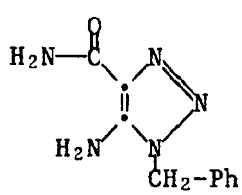

(IIf)

oder eines Salzes davon mit einer Verbindung der Formel $R_2$-X (IIg), worin X für die funktionelle Gruppe einer Carbonsäure oder eines funktionellen Derviates davon, beispielsweise für eine Carboxylgruppe der Formel -C(=O)-OH oder eine Niederalkoxycarbonylgruppe der Formel -C(=O)-O-Alk, worin Alk für Niederalkyl, wie Methyl, steht, insbesondere aber für eine Halogencarbonylgruppe der Formel -C(=O)-Hal, worin Hal für Halogen, wie Chlor oder Brom, steht, eine Amidgruppe der Formel -C(=O)-Am, worin Am für gegebenenfalls substituiertes Amino, beispielsweise Amino, N-Niederalkylamino, wie N-Methylamino, oder N,N-Diniederalkylamino, wie N,N-Dimethyl- oder N,N-Diisopropylamino, steht, oder eine Orthoestergruppe der Formel $-C(O-Alk)_3$, worin Alk Niederalkyl, wie Aethyl, bedeutet, steht, bzw. gegebenenfalls einem Salz davon unter den üblichen Reaktionsbedingungen, beispielsweise in Gegenwart eines Kondensationsmittels, wie eines basischen Mittels, und/oder unter Erwärmen, z.B. im Temperaturbereich von etwa 20 bis etwa 200°C, erhältlich ist.

Verbindungen IIc, worin $X_2$ Halogen, wie Chlor, ist, oder deren Tautomere und/oder Salze werden beispielsweise erhalten, indem man eine Verbindung IIe, worin Y′ und Y″ gemeinsam eine Gruppe =N-R′, in der R′ dieselbe Bedeutung hat wie in der Teilstruktur -NHR′, die für einen Rest $R_1$ steht, $Y_1$ und $Y_4$ Wasserstoff und $Y_2$ und $Y_3$ gemeinsam eine Gruppe $X_3$, vorzugsweise Oxo, bedeuten, oder ein Tautomeres und/oder Salz davon mit einem Halogenierungsmittel, beispielsweise Phosphoroxytrichlorid, umsetzt, wobei die entsprechende Verbindung IIe bzw. ein Tautomeres und/oder Salz davon beispielsweise durch Umsetzung einer Verbindung der Formel

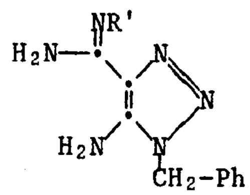

(IIh),

welche ihrerseits aus einer Verbindung IIf bzw. einem Salz davon durch übliche Umsetzung mit einer Verbindung der Formel $NH_2R'$ (IIi) bzw. einem Salz davon erhältlich ist, oder eines Tautomeren und/oder Salzes davon mit einem doppelten Säurederivat der Kohlensäure, beispielsweise Harnstoff oder Phosgen, unter den üblichen Reaktionsbedingungen, beispielsweise in Gegenwart eines Kondensationsmittels, wie eines basischen Mittels, und/oder unter Erwärmen, z.B. im Temperaturbereich von etwa 20 bis etwa 200°C, erhalten wird.

Verbindungen IId, worin $X_1$ und $X_2$ Halogen, wie Chlor, ist, oder deren Salze werden beispielsweise erhalten, indem man eine Verbindung IIe, worin Y′ und Y″ sowie $Y_2$ und $Y_3$ jeweils gemeinsam Oxo und $Y_1$ und $Y_4$ jeweils Wasserstoff darstellen, oder ein Tautomeres und/oder Salz davon mit einem Halogenierungsmittel, beispielsweise Phosphoroxytrichlorid, umsetzt, wobei die entsprechende Verbindung IIe bzw. ein Tautomeres und/oder Salz davon beispielsweise durch Umsetzung einer Verbindung IIf oder eines Salzes davon mit einem doppelten Säurederivat der Kohlensäure, beispielsweise Harnstoff oder Phosgen, unter den üblichen Reaktionsbedingungen hergestellt wird.

In einer besonders bevorzugten Ausführungsform kann man eine Verbindung IIe oder ein Tautomeres und/oder Salz davon wie vorstehend beschrieben in eine Verbindung IIa, IIc oder IId bzw. jeweils ein Tautomeres und/oder Salz davon überführen und anschliessend ohne zusätzliche Reinigung oder Isolierung das gebildete Zwischenprodukt IIa, IIc bzw. IId in situ in eine Verbindung I bzw. deren Salz überführen, wobei vorzugswiese in einem aromatischen Kohlenwasserstoff, z.B. Toluol oder Xylol, gearbeitet wird.

Die Verbindungen IIa, IIc und IId, deren Tautomere und jeweils ihre Salze lassen sich auch in analoger Weise wie unter der Verfahrensvariante d) beschrieben herstellen, indem man von einem entsprechenden Salz der Formel

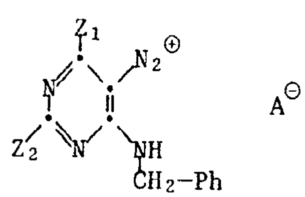

(Vb),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht und worin A für das Anion einer Protonensäure steht, ausgeht und dieses Salz Vb in analoger Weise wie unter der Verfahrensvariante d) beschrieben cyclisiert.

Für die Tautomeren und/oder Salze der in der Verfahrensvariante b) verwendeten Ausgangsverbindungen III gilt das vorstehend für Tautomere und/oder Salze von Verbindungen II Gesagte in analoger Weise.

Gegebenenfalls aliphatisch substituiertes Amino Y in Verbindungen III, deren Tautomeren und jeweils ihren Salzen ist beispielsweise solches, wie es vorstehend bei der Erläuterung der Reste $R_1$ und $R_2$ aufgeführt wird.

Die Eliminierung der Verbindung Y-H aus Verbindungen III, deren Tautomeren und jeweils ihren Salzen erfolgt in üblicher Weise, beispielsweise in einem inerten Lösungs- oder Verdünnungsmittel, beispielsweise der unter der Verfahrensvariante a) erwähnten Art, durch Erwärmen, z.B. im Temperaturbereich von etwa 40 bis etwa 250°C, vorzugsweise von etwa 80 bis etwa 200°C, und/oder durch Säurebehandlung. Dafür geeignete Säuren sind beispielsweise Mineralsäuren oder deren Anhydride bzw. sauren Salze, z.B. Halogenwasserstoffsäuren, Schwefelsäure, Alkalimetallhydrogensulfate, Phosphorsäure, Polyphosphorsäure, Phosphorpentoxid, Phosphortrichlorid oder Phosphoroxytrichlorid, organische Sulfonsäuren, wie p-Toluolsulfonsäure, oder Carbonsäuren bzw. deren Anhydride oder Halogenide, wie Niederalkansäuren und deren Anhydride oder Halogenide, z.B. Essigsäure, Acetanhydrid oder Acetylchlorid, ferner gepufferte Säurelösungen, z.B. Phosphat- oder Acetatpuffer, oder Hydrohalogenide von Stickstoffbasen, z.B. Ammonium- oder Pyridiniumchlorid.

In einer bevorzugten Ausführungsform der Verfahrensvariante b) wird beispielsweise eine Verbindung III, worin Y Hydroxy bedeutet, oder ein Tautomeres und/oder Salz davon durch Erwärmen auf 100 bis 200°C in einem inerten Lösungsmittel, beispielsweise einem Niederalkansäureamid, wie Formamid oder Acetamid, unter Austritt eines Aequivalents Wasser in eine Verbindung I bzw. ein Salz davon überführt.

Wegen der leichten Zugänglichkeit der entsprechenden Ausgangsverbindungen III ist die Verfahrensvariante b) insbesondere zur Herstellung von Verbindungen I, worin $R_1$ Amino ist, bzw. deren Salzen geeignet. So lassen sich derartige Ausgangsverbindungen III, deren Tautomere und jeweils ihre Salze in Analogie zu bekannten Methoden erhalten und werden vorzugsweise in situ hergestellt, zum Beispiel durch Cyclisierung einer Verbindung der Formel

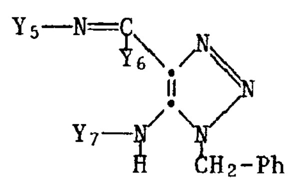

(IIIa),

worin entweder $Y_5$ und $Y_7$ Wasserstoff und $Y_6$ eine Gruppe der Formel $R_2$-C(=$X_3$)-NH- (IIIb) bedeuten oder $Y_5$ und $Y_6$ gemeinsam für eine zusätzliche Bindung und $Y_7$ für eine Gruppe der Formel $R_2$-C(Y)($NH_2$)- (IIIc) stehen, oder eines Tautomeren und/oder Salzes davon, wobei die intermediär gebildete Verbindung III bzw. ein Tautomeres und/oder Salz davon im allgemeinen ohne Isolierung erfindungsgemäss weiterreagiert.

In Gruppen der Formel IIIb steht $X_3$ für gegebenenfalls funktionell abgewandeltes Oxo, wie Oxo, Thioxo oder gegebenenfalls substituiertes Imino, wie Imino, N-Niederalkylimino, N-Cycloalkylimino oder N-Niederalkanoylimino, ferner gegebenenfalls substituiertes N-Benzoylimino, N-Niederalkansulfonylimino oder N-Arylimino.

Für Salze und/oder Tautomere von Verbindungen IIIa gilt in analoger Weise das vorstehend für Salze und/oder Tautomere von Verbindungen II Gesagte.

Die Cyclisierung von Verbindungen IIIa, deren Tautomeren und jeweils ihren Salzen und gegebenenfalls die nachfolgende in situ-Eliminierung von Y-H aus den gebildeten Verbindungen III bzw. deren Tautomeren und/oder Salzen erfolgen in üblicher Weise, z.B. unter neutralen, sauren oder basischen Bedingungen, erforderlichenfalls in Gegenwart einer Säure oder eines basischen Mittels, in Gegenwart eines inerten Lösungs- oder Verdünnungsmittels, bei Raumtemperatur oder vorzugsweise unter Erwärmen, z.B. im Temperaturbereich von etwa 20 bis etwa 250°C, und/oder unter Inertgas, wie Stickstoff. Als Säuren, basische Mittel und inerte Lösungs-oder Verdünnungsmittel können beispielsweise die entsprechenden unter der Verfahrensvariante a) erläuterten Agentien Verwendung finden. Als inertes Lösungs- oder Verdünnungsmittel kann in besonders vorteilhafter Weise aber auch ein Niederalkansäureamid, wie Formamid oder Acetamid, dienen.

In einer besonders bevorzugten Ausführungsform wird beispielsweise eine Verbindung IIIa, worin $Y_5$ und $Y_7$ Wasserstoff und $Y_6$ eine Gruppe $R_2$-C(=0)-NH- (IIIb) bedeuten oder $Y_5$ und $Y_6$ gemeinsam für eine zusätzliche Bindung und $Y_7$ für eine Gruppe $R_2$-C(OH)($NH_2$)- (IIIc) stehen, oder ein Tautomeres und/oder Salz davon durch mehrstündiges Erhitzen, beispielsweise im Temperaturbereich von etwa 80 bis etwa 200°C, in einem Niederalkansäureamid, wie Formamid oder Acetamid, cyclisiert, wobei unter den Reaktionsbedingungen in situ eine Weiterreaktion der gebildeten Verbindung III, worin Y Hydroxy ist, bzw. eines Tautomeren und/oder Salzes davon zu dem gewünschten Endprodukt der Formel I bzw. einem Salz davon eintritt. In analoger Weise lassen sich entsprechende Verbindungen IIIa mit Gruppen IIIb, worin $X_3$ Imino ist, oder Gruppen IIIc, worin Y Amino ist, oder Tautomere und/oder Salze davon in einem inerten Lösungsmittel, wie einem Halogenalkan, beispielsweise Tetrachlormethan, cyclisieren und zu Verbindungen I bzw. deren Salzen weiterumsetzen.

Die Verbindungen IIIa, deren Tautomere und jeweils ihre Salze erhält man aus Verbindungen der Formel

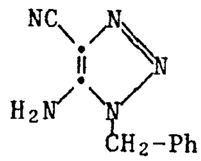

(IIId)

bzw. deren Salzen durch Reaktion mit einer Verbindung der Formel $R_2$-C(=$X_3$)-$NH_2$ (IIIe), worin $X_3$ für gegebenenfalls funktionell abgewandeltes Oxo, beispielsweise der bei der Gruppe IIIb erläuterten Art, steht, bzw. deren Salzen, wobei in Analogie zu bekannten Verfahren gearbeitet wird, beispielsweise bei Raumtemperatur oder vorzugsweise unter Erwärmen z.B. im Temperaturbereich von etwa 20 bis etwa 250°C, unter neutralen, sauren oder basischen Bedingungen, erforderlichenfalls in Gegenwart einer Säure oder eines basischen Mittels, beispielweise der vorstehend erwähnten Art, in Gegenwart eines inerten Lösungs- oder Verdünnungsmittels, z.B. der vorstehend erwähnten Art, und/oder unter Inertgas, wie Stickstoff. Als inertes Lösungs- oder Verdünnungsmittel kann dabei insbesondere auch ein Niederalkansäureamid, wie Form- oder Acetamid, dienen.

In einer besonders bevorzugten Ausführungsform wird eine Verbindung IIId bzw. ein Salz davon mit einem grossen Ueberschuss einer Verbindung IIIe ($X_3$=gegebenenfalls funktionell abgewandeltes Oxo, vorzugsweise Oxo), beispielsweise in Formamid oder Acetamid ($X_3$ =Oxo, $R_2$ = Wasserstoff bzw. Methyl), unter Erwärmen, beispielsweise im Temperaturbereich von etwa 80 bis etwa 200°C, zu einer Verbindung IIIa bzw. einem Tautomeren und/oder Salz davon umgesetzt, wobei die Reaktionskomponente IIIe gleichzeitig als Lösungs- bzw. Verdünnungsmittel dient.

Vorteilhafterweise werden allerdings auch die Verbindungen IIIa, deren Tautomere und jeweils ihre Salze in situ hergestellt und ohne Isolierung zu Verbindungen III bzw. deren Tautomeren und/oder Salzen weiterumgesetzt, welche ihrerseits, wie vorstehend beschrieben, im allgemeinen gleichfalls in situ zu Verbindungen I bzw. deren Salzen weiterreagieren.

So kann nach Art einer Eintopfreaktion eine Verbindung IIId bzw. ein Salz davon unter Erwärmen mit einem grossen Ueberschuss einer Verbindung IIIe ($X_3$=gegebenenfalls funktionell abgewandeltes Oxo, vorzugsweise Oxo), beispielsweise in Formamid oder Acetamid ($X_3$ = Oxo, $R_2$ = Wasserstoff bzw. Methyl), umgesetzt werden, wobei zunächst eine Verbindung IIIa bzw. ein Tautomeres und/oder Salz davon gebildet wird, woran sich in situ bei Aufrechterhaltung der Wärmezufuhr die Cyclisierung zu einer Verbindung III bzw. einem Tautomeren und/oder Salz davon anschliesst, ihrerseits gefolgt von einer in situ-Eliminierung einer Verbindung Y-H (Y = Hydroxy) zu einer Verbindung I ($R_1$ = Amino, $R_2$ beispielsweise=Wasserstoff bzw. Methyl) bzw. einem Salz davon.

Für die Tautomeren und/oder Salze der in der Verfahrensvariante c) verwendeten Ausgangsstoffe IV gilt das vorstehend für Tautomere und/oder Salze von Verbindungen II Gesagte in analoger Weise.

Die Cyclisierung von Verbindungen IV, deren Tautomeren und jeweils ihren Salzen zu Verbindungen I, worin $R_2$ Amino bedeutet, bzw. deren Salzen erfolgt unter den üblichen Cyclisierungsbedingungen, beispielsweise in analoger Weise wie unter der Verfahrensvariante b) für Cyclisierungen von Verbindungen IIIa zu Verbindungen III beschrieben.

Die Ausgangsstoffe IV, deren Tautomere und jeweils ihre Salze lassen sich in Analogie zu bekannten Methoden erhalten, beispielsweise durch Reaktion einer Verbindung der Formel

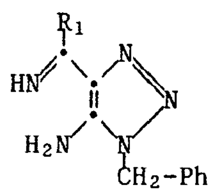

(IVa)

oder eines Tautomeren und/oder Salzes davon mit einer Verbindung der Formel $X_1$—C≡N (IVb), worin $X_1$ eine nucleofuge Abgangsgruppe, beispielsweise der unter der Verfahrensvariante a) beschriebenen Art, vorzugsweise Halogen, wie Chlor oder Brom, oder freies oder wie für Reste $R_1$ bzw. $R_2$ angegeben substituiertes Amino darstellt, wobei unter den üblichen Reaktionsbedingungen, beispielsweise bei Raumtemperatur oder unter Erwärmen, in einem inerten Lösungs- oder Verdünnungsmittel, z.B. der vorstehend erwähnten Art, gegebenenfalls in Gegenwart eines Kondensationsmittels, beispielsweise eines basischen Mittels, z.B. der vorstehend beschriebenen Art, und/oder unter Inertgas, wie Stickstoff, gearbeitet wird.

Für die Tautomeren und/oder Salze der Verbindungen IVa gilt das vorstehend für Tautomere und/oder Salze von Verbindungen II Gesagte in analoger Weise.

In einer bevorzugten Ausführungsform werden die Verbindungen IV bzw. deren Tautomere und/oder Salze nicht isoliert, sondern in situ generiert und ohne Isolierung oder zusätzliche Reinigung erfindungsgemäss zu Verbindungen I bzw. deren Salzen cyclisiert.

Die Verbindungen IVa, deren Tautomere und jeweils ihre Salze können in Analogie zu bekannten Methoden hergestellt werden, beispielsweise durch Umsetzung einer Verbindung IIId bzw. eines Salzes davon mit Ammoniak oder einem Amin der Formel H-$R_1$ (IIb) bzw. einem Salz davon unter den üblichen Reaktionsbedingungen.

Anionen A von Protonensäuren in Salzen der Formel V, die als Ausgangsstoffe gemäss der Verfahrensvariante d) verwendet werden, sind beispielsweise Anionen der vorstehend für die Bildung von Säureadditionssalzen von Verbindungen I genannten Säuren, insbesondere Anionen von starken anorganischen Protonensäuren, wie Anionen von Mineralsäuren, z.B. Schwefelsäure, einer Phosphorsäure oder einer Halogenwasserstoffsäure, oder von Tetrafluoroborsäure, oder Anionen von starken organischen Carbonsäuren, wie Niederalkancarbonsäuren, z.B. Ameisen-oder Essigsäure, beispielsweise das Suflat-, Phosphat-, Chlorid-, Bromid-, Tetrafluorborat- oder Acetation.

Die Cyclisierung von Salzen der Formel V zu Verbindungen I bzw. deren Salzen erfolgt in Analogie zu bekannten Verfahrensweisen unter den üblichen Reaktionsbedingungen, beispielsweise in einem Lösungsoder Verdünnungsmittel, vorzugweise in Wasser, und/oder unter Kühlung, bei Raumtemperatur oder unter Erwärmen, beispielsweise im Temperaturbereich von etwa -20 bis etwa +150°C, vorzugseise von etwa 0 bis etwa +100°C.

Die Ausgangsstoffe V sind bekannt oder lassen sich in Analogie zu bekannten Methoden, beispielsweise durch Umsetzung einer Verbindung der Formel

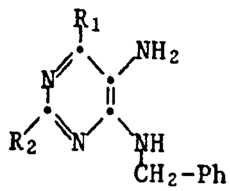

(Va)

oder eines Salzes davon mit salpetriger Säure, herstellen, wobei unter den üblichderweise angewendeten Reaktionsbedingungen gearbeitet wird, beispielsweise in einem Lösungs- oder Verdünnungsmittel, vorzugsweise in Wasser, und/oder unter Kühlung, bei Raumtemperatur oder unter Erwärmen, beispielsweise im Temperaturbereich von etwa -20 bis etwa +150°C. Die salpetrige Säure wird dabei vorzugsweise in situ, beispielsweise durch Umsetzung eines Alkalimetallnitrits, wie Natriumnitrit, mit einer starken Protonensäure, beispielsweise einer Halogenwasserstoffsäure, wie Chlorwasserstoffsäure, oder einer Niederalkancarbonsäure, wie Ameisensäure oder Eisessig, hergestellt.

In einer besonders bevorzugten Ausführungsform der Verfahrensvariante d) werden die Verbindungen Va bzw. deren Salze wie vorstehend erläutert mit, beispielswiese in situ erzeugter, salpetriger Säure umgesetzt und die zunächst gebildeten Salze V anschliessend ohne Isolierung und/oder zusätzliche Reinigung in situ erfindungsgemäss zu den gewünschten Verbindungen I bzw. deren Salzen cyclisiert.

Die Verbindungen Va bzw. deren Salze sind bekannt oder in Analogie zu bekannten Methoden herstellbar.

Salze der in der Verfahrensvariante e) verwendeten Ausgangsstoffe VI sind insbesondere Metallsalze, wie Alkali- oder Erdalkalimetallsalze, beispielsweise Natrium-, Kalium-, Magnesium- oder Calciumsalze, oder Uebergangsmetallsalze, z.B. pharmazeutisch verwendbare Uebergangsmetallsalze, beispielsweise Zink- oder Kupfersalze, derselben.

Nucleofuge Abgangsgruppen $X_1$ in Verbindungen VII sind beispielsweise solche der unter der Verfahrensvariante a) angegebenen Art.

Die Umsetzung einer Verbindung VI mit einer Verbindung VII erfolgt in üblicher Weise, beispielsweise in Gegenwart eines basischen Kondensationsmittels oder vorteilhaft, indem man die Komponente der Formel VI in Form eines ihrer Metallsalze einsetzt, bei Raumtemperatur oder vorzugsweise unter Erwärmen, beispielsweise in einem Temperaturbereich von etwa 20 bis etwa 200°C, insbesondere von etwa 50 bis etwa 150°C, in einem inerten Lösungs- oder Verdünnungsmittel, beispielsweise der vorstehend erwähnten Art, und/oder unter Inertgas, wie Stickstoff. Als basische Kondensationsmittel sind insbesondere mit der Komponente VI salzbildende basische Kondensationsmittel geeignet, beispielsweise die unter der Verfahrensvariante a) erwähnten basischen Mittel. Wie erwähnt wird die Ueberführung der Komponente VI in eines ihrer Salze vorteilhaft vorab

durchgeführt, beispielsweise durch Umsetzung mit einem der genannten basischen Mittel.

Die Ausgangsstoffe VI sind bekannt oder in Analogie zu bekannten Methoden herstellbar, beispielsweise entsprechend der Verfahrensvariante d) durch Cyclisierung entsprechender Salze V, die in 4-Position anstelle der Gruppe $-NH-CH_2-Ph$ eine unsubstituierte Aminogruppe aufweisen. Die Ausgangsstoffe VII sind bekannt oder können in Analogie zu bekannten Methoden erhalten werden.

Verfahrensgemäss oder anderweitig erhältliche Verbindungen der Formel I können in andere Verbindungen der Formel I überführt werden, indem man eine oder mehrere Variablen der allgemeinen Formel I in andere überführt.

So kann man in Verbindungen I unsubstituiertes Amino $R_1$ und/oder $R_2$ in N-Mono- oder N,N-Diniederalkylamino, ebenso N-Mononiederalkylamino $R_1$ und/oder $R_2$ in N,N-Diniederalkylamino überführen, beispielsweise durch Behandeln mit einem reaktiven Ester eines Niederalkanols, wie einem Niederalkylhalogenid, z.B. -bromid oder -iodid, Niederalkansulfonat, z.B. Methansulfonat, einem gegebenenfalls substituierten Arylsulfonat, wie Benzol- oder p-Toluolsulfonat, oder einem Diniederalkylsulfat, z.B. Dimethylsulfat, vorzugsweise unter basischen Bedingungen, wie in Gegenwart von Natriumhydrid oder von Natronlauge oder Kalilauge und vorteilhaft in Gegenwart eines Phasentransfer-Katalysators, wie Tetrabutylammoniumbromid oder Benzyltrimethylammoniumchlorid. Dabei kann entweder nur eine N-Niederalkylgruppe eingeführt werden, oder es können in einem einzigen Reaktionsschritt auch mehrere, insbesondere 2 bis und mit 4, N-Niederalkylgruppen eingeführt werden. Ebenso ist es möglich, in aufeinanderfolgenden Reaktionsschritten bei geeigneter Wahl der Niederalkylkomponenten unterschiedliche N-Niederalkylgruppen in unsubstituiertes Amino bzw. N-Mononiederalkylamino $R_1$ und/oder $R_2$ einzuführen.Gemäss dieser Methode der N-Alkylierung erhält man jeweils Verbindungen I, in denen alle im selben Reaktionsschritt eingeführten N-Niederalkylgruppen gleichartig sind.

In analoger Weise kann auch in N-(Hydroxyniederalkyl)amino, N-Monocycloalkylamino, N-Mono(cycloalkylniederalkyl)amino und N-Niederalkanoylamino $R_1$ und/oder $R_2$ jeweils eine N-Niederalkylgruppe eingeführt werden, wobei man zu N-(Hydroxyniederalkyl)-N-niederalkyl-amino, N-Cycloalkyl-N-niederalkyl-amino, N-(Cycloalkylniederalkyl)-N-niederalkyl-amino und N-Niederalkanoyl-N-niederalkyl-amino $R_1$ und/oder $R_2$ gelangt.

Ebenso kann bei entsprechender sinnvoller Abwandlung der Alkylierungskomponenten auch unsubstituiertes Amino $R_1$ und/oder $R_2$ durch Einführung einer N-(Niederalkoxyniederalkyl)gruppe in N-(Niederalkoxyniederalkyl)amino $R_1$ und/oder $R_2$, durch Einführung einer N-(Hydroxyniederalkyl)gruppe in N-(Hydroxyniedereralkyl)amino $R_1$ und/oder $R_2$, durch Einführung einer oder mehrerer, insbesondere 2 bis und mit 4, N-Cycloalkylgruppe(n) in N-Mono- oder N,N-Dicycloalkylamino $R_1$ und/oder $R_2$ oder durch Einführung einer oder meherer, insbesondere 2 bis und mit 4,N-(Cycloalkylniederalkyl)gruppe(n) in N-Mono- oder N,N-Di(cycloalkylniederalkyl)amino $R_1$ und/oder $R_2$, weiterhin N-Niederalkylamino $R_1$ und/oder $R_2$ durch Einführung einer N-(Hydroxyniederalkyl)gruppe in N-(Hydroxyniederalkyl)-N-niederalkyl-amino $R_1$ und/oder $R_2$, durch Einführung einer N-Cycloalkylgruppe in N-Cycloalkyl-N-niederalkyl-amino $R_1$ und/oder $R_2$ oder durch Einführung einer N-(Cycloalkylniederalkyl)gruppe in N-(Cycloalkylniederalkyl)-N-niederalkyl-amino $R_1$ und/oder $R_2$, weiterhin N-Monocycloalkylamino $R_1$ und/oder $R_2$ durch Einführung einer N-Cycloalkylgruppe in N,N-Dicycloalkylamino $R_1$ und/oder $R_2$ sowie N-Mono(cycloalkylniederalkyl)amino $R_1$ und/oder $R_2$ durch Einführung einer N-(Cycloalkylniederalkyl)gruppe in N,N-Di(cycloalkylniederalkyl)amino $R_1$ und/oder $R_2$ umgewandelt werden.

Weiterhin kann man unsubstituiertes Amino $R_1$ und/oder $R_2$ N-acylieren, beispielsweise durch Umsetzung mit einer Niederalkansäure, wie Ameisen-, Essig- oder Propionsäure, oder einem reaktiven Derivat einer solchen Säure, z.B. einem Säurehalogenid, wie Säurechlorid, Ester oder insbesondere einem Anhydrid, z.B. Acetylchlorid oder Acetanhydrid, in N-Niederalkanoylamino $R_1$ und/oder $R_2$ überführen. Ebenso kann N-Niederalkylamino $R_1$ und/oder $R_2$ in N-Niederalkanoyl-N-niederalkyl-amino $R_1$ und/oder $R_2$ überführt werden. Dabei ist es wiederum möglich, entweder nur eine N-Acylgruppe einzuführen oder in einem Reaktionsschritt sowohl Amino oder N-Niederalkylamino $R_1$ als auch Amino oder N-Niederalkylamino $R_2$ zu N-acylieren. Ebenso ist es möglich, in aufeinander folgenden Reaktionsschritten bei geeigneter Wahl der Acylierungsagentien unterschiedliche N-Acylgruppen in unsubstituiertes Amino oder N-Niederalkylamino $R_1$ und $R_2$ einzuführen. In jedem Fall erhält man jeweils Verbindungen I, in denen alle im selben Reaktionsschritt eingeführten N-Acylgruppen gleichartig sind.

Ebenso lässt sich N-Niederalkanoylamino $R_1$ und/oder $R_2$ in unsubstituiertes Amino $R_1$ und/oder $R_2$ überführen, beispielsweise durch Reduktion, also Austausch der Acylgruppe(n) gegen Wasserstoff, wozu die üblichen Reduktionssysteme und Reaktionsbedingungen in Betracht kommen, z.B. Diboran, Lithiumaluminiumhydrid in Tetrahydrofuran, Diäthyläther oder Dioxan, Natriumborhydrid/Cobalt(II)chlorid, Natriumborhydrid/Trifluoressigsäure oder Trihalogensilane, wie Trichlorsilan. Weiterhin lässt sich N-Niederalkanoylamino $R_1$ und/oder $R_2$ auch durch Hydrolyse in unsubstituiertes Amino $R_1$ und/oder $R_2$ überführen, wobei unter den üblichen Reaktionsbedingungen gearbeitet wird, beispielsweise in wässriger Lösung, in Gegenwart eines basischen Mittels, insbesondere z.B. in Gegenwart eines Alkalimetallhydroxids oder -niederalkanolats, wie Natrium- oder Kaliumhydroxid oder Natriummethanolat, vorzugsweise in einem organischen Lösungs- oder Verdünnungsmittel bzw. Cosolvens und/oder unter Erwärmen, vorzugsweise im Temperaturbereich von etwa 20 bis etwa 150°C, insbesondere von etwa 40 bis etwa 100°C. Dabei ist es möglich, je nach Anzahl eingesetzter Aequivalente des Reduktions mittels bzw. des basischen Mittels nur eine oder, falls vorhanden, beide Acylgruppen zu unsubstituiertem Amino $R_1$ bzw. $R_2$ zu reduzieren bzw. hydrolysieren.

Die neuen Verbindungen und ihre Salze können je nach Anzahl der asymmetrischen Kohlenstoffatome Stereoisomere, beispielsweise Diastereomere oder Enantiomere, bilden. Asymmetrische Kohlenstoffatome können beispielsweise in den Verbindungen I bzw. deren Salzen in entsprechenden Niederalkylresten $R_2$ auftreten.

Erhaltene Isomeren- und Diastereomerengemische können auf Grund der unterschiedlichen physikalischen Eigenschaften der Komponenten nach üblichen physikalischen Trennverfahren, beispielsweise durch Destillation, Kristallisation und/oder Chromatographie, in die Komponenten aufgetrennt werden.

Erhaltene Enantiomerengemische, z.B. Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man jeweils das wirksamere Stereoisomere.

Ferner können erhaltene freie Verbindungen der Formel I mit basischen Zentren in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon oder mit einem geeigneten Anionenaustauscher.

Erhaltene Säureadditionssalze von Verbindungen der Formel I können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditionssalze von Verbindungen der Formel I können in an sich bekannter Weise in andere Säureadditionssalze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet.

Die neuen Verbindungen der Formel I und ihre Salze können auch in Form ihrer Hydrate erhalten werden und/oder andere, z.B. gegebenenfalls zur Kristallisation von in fester Form vorliegenden Stoffen verwendete, Lösungsmittel einschliessen.

Je nach Verfahrensweise bzw. Reaktionsbedingungen können die erfindungsgemässen Verbindungen der Formel I in freier Form oder in Form ihrer Salze erhalten werden.

Infolge der engen Beziehung zwischen den neuen Verbindungen der Formel I in freier Form und in Form ihrer Salze sind vorstehend und nachfolgend unter den freien Verbindungen der Formel I sinn- und zweckgemäss gegebenenfalls auch die entsprechenden Salze bzw. unter den Salzen auch die entsprechenden freien Verbindungen der Formel I zu verstehen.

Die Erfindung betrifft auch diejenigen Ausführungsformen des Verfahrens, nach denen man von einer der auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Neue Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung sowie ihre Verwendung als Zwischenprodukte bilden ebenfalls einen Gegenstand der Erfindung.

Die Erfindung betrifft ebenfalls die Verwendung der neuen Verbindungen der Formel I bzw. ihrer pharmazeutisch verwendbaren Salze, insbesondere als pharmakologische, in erster Linie antikonvulsiv wirksame, Wirksubstanzen. Dabei kann man sie, vorzugsweise in Form pharmazeutischer Präparate, in einem Verfahren zur prophylaktischen und/oder therapeutischen Behandlung des tierischen oder menschlichen Körpers, insbesondere als Antikonvulsiva, z.B. zur Behandlung von Konvulsionen verschiedenartiger Genese, beispielsweise zur Behandlung der Epilepsie, anwenden.

Die Erfindung betrifft gleichfalls pharmazeutische Präparate, die eine Verbindung der Formel I bzw. ein pharmazeutisch verwendbares Salz davon als Wirkstoff enthalten, sowie Verfahren zu ihrer Herstellung.

Bei den erfindungsgemässen pharmazeutischen Präparaten handelt es sich um solche, die eine therapeutisch wirksame Menge der erfindungsgemässen Aktivsubstanz, gegebenenfalls zusammen mit anorganischen oder organischen, festen oder flüssigen, pharmazeutisch verwendbaren Hilfsstoffen enthalten, und die sich zur enteralen, z.B. oralen, oder parenteralen Verabreichung an Warmblüter eignen. So verwendet man vorzugsweise pharmazeutische Präparate in Dosis einheitsformen, wie Dragées, Tabletten, Kapseln oder Suppositorien, ferner Ampullen, welche den Wirkstoff zusammen mit Verdünnungsmitteln, z.B. Laktose, Dextrose, Saccharose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, Adsorptionsmittel, Farbstoffe, Geschmacksstoffe und/oder Süssmittel enthalten. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer, enthalten. Die neuen pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0.1 % bis etwa 100 %, insbesondere von etwa 1 % bis etwa 50 %, Lyophilisate bis zu 100 % des Aktivstoffes.

Die Dosierung kann von verschiedenen Faktoren, wie Applikationsweise, Warmblüter-Spezies, Alter und/oder individuellen Zustand abhängen. Die täglich zu verabreichenden Dosen liegen im Normalfall bei oraler Applikation zwischen etwa 1 und etwa 30 mg/kg und für Warmblüter mit einem Gewicht von etwa 70 kg vorzugsweise zwischen etwa 0.1 g und etwa 3,0 g, wobei zur Erreichung der Tagesdosis auch mehrere Teildosen verabreicht werden können.

Die folgenden Beispiele dienen zur Illustration der oben beschriebenen Erfindung; sie sollen jedoch diese in ihrem Umfang in keiner Weise einschränken. Temperaturen werden in Celsiusgraden angegeben.

Beispiel 1:

Eine Lösung von 9,22 g (35 mmol) rohem 7-Chlor-3-(2-fluorbenzyl)-3H-1,2,3-triazolo[4,5-d]pyrimidin in 150 ml Toluol tropft man unter Rühren zu einer Mischung von 750 ml Aethanol und 200 ml 40%iger wässriger Methylaminlösung. Man lässt 15 Stunden bei Raumtemperatur stehen und destilliert dann die Lösungsmittel unter vermindertem Druck ab. Den Rückstand versetzt man mit 500 ml Wasser. Das ausgefallene Produkt wird abgesaugt und aus Methanol umkristallisiert. Man erhält so das 3-(2-Fluorbenzyl)-7-(N-methylamino)-3H-1,2,3-triazolo[4,5-d]pyrimidin, das bei 180-182° schmilzt.

Das 7-Chlor-3-(2-flurobenzyl)-3H-1,2,3-triazolo[4,5-d]pyrimidin kann man beispielsweise wie folgt erhalten:

70,5 g (0,3 mol) 5-Amino-1-(2-fluorbenzyl)-1H-1,2,3-triazol-4-carboxamid und 339 g (300 ml; 7,53 mol) Formamid werden 2 Stunden zum schwachen Sieden erhitzt. Dann lässt man auf etwa 100° abkühlen und giesst die Reaktionslösung auf 2 l Eiswaser. Das ausgefallene Produkt wird abgesaugt und mit Wasser gewaschen. Nach Trocknung bei 100° erhält man das 3-(2-Fluorbenzyl)-3H,6H,7H-1,2,3-triazolo[4,5-d]pyrimidin-7-on vom Smp. 215-218°.

18 g (73,5 mmol) 3-(2-Fluorbenzyl)-3H,6H,7H-1,2,3-triazolo[4,5-d]pyrimidin-7-on und 151 g (90 ml; 0,98 mol) Phosphoroxytrichlorid werden 4 Stunden unter Rückfluss erhitzt und dann nach Abkühlen auf Raumtemperatur mit 1 l Toluol verdünnt. Die trübe Lösung wird mit Aktivkohle versetzt und durch Hyflo filtriert. Das Filtrat dampft man unter vermindertem Druck auf etwa ein Viertel seines Ausgangs volumens ein. Das rohe 7-Chlor-3-(2-fluorbenzyl)-3H-1,2,3-triazolo[4,5-d]pyrimidin kann ohne zusätzliche Reinigung in Form der Toluollösung weiter eingesetzt werden.

Beispiel 2:

In analoger Weise wei in Beispiel 1 beschrieben erhält man, ausgehend von 7-Chlor-3-(2-fluorbenzyl)-3H-1,2,3-triazolo[4,5-d]pyrimidin, unter Verwendung von Dimethylamin das 7-(N,N-Dimethylamino)-3-(2-fluorbenzyl)-3H-1,2,3-triazolo[4,5-d]-pyrimidin. Nach Umkristallisation aus Toluol/Cyclohexan schmilzt es bei 117-119°.

Beispiel 3:

6,9 g (26 mmol) 3-(2,6-Difluorbenzyl)-3H,6H,7H-1,2,3-triazolo[4,5-d]pyrimidin-7-on und 50,5 g (30 ml; 327 mmol) Phosphoroxytrichlorid werden 1 Stunde unter Rückfluss erhitzt. Nach dem Abkühlen auf Raumtemperatur wird die Reaktionslösung mit 300 ml Xylol verdünnt. Die trübe Lösung wird mit Aktivkohle versetzt und durch Hyflo filtriert. Das Filtrat dampft man unter vermindertem Druck bei 60° auf etwa ein Viertel des Ausgangsvolumens ein. Diese Lösung des rohen 7-Chlor-3-(2,6-difluorbenzyl)-3H-1,2,3-triazolo[4,5-d]pyrimidins in Xylol giesst man zu einer gerührten Mischung von 400 ml Aethanol und 100 ml 40%iger wässiger Methylaminlösung. Nach einer Stunde dampft man das Reaktionsgemisch unter vermindertem Druck ein und behandelt den Rückstand mit Wasser. Das ausgefallene Rohprodukt wird abgesaugt und aus Methanol umkristallisiert. Das erhaltene 3-(2,6-Difluorbenzyl)-7-(N-methylamino)-3H-1,2,3-triazolo[4,5-d]pyrimidin besitzt einen Smp. von 225-227°.

Das 3-(2,6-Difluorbenzyl)-3H,6H,7H-1,2,3-triazolo[4,5-d]pyrimidin-7-on lässt sich zum Beispiel folgendermassen herstellen:

7,6 g (30 mmol) 5-Amino-1-(2,6-difluorbenzyl)-1H-1,2,3-triazol-4-carboxamid und 84,7 g (75 ml; 1.88 mol) Formamid werden 45 Minuten unter schwachem Rückfluss erhitzt. Nach dem Abkühlen auf etwa 100° giesst man die Reaktionslösung auf 400 ml Eiswasser und saugt das ausgefallene Produkt ab. Dieses wird mit Wasser gewaschen und getrocknet. Das erhaltene 3-(2,6-Difluorbenzyl)-3H,6H,7H-1,2,3-triazolo[4,5-d]pyrimidin-7-on schmilzt bei 235-237°.

Beispiel 4:

In analoger Weise wie in Beispiel 3 beschrieben erhält man, ausgehend von 3-(2-Fluorbenzyl)-5-methyl-3H,6H,7H-1,2,3-triazolo[4,5-d]pyrimidin-7-on, das 7-Chlor-3-(2-fluorbenzyl)-5-methyl-3H-1,2,3-triazolo[4,5-d]pyrimidin und hieraus das 3-(2-Fluorbenzyl)-5-methyl-7-(N-methylamino)-3H-1,2,3-triazolo[4,5-d]pyrimidin, das bei 204-206° (aus Methanol) schmilzt.

Die 3-(2-Fluorbenzyl)-5-methyl-3H,6H,7H-1,2,3-triazolo[4,5-d]pyrimidin-7-on kann man z.B. in analoger Weise wie in Beispiel 3 beschrieben durch zweistündiges Erhitzen von 5,9 g (25 mmol) 5-Amino-1-(2-fluorbenzyl)-1H-1,2,3-triazol-4-carboxamid und 59 g (1 mol) Acetamid auf 230° erhalten; es besitzt einen Smp. von 218-220°.

Beispiel 5:

5 g (23 mmol) 5-Amino-4-cyano-1-(2-fluorbenzyl)-1H-1,2,3-triazol und 45 g (40 ml; 1 mol) Formamid werden 3 Stunden auf 180° erhitzt. Nach dem Abkühlen auf Raumtemperatur verdünnt man mit 100 ml Wasser. Das ausgefallene Produkt wird abgesaugt und aus 75%iger Essigsäure umkristallisiert. Das erhaltene 7-Amino-3-(2-fluorbenzyl)-3H-1,2,3-triazolo[4,5-d]pyrimidin schmilzt bei 254-257°.

Beispiel 6:

In analoger Weise wie in Beispiel 5 beschrieben erhält man durch Umsetzung von 5-Amino-4-cyano-1-(2-fluorbenzyl)-1H-1,2,3-triazol mit Acetamid das 7-Amino-3-(2-fluorbenzyl)-5-methyl-3H-1,2,3-triazolo[4,5-d]pyrimidin, das einen Smp. von 242-244° besitzt.

Beispiel 7:

In analoger Weise wie in den Beispielen 1 bis 6 beschrieben kann man ferner herstellen:
das 7-(N-Acetylamino)-3-(2-fluorbenzyl)-3H-1,2,3-triazolo[4,5-d]pyrimidin und
das 7-(N-Acetyl-N-methyl-amino)-3-(2-fluorbenzyl)-3H-1,2,3-triazolo[4,5-d]pyrimidin.

Beispiel 8:

In analoger Weise wie in Beispiel 3 beschrieben erhält man, ausgehend von 3-(2-Chlorbenzyl)-3H,6H,7H-1,2,3-triazolo[4,5-d]pyrimidin-7-on, das 7-Chlor-3-(2-chlorbenzyl)-3H-1,2,3-triazolo[4,5-d]pyrimidin und hieraus durch Umsetzung mit Methylamin das 3-(2-Chlorbenzyl)-7-(N-methylamino)-3H-1,2,3-triazolo[4,5-d]pyrimidin vom Smp. 192-194° (aus Aethanol) bzw. durch Umsetzung mit Dimethylamin das 3-(2-Chlorbenzyl)-7-(N,N-dimethylamino)-3H-1,2,3-triazolo[4,5-d]pyrimidin vom Smp. 131-133°C (aus Acetonitril).

Das 3-(2-Chlorbenzyl)-3H,6H,7H-1,2,3-triazolo[4,5-d]pyrimidin-7-on erhält man z.B. in analoger Weise wie

in Beispiel 3 beschrieben durch Erhitzen von 5-Amio-1-(2-chlorbenzyl)-1H-1,2,3-triazol-4-carboxamid und Formamid [Smp.: 285-288°C (Zersetzung; aus Eisessig)].

Beispiel 9:

In analoger Weise wie in Beispiel 3 beschrieben erhält man, ausgehend von 3-(2-Methylbenzyl)-3H,6H,7H-1,2,3-triazolo[4,5-d]pyrimidin-7-on, das 7-Chlor-3-(2-methylbenzyl)-3H-1,2,3-triazolo[4,5-d]pyrimidin und hieraus durch Umsetzung mit Methylamin das 7-(N-Methylamino)-3-(2-methylbenzyl)-3H-1,2,3-triazolo[4,5-d] pyrimidin, das bei 193-195° schmilzt (aus Methanol).

Das 3-(2-Methylbenzyl)-3H,6H,7H-1,2,3-triazolo[4,5-d]pyrimidin-7-on erhält man z.B. in analoger Weise wie in Beispiel 3 beschrieben durch Erhitze von 5-Amino-1-(2-methylbenzyl)-1H-1,2,3-triazol-4-carboxamid und Formamid [Smp.: 265-267° (aus Eisessig)].

Beispiel 10:

In analoger Weise wie in Beispiel 3 beschrieben erhält man, ausgehend von 3-(3-Fluorbenzyl)-3H,6H,7H-1,2,3-triazolo[4,5-d]pyrimidin-7-on, das 7-Chlor-3-(3-fluorbenzyl)-3H-1,2,3-triazolo[4,5-d]pyrimidin und hieraus durch Umsetzung mit Methylamin das 3-(3-Fluorbenzyl)-7-(N-methylamino)-3H-1,2,3-triazolo[4,5-d]pyrimidin vom Smp. 187-189° (aus Toluol).

Das 3-(3-Fluorbenzyl)-3H,6H,7H-1,2,3-triazolo[4,5-d]pyrimidin-7-on erhält man z.B. in analoger Weise wie in Beispiel 3 beschrieben durch Erhitzen von 5-Amino-1-(3-fluorbenzyl)-1H-1,2,3-triazol-4-carboxamid und Formamid [Smp.: 235-237° (aus 50%iger Essigsäure)].

Beispiel 11:

In analoger Weise wie in Beispiel 3 beschrieben erhält man, ausgehend von 3-(4-Fluorbenzyl)-3H,6H,7H-1,2,3-triazolo[4,5-d]pyrimidin-7-on, das 7-Chlor-3-(4-fluorbenzyl)-3H-1,2,3-triazolo[4,5-d]pyrimidin und hieraus durch Umsetzung mit Methylamin das 3-(4-Fluorbenzyl)-7-(N-methylamino)-3H-1,2,3-triazolo[4,5-d]pyrimidin vom Smp. 221-223° [aus Aethylacetat/Aethanol (1:1)].

Das 3-(4-Fluorbenzyl)-3H,6H,7H-1,2,3-triazolo[4,5-d]pyrimidin-7-on erhält man z.B. in analoger Weise wie in Beispiel 3 beschrieben durch Erhitzen von 5-Amino-1-(4-fluorbenzyl)-1H-1,2,3-triazol-4-carboxamid und Formamid [Smp.: 230-232° (Rohprodukt)].

Beispiel 12:

Man löst 2,8 g (10 mmol) 5-Amino-7-chlor-3-(2-fluorbenzyl)-3H-1,2,3-triazolo[4,5-d]pyrimidin unter Erwärmen auf 40-50° in 150 ml Aethanol und versetzt die erhaltene Lösung mit 10 ml einer wässrigen Lösung von Dimethylamin (40 %). Man lässt das Reaktionsgemisch 2 Stunden bei Raumtemperatur stehen. Sodann wird das ausgefallene Produkt abgesaugt und mit Wasser gewaschen. Nach Umkristallisation aus Aethanol erhält man das 5-Amino-7-(N,N-dimethylamino)-3-(2-fluorbenzyl)-3H-1,2,3-triazolo[4,5-d]pyrimidin vom Smp. 165-167°.

Das 5-Amino-7-chlor-3-(2-fluorbenzyl)-3H-1,2,3-triazolo[4,5-d]pyrimidin kann beispielsweise wie folgt hergestellt werden:

Man löst 53 g (323 mmol) 2-Amino-4,6-dichlor-pyrimidin, 40,4 g (323 mmol) 2-Fluorbenzylamin und 33,4 g (330 mmol) Triaethylamin in 800 ml Aethanol und erhitzt das Reaktionsgemisch 20 Stunden unter Rückfluss. Nach dem Abdestillieren von 400 ml Aethanol verdünnt man den Rückstand mit 500 ml Wasser, saugt das ausgefallene Produkt ab und kristallisiert dieses aus 1 l Toluol um. Man erhält so das 2-Amino-4-chlor-6-(2-fluorbenzylamino)-pyrimidin, das bei 130-133° schmilzt.

Eine Lösung von 11,5 g (90 mmol) 4-Chloranilin in 33 ml konzentrierter Salzsäure und 80 ml Wasser wird bei 0-5° mit einer Lösung von 6,2 g (90 mmol) Natriumnitrit in 30 ml Wasser diazotiert. Diese Diazoniumsalz-Lösung tropft man sodann bei 14-17° zu einer Mischung von 20,6 g (81,5 mmol) 2-Amino-4-chlor-6-(2-fluorbenzylamino)-pyrimidin und 68 g (500 mmol) kristallinem Natriumacetat in 500 ml 65%iger Essigsäure. Man rührt das Reaktionsgemisch 40 Stunden bei Raumtemperatur, saugt dann das ausgefallene Produkt ab und wäscht es mehrmals mit Wasser nach. Nach Trocknen bei Raumtemperatur über Calciumchlorid erhält man so das 2-Amino-4-chlor-6-(o-fluorbenzylamino)-pyrimidin-5-azo-(4′-chlorbenzol) als gelbes Kristallpulver vom Smp. 215-225° (Zersetzung).

Man suspendiert 59 g (151 mmol) 2-Amino-4-chlor-6-(o-fluorbenzylamino)-pyrimidin-5-azo-(4′-chlorbenzol) in 3 l 50%igem Aethanol und 160 ml Essigsäure und trägt bei 70° während 1 Stunde unter Argon 124 g

(1,9 mol) Zinkstaub portionsweise in die Suspension ein. Man rührt weitere 5 Stunden bei 70° und filtriert dann vom überschüssigen Zink ab. Das Filtrat wird im Vakuum auf die Hälfte des Volumens eingeengt und dann durch Zugabe von 240 ml konzentrierter Natronlauge stark alkalisch gestellt. Das Produkt wird mit Essigester aus dem Gemisch extrahiert. Nach dem Abdestillieren des Essigesters kristallisiert man den Rückstand zuerst aus Toluol, dann aus Acetonitril um. Man erhält so das 4-Chlor-2,5-diamino-6-(2-fluorbenzylamino)-pyrimidin in Form von braunroten Kristallen, die bei 180-182° schmelzen.

Zu einer Suspension von 16 g (60 mmol) 4-Chlor-2,5-diamino-6-(2-fluorbenzylamino)-pyrimidin in 200 ml 25%iger Essigsäure tropft man bei 0° unter kräftigem Rühren eine Lösung von 4,5 g (65 mmol) Natriumnitrit in 100 ml Wasser. Man rührt 2 Stunden bei Raumtemperatur und saugt dann das Produkt ab. Nach Umkristallisation aus Essigester/Hexan erhält man so das 5-Amino-7-chlor-3-(2-fluorbenzyl)-3H-1,2,3-triazolo[4,5-d]pyrimidin vom Smp. 149-151°.

Beispiel 13:

In analoger Weise wie in Beispiel 12 beschrieben erhält man durch Umsetzung von 5-Amino-7-chlor-3-(2-fluorbenzyl)-3H-1,2,3-triazolo[4,5-d]pyrimidin mit Methylamin das 5-Amino-3-(2-fluorbenzyl)-7-(N-methylamino)-3H-1,2,3-triazolo[4,5-d]pyrimidin vom Smp. 252-254° (Dioxan/Toluol).

Beispiel 14:

In analoger Weise wie in Beispiel 3 beschrieben erhält man, ausgehend von 3-(3-Trifluormethylbenzyl)-3H,6H,7H-1,2,3-triazolo[4,5-d]pyrimidin7-on, das 7-Chlor-3-(3-trifluormethylbenzyl)-3H-1,2,3-triazolo[4,5-d]pyrimidin und hieraus durch Umsetzung mit Methylamin das 7-(N-Methylamino)-3-(3-trifluormethylbenzyl)-3H-1,2,3-triazolo[4,5-d]pyrimidin vom Smp. 211-213° (aus Essigester) bzw. durch Umsetzung mit Dimethylamin das 7-(N,N-Di-methylamino)-3-(3-trifluormethylbenzyl)-3H-1,2,3-triazolo[4,5-d]pyrimidin vom Smp. 100-101° (aus Methanol).

Das 3-(3-Trifluormethylbenzyl)-3H,6H,7H-1,2,3-triazolo[4,5-d]pyrimidin-7-on erhält man z.B. in analoger Weise wie in Beispiel 3 beschrieben durch Erhitzen von 5-Amino-1-(3-trifluormethylbenzyl)1H-1,2,3-triazol-4-carboxamid und Formamid (Smp.: 202-204°).

Beispiel 15:

Ein Gemisch von 0,82 g (10 mmol) Dimethylamin-hydrochlorid, 1,27 g (10 mmol) N-Cyclohexyl-N,N-dimethyl-amin und 1,42 g (10 mmol) Phosphorpentoxid erhitzt man während 15 Minuten auf 200° und gibt dann zu der entstandenen Schmelze 0,49 g (2 mmol) 3-(2-Fluorbenzyl)-3H,6H,7H-1,2,3-triazolo[4,5-d]pyrimidin-7-on. Das Reaktionsgemisch wird 2 Stunden bei 200° gehalten, dann auf 100° abgekühlt und mit 25 ml Wasser behandelt. Nach dem Abkühlen auf Raumtemperatur saugt man das ausgefallene Produkt ab, löst es in 25 ml Essigester und trocknet die Lösung über Natriumsulfat. Man dampft die Essigesterlösung ein, kristallisiert den Rückstand aus Toluol/Cyclohexan um und erhält so das 7-(N,N-Dimethylamino)-3-(2-fluorbenzyl)-3H-1,2,3-triazolo[4,5-d]pyrimidin vom Smp. 117-119°.

Das 3-(2-Fluorbenzyl)-3H,6H,7H-1,2,3-triazolo[4,5-d]pyrimidin-7-on erhält man z.B. in analoger Weise wie in Beispiel 3 beschrieben durch Erhitzen von 5-Amino-1-(2-fluorbenzyl)-1H-1,2,3-triazol-4-carboxamid und Formamid.

Beispiel 16:

In analoger Weise wie in Beispiel 12 beschrieben löst man 1,95 g (6,36 mmol) 7-Chlor-5-(N,N-dimethylamino)-3-(2-fluorbenzyl)-3H-1,2,3-triazolo[4,5-d]pyrimidin in 200 ml warmen Aethanol, gibt 7 ml wässrige Dimethylaminlösung (40 %) zu und lässt das Gemisch 30 Minuten stehen. Nach Verdünnen mit 200 ml Wasser saugt man das Produkt ab und kristallisiert es aus Aethanol um. Man erhält so das 5,7-Bis(N,N-dimethylamino)-3-(2-fluorbenzyl)-3H-1,2,3-triazolo[4,5-d]pyrimidin vom Smp. 123-125°.

Das 7-Chlor-5-(N,N-dimethylamino)-3-(2-fluorbenzyl)-3H-1,2,3-triazolo[4,5-d]pyrimidin kann beispielsweise wie folgt hergestellt werden:
Analog Beispiel 12 werden 13,9 g (110 mmol) 2-Fluorbenzylamin, 21-3 g (110 mmol) 4,6-Dichlor-2-(N,N-dimethylamino)-pyrimidin und 11,5 g (114 mmol) Triäthylamin in 300 ml absolutem Aethanol gelöst und das Gemisch 48 Stunden unter Rückfluss erhitzt. Nach dem Abdestillieren des Aethanols versetzt man den Rückstand mit Wasser, saugt das Produkt ab und erhält nach Umkristallisation aus Cyclohexan das 4-Chlor-2-(N,N-dimethylamino)-6-(2-fluorbenzylamino)-pyrimidin vom Smp. 71-73°.

Analog Beispiel 12 erhält man aus 16 g (57 mmol) 4-Chlor-2-(N,N-dimethylamino)-6-(2-fluorbenzylamino)-pyrimidin und 62 mmol p-Chlorphenyldiazoniumchlorid in wässriger Essigsäure mit 46,5 g (340 mmol) kristallinem Natriumacetat das 4-Chlor-2-(N,N-dimethylamino)-6-(o-fluorbenzylamino)-pyrimidin-5-azo-(4′-chlorbenzol) vom Smp. 175-176° (Zersetzung).

Analog Beispiel 12 reduziert man das 4-Chlor-2-(N,N-dimethylamino)-6-(o-fluorbenzylamino)-pyrimidin-5-azo-(4′-chlorbenzol) mit Zinkstaub in Aethanol/Wasser/Essigsäure und erhält so das 5-Amino-4-chlor-2-(N,N-dimethylamino)-6-(2-fluorbenzylamino)-pyrimidin von Smp. 103-105° (aus Cyclohexan).

Analog Beispiel 12 erhält man aus 6,8 g (23 mmol) 5-Amino-4-chlor-2-(N,N-dimethylamino)-6-(2-fluorbenzylamino)-pyrimidin in 150 ml 50%iger Essigsäure durch Umsetzung mit 1,84 g (26,7 mmol) Natriumnitrit das 7-Chlor-5-(N,N-dimethylamino)-3-(2-fluorbenzyl)-3H-1,2,3-triazolo[4,5-d]pyrimidin vom Smp. 153-155° (aus Acetonitril).

Beispiel 17:

In analoger Weise wie in Beispiel 16 beschrieben erhält man unter Verwendung von wässriger Monomethylaminlösung (40 %) das 5-(N,N-Dimethylamino)-3-(2-fluorbenzyl)-7-(N-methylamino)-3H-1,2,3-triazolo[4,5-d]pyrimidin vom Smp. 199-200° (aus Aethanol).

Beispiel 18:

In analoger Weise wie in den Beispielen 1 bis 17 beschrieben, kann man auch
das 7-(N-Methylamino)-3-(2-trifluormethylbenzyl)-3H-1,2,3-triazolo[4,5-d]pyrimidin,
das 7-(N-Methylamino)-3-(4-trifluormethylbenzyl)-3H-1,2,3-triazolo[4,5-d]pyrimidin,
das 3-(2-Cyanobenzyl)-7-(N-methylamino)-3H-1,2,3-triazolo[4,5-d]pyrimidin,
das 3-(3-Cyanobenzyl)-7-(N-methylamino)-3H-1,2,3-triazolo[4,5-d]pyrimidin und
das 3-(4-Cyanobenzyl)-7-(N-methylamino)-3H-1,2,3-triazolo[4,5-d]pyrimidin herstellen.

Beispiel 19:

Tabletten, enthaltend je 50 mg des Wirkstoffs, z.B. das 3-(2-Fluorbenzyl)-7-(N-methylamino)-3H-1,2,3-triazolo[4,5-d]-pyrimidin oder ein pharmazeutisch verwendbares Salz davon, können wie folgt hergestellt werden:

Zusammensetzung (für 10000 Tabletten):

| | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Aethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 20:

Lacktabletten, enthaltend je 200 mg des Wirkstoffs, z.B. das 3-(2-Fluorbenzyl)-7-(N-methylamino)-3H-1,2,3-triazolo[4,5-d]pyrimidin oder ein pharmazeutisch verwendbares Salz davon, können wie folgt hergestellt werden:

Zusammensetzung (für 500 Tabletten):

| | |
|---|---|
| Wirkstoff | 100.00 g |
| Lactose | 100.00 g |

| | |
|---|---|
| Maisstärke | 70.00 g |
| Talk | 8.50 g |
| Calciumstearat | 1.50 |
| Hydroxypropylmethylcellulose | 1.18 g |
| Schellack | 0.32 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 560 mg) verpresst und diese mit einer Lösung der Hydroxypropylmethylcellulose und des Schellacks in Dichlormethan lackiert; Endgewicht der Lacktablette: 563 mg.

Beispiel 21:

In analoger Weise wie in den Beispielen 19 und 20 beschrieben können auch pharmazeutische Präparate, enthaltend eine andere Verbindung der Formel I oder ein pharmazeutisch verwendbares Salz davon, beispielsweise gemäss den Beispielen 1 bis 18, hergestellt werden.

## Patentansprüche

### Patentansprüche für folgende Verstragsstaat: AT, BE, CH, DE, FR, GB, IT, LI, LU, NL, SE

1. Eine Verbindung der Formel

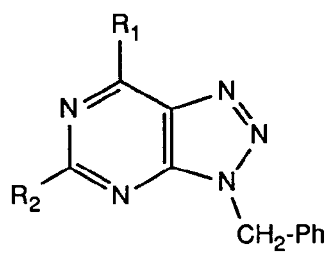

(I),

worin Ph einen durch Halogen mit einer Atomnummer bis und mit 35, $C_1$-$C_7$-Alkyl, Trifluormethyl und/oder Cyano substituierten Phenylrest bedeutet, $R_1$ Amino, N-Mono-$C_1$-$C_7$-alkylamino, N,N-Di-$C_1$-$C_7$-alkylamino, worin die beiden N-Alkylgruppen gleich oder verschieden sein können, N-($C_1$-$C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$-alkyl-amino, N-Mono($C_3$-$C_8$-Cycloalkyl-$C_1$-$C_7$-alkyl)amino, N,N-Di($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkylamino darstellt und $R_2$ für Wasserstoff, $C_1$-$C_7$-Alkyl, Amino, N-Mono-$C_1$-$C_7$-alkylamino, N,N-Di-$C_1$-$C_7$-alkylamino, worin die beiden N-Alkylgruppen gleich oder verschieden sein können, N-($C_1$-$C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$-alkyl-amino, N-Mono($C_3$-$C_8$-Cycloalkyl-$C_1$-$C_7$-alkyl)amino, N,N-Di($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkyl-amino steht, in freier Form oder in Salzform, mit der Massgabe, dass in einer Verbindung der Formel I in freier Form, worin $R_1$ N,N-Di-$C_1$-$C_6$-alkylamino, worin die beiden N-$C_1$-$C_6$-Alkylgruppen gleich oder verschieden sind, N-Mono-$C_1$-$C_6$-alkylamino oder Amino bedeutet, $R_2$ von Wasserstoff und von $C_1$-$C_6$-Alkyl verschieden ist, wenn Ph durch Halogen mit einer Atomnummer bis und mit 35 oder durch Trifluormethyl einfach substituiertes Phenyl darstellt, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder

worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist.

2. Eine Verbindung der Formel I gemäss Anspruch 1, worin, unter Berücksichtigung der in Anspruch 1 erwähnten Massgabe, Ph einen durch mindestens ein Halogenatom mit einer Atomnummer bis und mit 35 substituierten Phenylrest bedeutet, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

3. Eine Verbindung der Formel I gemäss Anspruch 1, worin, unter Berücksichtigung der in Anspruch 1 erwähnten Massgabe, Ph 2-, 3- oder 4-Halogenphenyl, 2,3-, 2,5- oder 2,6-Dihalogenphenyl, 2-, 3- oder 4-$C_1$-$C_4$-Alkylphenyl, 2-, 3- oder 4-Trifluormethylphenyl oder 2-, 3- oder 4-Cyanophenyl bedeutet, wobei Halogen jeweils Halogen mit einer Atomnummer bis und mit 35 sein kann, $R_1$ Amino, N-Mono-$C_1$-$C_4$-alkylamino, N,N-Di-$C_1$-$C_4$-alkylamino, N-$C_3$-$C_6$-Cycloalkylamino oder N-$C_2$-$C_5$-Alkanoylamino darstellt und $R_2$ für Wasserstoff, $C_1$-$C_4$-Alkyl, Amino, N-Mono-$C_1$-$C_4$-alkylamino, N,N-Di-$C_1$-$C_4$-alkylamino, N-$C_3$-$C_6$-Cycloalkylamino oder N-$C_2$-$C_5$-Alkanoyl amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist.

4. Eine Verbindung der Formel I gemäss Anspruch 3, worin, unter Berücksichtigung der in Anspruch 1 erwähnten Massgabe, Ph 2-Halogenphenyl, das zusätzlich in 3-, 5- oder 6-Position durch Halogen substituiert sein kann, bedeutet, wobei Halogen jeweils Halogen mit einer Atomnummer bis und mit 35 sein kann, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

5. Eine Verbindung der Formel I gemäss Anspruch 3, worin, unter Berücksichtigung der in Anspruch 1 erwähnten Massgabe, Ph 2- oder 3-Fluorphenyl, 2-Chlorphenyl, 2,6-Difluorphenyl oder 2-$C_1$-$C_4$-Alkylphenyl bedeutet, $R_1$ N-Mono-$C_1$-$C_4$-alkylamino, N,N-Di-$C_1$-$C_4$-alkylamino oder N-$C_3$-$C_6$-Cycloalkylamino darstellt und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino ist und $R_2$ Wasserstoff oder Methyl ist oder worin Ph o-Fluorphenyl oder o-Chlorphenyl ist, $R_1$ N,N-Dimethylamino ist und $R_2$ Wasserstoff ist oder worin Ph m-Fluorphenyl oder o-Chlorphenyl ist, $R_1$ N-Monomethylamino ist und $R_2$ Wasserstoff ist.

6. Eine Verbindung der Formel I gemäss Anspruch 4, worin, unter Berücksichtigung der in Anspruch 1 erwähnten Massgabe, Ph 2-Fluorphenyl, das zusätzlich in 5- oder 6-Position durch Halogen mit einer Atomnummer bis und mit 35 subsstituiert sein kann, bedeutet, $R_1$ Amino, N-Mono-$C_1$-$C_4$-alkylamino, N,N-Di-$C_1$-$C_4$-alkylamino oder N-$C_3$-$C_6$-Cycloalkylamino darstellt und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

7. Eine Verbindung der Formel I gemäss Anspruch 6, worin, unter Berücksichtigung der in Anspruch 1 erwähnten Massgabe, Ph 2-Fluor- oder 2,6-Difluorphenyl bedeutet, $R_1$ Amino, N-Mono-$C_1$-$C_4$-alkylamino oder N,N-Di-$C_1$-$C_4$-alkylamino darstellt und $R_2$ für Wasserstoff oder $C_1$-$C_4$-Alkyl steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

8. Eine Verbindung der Formel I gemäss Anspruch 6, worin, unter Berücksichtigung der in Anspruch 1 erwähnten Massgabe, Ph 2-Fluorphenyl bedeutet, $R_1$ N-Mono-$C_1$-$C_4$-alkylamino oder N,N-Di-$C_1$-$C_4$-alkylamino darstellt und $R_2$ für Wasserstoff oder Amino steht, in freier Form oder in Salzform, sowie eine Verbindung der Formel I in freier Form, worin entweder Ph o-Fluorphenyl ist, $R_1$ N-Monomethylamino ist und $R_2$ Wasserstoff ist oder worin Ph o-Fluorphenyl ist, $R_1$ N,N-Dimethylamino ist und $R_2$ Wasserstoff ist.

9. 3-(2-Fluorbenzyl)-7-(N-methylamino)-3H-1,2,3-triazolo[4,5-d]pyrimidin oder ein Salz davon.

10. 7-(N,N-Dimethylamino)-3-(2-fluorbenzyl)-3H-1,2,3-trizolo[4,5-d] pyrimidin, 3-(2,6-Difluorbenzyl)-7-(N-methylamino)-3H-1,2,3-triazolo[4,5-d]pyrimidin oder 3-(2-Fluorbenzyl)-5-methyl-7-(N-methylamino)-3H-1,2,3-triazolo[4,5-d]pyrimidin oder jeweils ein Salz davon.

11. 3-(2-Chlorbenzyl-7- N-methylamino)-3H-1,2,3-triazolo[4,5-d]pyrimidin,3-(2-Chlorbenzyl)-7-(N,N-dimethylamino)-3H-1,2,3-triazolo[4,5-d]pyrimidin, 7-(N-Methylamino)-3-(2-methylbenzyl)-3H-1,2,3-triazolo[4,5-d]pyrimidin, 3-(3-Fluorbenzyl)-7-(N-methylamino)-3H-1,2,3-triazolo[4,5-d]pyrimidin oder 5-Amino-7-(N,N-dimethylamino)-3-(2-fluorbenzyl)-3H-1,2,3-triazolo[4,5-d]pyrimidin oder jeweils ein Salz davon.

12. 7-Amino-3-(2-fluorbenzyl)-3H-1,2,3-triazolo[4,5-d]pyrimidin,7-Amino-3-(2-fluorbenzyl)-5-methyl-3H-1,2,3-triazolo[4,5-d]pyrimidin, 7-(N-Acetylamino)-3-(2-fluorbenzyl)-3H-1,2,3-triazolo[4,5-d]pyrimidin oder 7-(N-Acetyl-N-methyl-amino)-3-(2-fluorbenzyl)-3H-1,2,3-triazolo[4,5-d]pyrimidin oder jeweils ein Salz davon.

13. 3-(4-Fluorbenzyl)-7-(N-methylamino)-3H-1,2,3-triazolo[4,5-d]pyrimidin, 5-Amino-3-(2-fluorbenzyl)-7-(N-methylamino)-3H-1,2,3-triazolo[4,5-d]pyrimidin, 7-(N-Methylamino)-3-(3-trifluormethylbenzyl)-3H-1,2,3-triazolo[4,5-d]pyrimidin, 7-(N,N-Dimethylamino)-3-(3-trifluormethylbenzyl)-3H-1,2,3-triazolo[4,5-d]pyrimidin, 5,7-Bis(N,N-dimethylamino)3-(2-fluorbenzyl)-3H-1,2,3-triazolo[4,5-d]pyrimidin, 5-(N,N-Dimethylamino)-3-(2-fluorbenzyl)-7-(N-methylamino)-3H-1,2,3-triazolo[4,5-d]pyrimidin, 7-(N-methylamino)-3-(2-trifluormethylenzyl)-3H-1,2,3-triazolo[4,5-d]pyrimidin, 7-(N-Methylamino)-3-(4-trifluormethylbenzyl)-3H-1,2,3-triazolo[4,5-d]pyrimidin, 3-(2-Cyanobenzyl)-7-(N-methylamino)-3H-1,2,3-triazolo[4,5-d]pyrimidin, 3-(3-Cyanobenzyl)-7-(N-methylamino)-3H-1,2,3-triazolo[4,5-d]pyrimidin oder 3-(4-Cyanobenzyl)-7-(N-methylamino)3H-1,2,3-triazolo[4,5-d]pyrimidin oder jeweils ein Salz davon.

14. Eine Verbindung gemäss einem der Ansprüche 1 bis 13 oder ein pharmazeutisch verwendbares Salz davon zur Anwendung in einem Verfahren zur prophylatitischen und/oder therapeutischen Behandlung des tierischen oder menschlichen Körpers.

15. Eine Verbindung gemäss einem der Ansprüche 2, 4, 6, 7, 9, 10 und 12 oder ein pharmazeutisch verwendbares Salz davon zur Anwendung in einem Verfahren zur prophylaktischen und/oder therapeutischen Behandlung des tierischen oder menschlichen Körpers.

16. Eine Verbindung gemäss einem der Ansprüche 1 bis 13 zur Anwendung als Antikonvulsivum.

17. Eine Verbindung gemäss einem der Ansprüche 2, 4, 6, 7, 9, 10 und 12 zur Anwendung als Antikonvulsium.

18. Ein pharmazeutisches Präparat, als Wirkstoff enthaltend eine Verbindung gemäss einem der Ansprüche 1 bis 13 oder ein pharmazeutisch Verwendbares Salz davon, gegebenenfalls neben üblichen pharmazeutischen Hilfsstoffen.

19. Ein pharmazeutisches Präparat, als Wirkstoff enthaltend eine Verbindung gemäss einem der Ansprüche 2, 4, 6, 7, 9, 10, und 12 oder ein pharmazeutisch verwendbares Salz davon, gegebenenfalls neben üblichen pharmazeutischen Hilfsstoffen.

20. Ein antikonvulsiv wirksames pharmazeutisches Präparat gemäss Anspruch 18, dadurch gekennzeichnet, dass man einen antikonvulsiven Wirkstoff wählt.

21. Ein antikonvulsiv wirksames pharmazeutisches Präparat gemäss Anspruch 19, dadurch gekennzeichnet, dass man einen antikonvulsiven Wirkstoff wählt.

22. Verfahren zur Herstellung einer Verbindung der Formel I oder eines Salzes davon gemäss einem der Ansprüche 1 bis 13, dadurch gekennzeichnet, dass man

a) in einer Verbindung der Formel

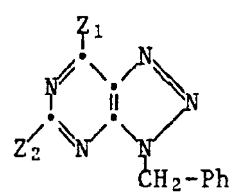

(II),

worin $Z_1$ eine nucleofuge Abgangsgruppe X, ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abganggruppe $X_2$ steht, oder einem Tautomeren und/oder Salz davon $X_1$ durch Umsetzung mit einer Verbindung der Formel H-$R_1$ (IIb) unter Abspaltung einer Verbindung $X_1$-H in $R_1$ und/oder $X_2$ durch Umsetzung einer Verbindung der Formel H-$R_2$(IIg) unter Abspaltung einer Verbindung $X_2$-H in $R_2$ überführt oder

b) aus einer Verbindung der Formel

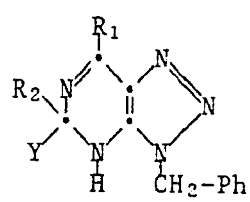

(III),

worin Y Hydroxy, Mercapto oder gegebenenfalls aliplatisch substituiertes Amino bedeutet, oder einem Tautomeren und/oder Salz davon die Verbindung Y-H eliminiert oder

c) ein Salz der Formel

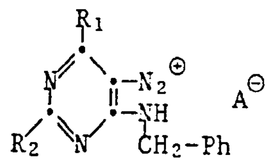

(V),

worin A für das Anion einer Protonensäure steht, cyclisiert oder

d) eine verbindung der Formel

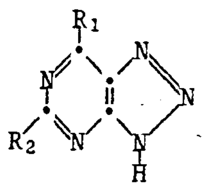

(VI)

oder ein Salz davon mit einer Verbindung der Formel $X_1$-$CH_2$-Ph (VII), worin $X_1$ eine nucleofuge Abgangsgruppe darstellt, umsetzt und

e) gewünschtenfalls jeweils ein gegebenenfalls verfahrensgemäss erhältliches Isomerengemisch in die Komponenten auftrennt und das Isomere der Formel I isoliert und

f) gewünschtenfalls jeweils eine verfahrensgemäss oder auf andere Weise erhältliche Verbindung I in eine andere Verbindung I überführt und

g) gewünschtenfalls jeweils eine verfahrensgemäss erhältliche freie Verbindung I in ein Salz übeführt oder ein verfahrensgemäss erhältliches Salz einer Verbindung I in die freie Verbindung I oder in ein anderes Salz der Verbindung I umwandelt und

h) gewünschtenfalls jeweils ein gegebenenfalls verfährensgemäss erhältliches Stereoisomerengemisch in die Stereoisomeren auftrennt und das gewünschte Stereoisomere isoliert.

23. Verfahren zur Herstellung einer Verbindung der Formel I, worin $R_2$ für Amino steht, oder eines Salzes davon gemäss einem der Ansprüche 1 bis 6, 8, 11 und 13, dadurch gekennzeichnet, dass man

a) eine Verbindung der Formel

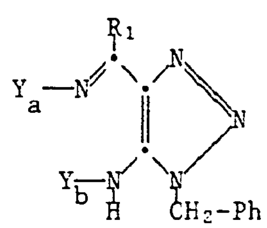

(IV),

worin einer der Reste $Y_a$ und $Y_b$ Cyano und der andere Wasserstoff bedeutet, oder ein Tautomeres und/oder Salz davon cyclisiert und

b) gewünschtenfalls ein gegebenenfalls verfährensgemäss erhältliches Isomerengemisch in die Komponenten auftrennt und das Isomere der Formel I isoliert und

c) gewünschtenfalls eine verfahrensgemäss erhältliche Verbindung I in eine andere Verbindung I überführt und

d) gewünschtenfalls eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und

e) gewünschtenfalls ein gegebenenfalls verfahrensgemäss erhältliches Stereoisomerengemisch in die Stereoisomeren auftrennt und das gewünschte Stereoisomere isoliert.

24. Verwendung einer Verbindung gemäss einem der Ansprüche 1 bis 13 oder eines pharmazeutisch verwendbaren Salzes davon zur Herstellung eines pharmazeutischen Präparates.

25. Verwendung einer Verbindung gemäss Anspruch 24 zur Herstellung eines Antikonvulsivums.

**Patenansprüche für folgende Vertragsstaaten: ES, GR**

1. Verfahren zur Herstellung einer Verbindung der Formel

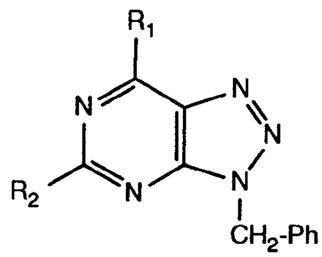
(I),

worin Ph einen durch Halogen mit einer Atomnummer bis und mit 35, $C_1$-$C_7$-Alkyl, Trifluormethyl und/oder Cyano substituierten Phenylrest bedeutet, $R_1$ Amino, N-Mono-$C_1$-$C_7$-alkylamino, N,N-Di-$C_1$-$C_7$-alkylamino, worin die beiden N-Alkylgruppen gleich oder verschieden sein können, N-($C_1C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$-alkyl-amino, N-Mono($C_3$-$C_8$-Cycloalkyl)-$C_1$-$C_7$-alkyl)amino, N,N-Di($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkylamino darstellt und $R_2$ für Wasserstoff, $C_1$-$C_7$-Alkyl, Amino, N-Mono-$C_1$-$C_7$-alkylamino, N,N-Di-$C_1$-$C_7$-alkylamino, worin die beiden N-Alkylgruppen gleich oder verschieden sein können, N-($C_1$-$C_4$-Alkoxy-$C_1$-$C_7$-alkyl)amino, N-(Hydroxy-$C_1$-$C_7$-alkyl)amino, N-(Hydroxy-$C_1$-$C_7$-alkyl)-N-$C_1$-$C_7$-alkyl-amino, N-Mono-$C_3$-$C_8$-cycloalkylamino, N,N-Di-$C_3$-$C_8$-cycloalkylamino, worin die beiden N-Cycloalkylgruppen gleich oder verschieden sein können, N-$C_3$-$C_8$-Cycloalkyl-N-$C_1$-$C_7$-alkyl-amino, N-Mono($C_3$-$C_8$-Cycloalkyl-$C_1$-$C_7$-alkyl)amino, N,N-Di($C_3$-$C_8$-cycloalkyl-$C_1$-$C_7$-alkyl)amino, worin die beiden N-Cycloalkylalkylgruppen gleich oder verschieden sein können, N-($C_3$-$C_8$-Cycloalkyl-$C_1$-$C_7$-alkyl)-N-$C_1$-$C_7$-alkyl-amino, N-$C_2$-$C_5$-Alkanoylamino oder N-$C_2$-$C_5$-Alkanoyl-N-$C_1$-$C_7$-alkyl-amino steht, in freier Form oder in Salzform, mit der Massgabe, dass in einer Verbindung der Formel I in freier Form, worin $R_1$ N,N-Di-$C_1$-$C_6$-alkylamino, worin die beiden N-$C_1$-$C_6$-Alkylgruppen gleich oder verschieden sind, N-Mono-$C_1$-$C_6$-alkylamino oder Amino bedeutet, $R_2$ von Wasserstoff und von $C_1$-$C_6$-Alkyl verschieden ist, wenn Ph durch Halogen mit einer Atomnummer bis und mit 35 oder durch Trifluormethyl einfach substituiertes Phenyl darstellt, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist, oder eines Salzes davon, dadurch gekennzeichnet, dass man

a) in einer Verbindung der Formel

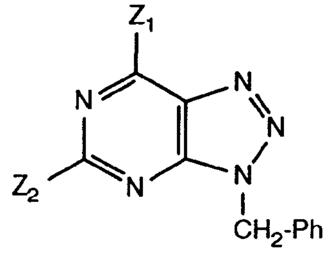
(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder einem Tautomeren und/oder Salz davon $X_1$ durch Umsetzung mit einer Verbindung der Formel H-$R_1$ (IIb) unter Abspaltung einer Verbindung $X_1$-H in $R_1$ und/oder $X_2$ durch Umsetzung mit einer Verbindung der Formel H-$R_2$ (IIj) unter Abspaltung einer Verbindung $X_2$-H in $R_2$ überführt oder

b) aus einer Verbindung der Formel

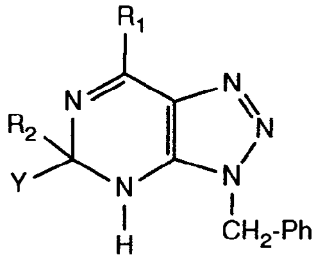

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) ein Salz der Formel

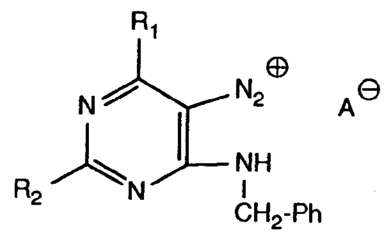

(V),

worin A für das Anion einer Protonensäure steht, cyclisiert oder

d) eine Verbindung der Formel

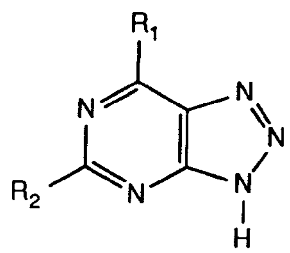

(VI)

oder ein Salz davon mit einer Verbindung der Formel $X_1$-$CH_2$-Ph (VII), worin $X_1$ eine nucleofuge Abgangsgruppe darstellt, umsetzt und

e) gewünschtenfalls jeweils ein gegebenenfalls verfahrensgemäss erhältliches Isomerengemisch in die Komponenten auftrennt und das Isomere der Formel I isoliert und

f) gewünschtenfalls jeweils eine verfahrensgemäss oder auf andere Weise erhältliche Verbindung I in eine andere Verbindung I überführt und

g) gewünschtenfalls jeweils eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und

h) gewünschtenfalls jeweils ein gegebenenfalls verfahrensgemäss erhältliches Stereoisomerengemisch in die Stereoisomeren auftrennt und das gewünschte Stereoisomere isoliert.

2. Verfahren gemäss Anspruch 1 zur Herstellung einer Verbindung der Formel I, worin, unter Berücksichtigung der in Anspruch 1 erwähnten Massgabe, Ph einen durch mindestens ein Halogenatom mit einer Atomnummer bis und mit 35 substituierten Phenylrest bedeutet, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

3. Verfahren gemäss Anspruch 1 zur Herstellung einer Verbindung der Formel I, worin, unter Berücksichtigung der in Anspruch 1 erwähnten Massgabe, Ph 2-, 3- oder 4-Halogenphenyl, 2,3-, 2,5- oder 2,6-Dihalogenphenyl, 2-, 3- oder 4-$C_1$-$C_4$-Alkylphenyl, 2-, 3- oder 4-Trifluormethylphenyl oder 2-, 3- oder 4-Cyanophenyl bedeutet, wobei Halogen jeweils Halogen mit einer Atomnummer bis und mit 35 sein kann, $R_1$ Amino, N-Mo-

no-$C_1$-$C_4$-alkylamino, N,N-Di-$C_1$-$C_4$-alkylamino, N-$C_3$-$C_6$-Cycloalkylamino oder N-$C_2$-$C_5$-Alkanoylamino darstellt und $R_2$ für Wasserstoff, $C_1$-$C_4$-Alkyl, Amino, N-Mono-$C_1$-$C_4$-alkylamino, N,N-Di-$C_1$-$C_4$-alkylamino, N-$C_3$-$C_6$-Cycloalkylamino oder N-$C_2$-$C_5$-Alkanoyl amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl, o-Chlorphenyl oder m-Trifluormethylphenyl ist, $R_1$ N,N-Dimethylamino ist und $R_2$ Wasserstoff ist oder worin Ph m-Fluorphenyl, p-Fluorphenyl, o-Chlorphenyl, o-Trifluormethylphenyl, m-Trifluormethylphenyl oder p-Trifluormethylphenyl ist, $R_1$ N-Monomethylamino ist und $R_2$ Wasserstoff ist.

4. Verfahren gemäss Anspruch 3 zur Herstellung einer Verbindung der Formel I, worin, unter Berücksichtigung der in Anspruch 1 erwähnten Massgabe, Ph 2-Halogenphenyl, das zusätzlich in 3-, 5- oder 6-Position durch Halogen substituiert sein kann, bedeutet, wobei Halogen jeweils Halogen mit einer Atomnummer bis und mit 35 sein kann, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

5. Verfahren gemäss Anspruch 3 zur Herstellung einer Verbindung der Formel I, worin, unter Berücksichtigung der in Anspruch 1 erwähnten Massgabe, Ph 2- oder 3-Fluorphenyl, 2-Chlorphenyl, 2,6-Difluorphenyl oder 2-$C_1$-$C_4$-Alkylphenyl bedeutet, $R_1$ N-Mono-$C_1$-$C_4$-alkylamino, N,N-Di-$C_1$-$C_4$-alkylamino oder N-$C_3$-$C_6$-Cycloalkylamino darstell und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino ist und $R_2$ Wasserstoff oder Methyl ist oder worin Ph o-Fluorphenyl oder o-Chlorphenyl ist, $R_1$ N,N-Dimethylamino ist und $R_2$ Wasserstoff ist oder worin Ph m-Fluorphenyl oder o-Chlorphenyl ist, $R_1$ N-Monomethylamino ist und $R_2$ Wasserstoff ist.

6. Verfahren gemäss Anspruch 4 zur Herstellung einer Verbindung der Formel I, worin, unter Berücksichtigung der in Anspruch 1 erwähnten Massgabe, Ph 2-Fluorphenyl, das zusätzlich in 5- oder 6-Position durch Halogen mit einer Atomnummer bis und mit 35 substituiert sein kann, bedeutet, $R_1$ Amino, N-Mono-$C_1$-$C_4$-alkylamino, N,N-Di-$C_1$-$C_4$-alkylamino oder N-$C_3$-$C_6$-Cycloalkylamino darstellt und $R_2$ für Wasserstoff, $C_1$-$C_4$-Alkyl oder Amino steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

7. Verfahren gemäss Anspruch 6 zur Herstellung einer Verbindung der Formel I, worin, unter Berücksichtigung der in Anspruch 1 erwähnten Massgabe, Ph 2-Fluor- oder 2,6-Difluorphenyl bedeutet, $R_1$ Amino, N-Mono-$C_1$-$C_4$-alkylamino oder N,N-Di-$C_1$-$C_4$-alkylamino darstellt und R2 für Wasserstoff oder $C_1$-$C_4$-Alkyl steht, in freier Form oder in Salzform, sowie eine Verbindung der Formel I in freier Form, worin entweder Ph o-Fluorphenyl ist, $R_1$ N-Monomethylamino oder Amino ist und $R_2$ Wasserstoff oder Methyl ist oder worin Ph o-Fluorphenyl ist, $R_1$ N,N-Dimethylamino ist und $R_2$ Wasserstoff ist.

8. Verfahren gemäss Anspruch 6 zur Herstellung einer Verbindung der Formel I, worin, unter Berücksichtigung der in Anspruch 1 erwähnten Massgabe, Ph 2-Fluorphenyl bedeutet, $R_1$ N-Mono-$C_1$-$C_4$-alkylamino oder N,N-Di-$C_1$-$C_4$-alkylamino darstellt und $R_2$ für Wasserstoff oder Amino steht, in freier Form oder in Salzform, sowie eine Verbindung der Formel I in freier Form, worin entweder Ph o-Fluorphenyl ist, $R_1$ N-Monomethyl amino ist und $R_2$ Wasserstoff ist oder worin Ph o-Fluorphenyl ist, $R_1$ N,N-Dimethylamino ist und $R_2$ Wasserstoff ist.

9. Verfahren zur Herstellung einer Verbindung der Formel (I) gemäss einem der Ansprüche 1 bis 8, worin $R_2$ für Amino steht, oder eines Salzes davon, dadurch gekennzeichnet, dass man

a) eine Verbindung der Formel

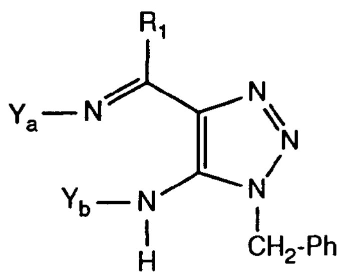

(IV),

worin einer der Reste Ya und Yb Cyano und der andere Wasserstoff bedeutet, oder ein Tautomeres und/oder Salz davon cyclisiert und

b) gewünschtenfalls jeweils ein gegebenenfalls verfahrensgemäss erhältliches Isomerengemisch in die

Komponenten auftrennt und das Isomere der Formel I isoliert und
c) gewünschtenfalls jeweils eine verfahrensgemäss oder auf andere Weise erhältliche Verbindung I in eine andere Verbindung I überführt und
d) gewünschtenfalls jeweils eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und
e) gewünschtenfalls jeweils ein gegebenenfalls verfahrensgemäss erhältliches Stereoisomerengemisch in die Stereoisomeren auftrennt und das gewünschte Stereoisomere isoliert.

10. Verfahren gemäss Anspruch 1 oder 9 zur Herstellung von 3-(2-Fluorbenzyl)-7-(N-methylamino)-3H-1,2,3-triazolo[4,5-d]pyrimidin oder eines Salz davon.

11. Verfahren gemäss Anspruch 1 oder 9 zur Herstellung von
7-(N,N-Dimethylamino)-3-(2-fluorbenzyl)-3H-1,2,3-triazolo[4,5-d]pyrimidin,
3-(2,6-Difluorbenzyl)-7-(N-methylamino)-3H-1,2,3-triazolo[4,5-d]pyrimidin,
3-(2-Fluorbenzyl)-5-methyl-7-(N-methylamino)-3H-1,2,3-triazolo[4,5-d]pyrimidin oder jeweils eines Salzes davon.

12. Verfahren gemäss Anspruch 1 oder 9 zur Herstellung von
3-(2-Chlorbenzyl)-7-(N-methylamino)-3H-1,2,3-triazolo[4,5-d]pyrimidin,
3-(2-Chlorbenzyl)-7-(N,N-dimethylamino)-3H-1,2,3-triazolo[4,5-d]-pyrimidin,
7-(N-Methylamino)-3-(2-methylbenzyl)-3H-1,2,3-triazolo[4,5-d]-pyrimidin,
3-(3-Fluorbenzyl)-7-(N-methylamino)-3H-1,2,3-triazolo[4,5-d]-pyrimidin oder
5-Amino-7-(N,N-dimethylamino)-3-(2-fluorbenzyl)-3H-1,2,3-triazolo[4,5-d]pyrimidin oder jeweils eines Salzes davon.

13. Verfahren gemäss Anspruch 1 oder 9 zur Herstellung von
7-Amino-3-(2-fluorbenzyl)-3H-1,2,3-triazolo[4,5-d]pyrimidin,
7-Amino-3-(2-fluorbenzyl)-5-methyl-3H-1,2,3-triazolo[4,5-d]pyrimidin,
7-(N-Acetylamino)-3-(2-fluorbenzyl)-3H-1,2,3-triazolo[4,5-d]pyrimidin oder
7-(N-Acetyl-N-methyl-amino)-3-(2-fluorbenzyl)-3H-1,2,3-triazolo[4,5-d]-pyrimidin oder jeweils eines Salzes davon.

14. Verfahren gemäss Anspruch 1 oder 9 zur Herstellung von
3-(4-Fluorbenzyl)-7-(N-methylamino)-3H-1,2,3-triazolo[4,5-d]pyrimidin,
5-Amino-3-(2-fluorbenzyl)-7-(N-methylamino)-3H-1,2,3-triazolo[4,5-d]pyrimidin,
7-(N-Methylamino)-3-(3-trifluormethylbenzyl)-3H-1,2,3-triazolo[4,5-d]pyrimidin,
7-(N,N-Dimethylamino)-3-(3-trifluormethylbenzyl)-3H-1,2,3-triazolo-[4,5-d]pyrimidin,
5,7-Bis(N,N-dimethylamino)-3-(2-fluorbenzyl)-3H-1,2,3-triazolo[4,5-d]pyrimidin,
5-(N,N-Dimethylamino)-3-(2-fluorbenzyl)-7-(N-methylamino)-3H-1,2,3-triazolo[4,5-d]pyrimidin,
7-(N-Methylamino)-3-(2-trifluormethylbenzyl)-3H-1,2,3-triazolo[4,5-d]pyrimidin,
7-(N-Methylamino)-3-(4-trifluormethylbenzyl)-3H-1,2,3-triazolo[4,5-d]pyrimidin,
3-(2-Cyanobenzyl)-7-(N-methylamino)-3H-1,2,3-triazolo[4,5-d]-pyrimidin,
3-(3-Cyanobenzyl)-7-(N-methylamino)-3H-1,2,3-triazolo[4,5-d]pyrimidin,
3-(4-Cyanobenzyl)-7-(N-methylamino)-3H-1,2,3-triazolo[4,5-d]pyrimidin oder jeweils eines Salzes davon.

15. Verfahren zur Herstellung eines pharmazeutischen Präparates, dadurch gekennzeichnet, dass man eine Verbindung, erhältlich gemäss einem der Ansprüche I bis 14, oder ein pharmazeutisch verwendbares Salz davon mit üblichen pharmazeutischen Hilfsstoffen vermischt.

16. Verfahren zur Herstellung eines pharmazeutischen Präparates, dadurch gekennzeichnet, dass man eine Verbindung, erhältlich gemäss einem der Ansprüche 2, 4, 6, 7, 9, 10 und 12 oder ein pharmazeutisch verwendbares Salz davon mit üblichen pharmazeutischen Hilfsstoffen, vermischt.

17. Verwendung einer Verbindung, erhältlich gemäss einem der Ansprüche 1 bis 14, oder eines pharmazeutisch verwendbaren Salzes davon zur Herstellung eines pharmazeutischen Präparates.

18 . Verwendung einer Verbindung gemäss Anspruch 17 zur Herstellung eines Antikonvulsivums.

## Claims

### Claims for the following Contracting States: AT, BE, CH, DE, FR, GB, IT, LI, LU, NL, SE

1. A compound of formula

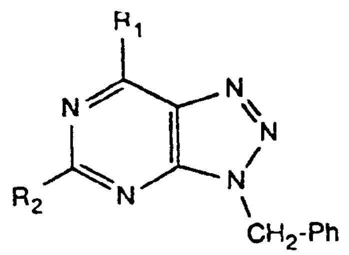

(I),

wherein Ph represents a phenyl radical substituted by halogen having an atomic number of up to and including 35, $C_1$-$C_7$alkyl, trifluoromethyl and/or by cyano, $R_1$ represents amino, N-mono-$C_1$-$C_7$alkylamino, N,N-di-$C_1$-$C_7$alkylamino wherein the two N-alkyl groups may be the same or different, N-($C_1$-$C_4$alkoxy-$C_1$-$C_7$alkyl)amino, N-(hydroxy-$C_1$-$C_7$alkyl)amino, N-(hydroxy-$C_1$-$C_7$alkyl)-N-$C_1$-$C_7$alkylamino, N-mono-$C_3$-$C_8$cycloalkylamino, N,N-di-$C_3$-$C_8$cycloalkylamino wherein the two N-cycloalkyl groups may be the same or different, N-$C_3$-$C_8$cycloalkyl-N-$C_1$-$C_7$alkylamino, N-mono($C_3$-$C_8$cycloalkyl-$C_1$-$C_7$alkyl)amino, N,N-di($C_3$-$C_8$cycloalkyl-$C_1$-$C_7$alkyl)amino wherein the two N-cycloalkylalkyl groups may be the same or different, N-($C_3$-$C_8$cycloalkyl-$C_1$-$C_7$ alkyl)-N-$C_1$-$C_7$alkylamino, N-$C_2$-$C_5$-alkanoylamino or N-$C_2$-$C_5$alkanoyl-N-$C_1$-$C_7$alkylamino and $R_2$ represents hydrogen, $C_1$-$C_7$alkyl, amino, N-mono-$C_1$-$C_7$alkylamino, N,N-di-$C_1$-$C_7$alkylamino wherein the two N-alkyl groups may be the same or different, N-($C_1$-$C_4$-alkoxy-$C_1$-$C_7$alkyl)amino, N-(hydroxy-$C_1$-$C_7$alkyl)amino, N-(hydroxy-$C_1$-$C_7$alkyl)-N-$C_1$-$C_7$alkylamino, N-mono-$C_3$-$C_8$cycloalkylamino, N,N-di-$C_3$-$C_8$cycloalkylamino wherein the two N-cycloalkyl groups may be the same or different, N-$C_3$-$C_8$cycloalkyl-N-$C_1$-$C_7$alkylamino, N-mono($C_3$-$C_8$cycloalkyl-$C_1$-$C_7$alkyl)amino, N,N-di($C_3$-$C_8$cycloalkyl-$C_1$-$C_7$alkyl)amino wherein the two N-cycloalkylalkyl groups may be the same or different, N-($C_3$-$C_8$cycloalkyl-$C_1$-$C_7$alkyl)-N-$C_1$-$C_7$alkylamino, N-$C_2$-$C_5$alkanoylamino or N-$C_2$-$C_5$alkanoyl-N-$C_1$-$C_7$alkylamino, in free form or in salt form, with the proviso that in a compound of formula I in free form in which $R_1$ represents N,N-di-$C_1$-$C_6$alkylamino wherein the two N-$C_1$-$C_6$alkyl groups are the same or different, N-mono-$C_1$-$C_6$alkylamino or amino, $R_2$ is other than hydrogen or $C_1$-$C_6$alkyl when Ph represents phenyl monosubstituted by halogen having an atomic number of up to and including 35 or by trifluoromethyl, and a compound of formula I in free form wherein Ph is o-fluorophenyl, $R_1$ is n-nonomethylamino or amino and $R_2$ is hydrogen or methyl, or Ph is o-fluorophenyl, o-chlorophenyl or m-trifluoromethylphenyl, $R_1$ is N,N-dimethylamino and $R_2$ is hydrogen, or Ph is m-fluorophenyl, p-fluorophenyl, o-chlorophenyl, o-trifluoromethylphenyl, m-trifluoronethylphenyl or p-trifluoronethylphenyl, $R_1$ is N-mononethylamino and $R_2$ is hydrogen.

2. A compound of formula I according to claim 1 wherein, taking into consideration the proviso mentioned in claim 1, Ph represents a phenyl radical substituted by at least one halogen atom having an atomic number of up to and including 35, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

3. A compound of formula I according to claim 1 wherein, taking into consideration the proviso mentioned in claim 1, Ph is 2-, 3- or 4-halophenyl, 2,3-, 2,5- or 2,6-dihalophenyl, 2-, 3- or 4-$C_1$-$C_4$alkylphenyl, 2-, 3- or 4-trifluoromethylphenyl or 2-, 3- or 4-cyanophenyl, it being possible for halogen in each case to be halogen having an atomic number of up to and including 35, $R_1$ is amino, N-mono-$C_1$-$C_4$alkylamino, N,N-di-$C_1$-$C_4$alkylamino, N-$C_3$-$C_6$cycloalkylamino or N-$C_2$-$C_5$alkanoylanino, and $R_2$ is hydrogen, $C_1$-$C_4$alkyl, amino, N-mono-$C_1$-$C_4$alkylamino, N,N-di-$C_1$-$C_4$alkylamino, N-$C_3$-$C_6$cycloalkylamino or N-$C_2$-$C_5$alknoylamino, in free form or in salt form, and a compound of formula I in free form wherein Ph is o-fluorophenyl, $R_1$ is N-monomethylamino or amino and $R_2$ is hydrogen or methyl, or Ph is o-fluorophenyl, o-chlorophenyl or m-trifluoromethylphenyl, $R_1$ is N,N-dimethylamino and $R_2$ is hydrogen, or Ph is m-fluorophenyl, p-fluorophenyl, o-chlorophenyl, o-trifluoromethylphenyl, m-trifluoromethylphenyl or p-trifluoromethylphenyl, $R_1$ is N-monomethylamino and $R_2$ is hydrogen.

4. A compound of formula I according to claim 3 wherein, taking into consideration the proviso mentioned in claim 1, Ph is 2-halophenyl which, in addition, may be substituted in the 3-, 5- or 6-position by halogen, it being possible for halogen in each case to be halogen having an atonic number of up to and including 35, in free form or in salt form, and a compound of formula I in free form wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

5. A compound of formula I according to claim 3 wherein, taking into consideration the proviso mentioned in claim 1, Ph is 2- or 3-fluorophenyl, 2-chlorophenyl, 2,6-difluorophenyl or 2-$C_1$-$C_4$alkylphenyl, $R_1$ is N-mono-$C_1$-$C_4$alkylamino, N,N-di-$C_1$-$C_4$alkylamino or N-$C_3$-$C_6$cycloalkylamino and $R_2$ is hydrogen, $C_1$-$C_4$alkyl or amino, in free form or in salt form, and a compound of formula I in free form, wherein Ph is o-fluorophenyl, $R_1$ is N-

monomethylamino and $R_2$ is hydrogen or methyl, or Ph is o-fluorophenyl or o-chlorophenyl, $R_1$ is N,N-dimethylamino and $R_2$ is hydrogen, or Ph is m-fluorophenyl or o-chlorophenyl, $R_1$ is N-monomethylanino and $R_2$ is hydrogen.

6. A compound of formula I according to claim 4 wherein, taking into consideration the proviso mentioned in claim 1, Ph is 2-fluorophenyl, which in addition may be substituted in the 5- or 6-position by halogen having an atomic number of up to and including 35, $R_1$ is amino, N-mono-$C_1$-$C_4$alkylamino, N,N-di-$C_1$-$C_4$alkylamino or N-$C_3$-$C_6$cycloalkylamino and $R_2$ is hydrogen, $C_1$-$C_4$alkyl or amino, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

7. A compound of formula I according to claim 6 wherein, taking into consideration the proviso mentioned in claim 1, Ph is 2-fluoro- or 2,6-difluorophenyl, $R_1$ is amino, N-mono-$C_1$-$C_4$alkylamino or N,N-di-$C_1$-$C_4$alkylamino and $R_2$ is hydrogen or $C_1$-$C_4$alkyl, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

8. A compound of formula I according to claim 6 wherein, taking into consideration the proviso mentioned in claim 1, Ph is 2-fluorophenyl, $R_1$ is N-mono-$C_1$-$C_4$alkylamino or N,N-di-$C_1$-$C_4$alkylamino and $R_2$ is hydrogen or amino, in free form or in salt form, and a compound of formula I in free form, wherein either Ph is o-fluorophenyl, $R_1$ is N-monomethylamino and $R_2$ is hydrogen, or Ph is o-fluorophenyl, $R_1$ is N,N-dimethylamino and $R_2$ is hydrogen.

9. 3-(2-Fluorobenzyl)-7-(N-methylamino)-3H-1,2,3-triazolo[4,5-d]pyrimidine or a salt thereof.

10. 7-(N,N-Dimethylamino)-3-(2-fluorobenzyl)-3H-1,2,3-triazolo[4,5-d]pyrimidine, 3-(2,6-difluorobenzyl)-7-(N-methylamino)-3H-1,2,3-triazolo[4,5-d]pyrimidine or 3-(2-fluorobenzyl)-5-methyl-7-(N-methylamino)-3H-1,2,3-triazolo[4,5-d]pyrimidine, or a salt thereof in each case.

11. 3-(2-Chlorobenzyl)-7-(N-methylamino)-3H-1,2,3-triazolo[4,5-d]pyrimidine, 3-(2-chlorobenzyl)-7-(N,N-dimethylamino)-3H-1,2,3-triazolo[4,5-d]pyrimidine, 7-(N-methylamino)-3-(2-methylbenzyl)-3H-1,2,3-triazolo[4,5d]pyrimidine, 3-(3-fluorobenzyl)-7-(N-methylamino)-3H-1,2,3-triazolo[4,5-d]pyrimidine or 5-amino-7-(N,N-dimethylamino)-3-(2-fluorobenzyl)-3H-1,2,3-triazolo[4,5-d]pyrimidine, or a salt thereof in each case.

12. 7-Amino-3-(2-fluorobenzyl)-3H-1,2,3-triazolo[4,5d]pyrimidine, 7-amino-3-(2-fluorobenzyl)-5-methyl-3H-1,2,3-triazolo[4,5-d]pyrimidine, 7-(N-acetylamino)-3-(2-fluorobenzyl)-3H-1,2,3-triazolo[4,5-d]pyrimidine or 7-(N-acetyl-N-methylamino)-3-(2-fluorobenzyl)-3H-1,2,3-triazolo[4,5-d]pyrimidine, or a salt thereof in each case.

13. 3-(4-Fluorobenzyl)-7-(N-methylamino)-3H-1,2,3-triazolo[4,5-d]pyrimidine, 5-amino-3-(2-fluorobenzyl)-7-(N-methylamino)-3H-1,2,3-triazolo[4,5-d]pyrimidine, 7-(N-methylamino)-3-(3-trifluoromethylbenzyl)-3H-1,2,3-triazolo[4,5-d]pyrimidine, 7-(N,N-dimethylamino)-3-(3-trifluoromethylbenzyl)-3H-1,2,3-triazolo[4,5-d]pyrimidine, 5,7-bis(N,N-dimethylamino)-3-(2-fluorobenzyl)3H-1,2,3-triazolo[4,5-d]pyrimidine, 5-(N,N-dinethylamino)-3-(2-fluorobenzyl)-7-(N-methylamino)-3H-1,2,3-triazolo[4,5-d]pyrimidine, 7-(N-methylamino)-3-(2-trifluoromethylbenzyl)-3H-1,2,3-triazolo[4,5-d]pyrimidine, 7-(N-methylamino)-3-(4-trifluoromethylbenzyl)-3H-1,2, 3-triazolo[4,5-d]pyrimidine, 3-(2-cyanobenzyl)-7-(N-methylamino)-3H-1,2,3-triazolo[4,5-d]pyrimidine, 3-(3-cyanobenzyl)-7-(N-methylamino)-3H-1,2,3-triazolo[4,5-d]pyrimidine or 3-(4-cyanobenzyl)-7-(N-methylamino)-3H-1,2,3-triazolo[4,5-d] pyrimidine, or a salt thereof in each case.

14. A compound according to any one of claims 1 to 13 or a pharmaceutically acceptable salt thereof for use in a method for the prophylactic and/or therapeutic treatment of the animal or human body.

15. A compound according to any one of claims 2, 4, 6, 7, 9, 10 and 12 or a pharmaceutically acceptable salt thereof for use in a method for the prophylactic and/or therapeutic treatment of the animal or human body.

16. A compound according to any one of claims 1 to 13 for use as an anticonvulsive.

17. A compound according to any one of claims 2, 4, 6, 7, 9, 10 and 12 for use as an anticonvulsive.

18. A pharmaceutical preparation comprising as active ingredient a compound according to any one of claims 1 to 13 or a pharmaceutically acceptable salt thereof, optionally in addition to customary pharmaceutical adjuncts.

19. A pharmaceutical preparation comprising as active ingredient a compound according to any one of claims 2, 4, 6, 7, 9, 10 and 12 or a pharmaceutically acceptable salt thereof, optionally in addition to customary pharmaceutical adjuncts.

20. An anticonvulsively effective pharmaceutical preparation according to claim 18, characterised in that an anticonvulsive active ingredient is chosen.

21. An anticonvulsively effective pharmaceutical preparation according to claim 19, characterised in that an anticonvulsive active ingredient is chosen.

22. A process for the manufacture of a compound of formula I or a salt thereof according to any one of claims 1 to 13, characterised in that

a) in a compound of formula

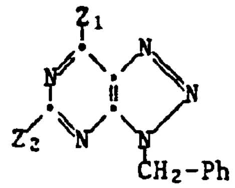

(II),

wherein $Z_1$ represents a nucleofugal leaving group $X_1$ and $Z_2$ represents a nucleofugal leaving group $X_2$ or a radical $R_2$, or wherein $Z_1$ is a radical $R_1$ and $Z_2$ represents a nucleofugal leaving group $X_2$, or in a tautomer and/or salt thereof, $X_1$ is converted into $R_1$ by reaction with a compound of formula $H\text{-}R_1$ (IIb) with the removal of a compound $X_1\text{-}H$, and/or $X_2$ is converted into $R_2$ by reaction with a compound of formula $H\text{-}R_2$ (IIj) with the removal of a compound $X_2\text{-}H$ , or
b) the compound Y-H is eliminated from a compound of formula

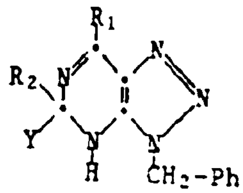

(III)

wherein Y represents hydroxy, mercapto or optionally aliphatically substituted amino, or from a tautomer and/or salt thereof, or
c) a salt of formula

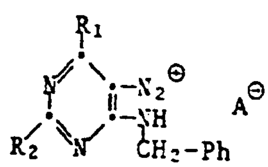

(V),

wherein A represents the anion of a protonic acid, is cyclised, or
d) a compound of formula

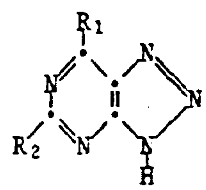

(VI)

or a salt thereof is reacted with a compound of formula $X_1\text{-}CH_2\text{-}Ph$ (VII), wherein $X_1$ represents a nucleofugal leaving group, and,
e) if desired, in each case an isomeric mixture that may be obtainable according to the process is separated into the components and the isomer of formula I is isolated and,
f) if desired, in each case a compound I obtainable according to the process or by another method is converted into a different compound I and,
g) if desired, in each case a free compound I obtainable according to the process is converted into a salt or a salt of a compound I obtainable according to the process is converted into the free compound I or into a different salt of compound I and,
h) if desired, in each case a stereoisomeric mixture that may be obtainable according to the process is separated into the stereoisomers and the desired stereoisomer is isolated.

23. A process for the manufacture of a compound of formula I wherein $R_2$ is amino, or a salt thereof, according to any one of claims 1 to 6, 8, 11 and 13, characterised in that

a) a compound of formula

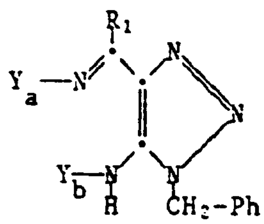

(IV)

wherein one of the radicals $Y_a$ and $Y_b$ is cyano and the other is hydrogen, or a tautomer and/or salt thereof, is cyclised and

b) if desired an isomeric mixture that may be obtainable according to the process is separated into the components and the isomer of formula I is isolated and

c) if desired a compound I obtainable according to the process is converted into a different compound I and

d) if desired a free compound I obtainable according to the process is converted into a salt or a salt of a compound I obtainable according to the process is converted into the free compound I or into a different salt of compound I and

e) if desired a stereoisomeric mixture that may be obtainable according to the process is separated into the stereoisomers and the desired stereoisomer is isolated.

24. The use of a compound according to any one of claims 1 to 13 or a pharmaceutically acceptable salt thereof for the manufacture of a pharmaceutical preparation.

25. The use of a compound according to claim 24 for the manufacture of an anticonvulsive.

**Claims for the following Contracting States: ES, GR**

1. A process for the manufacture of a compound of formula

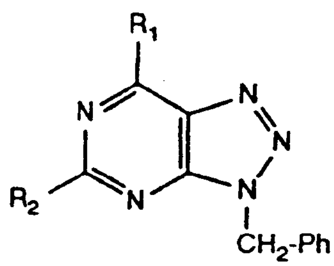

(I),

wherein Ph represents a phenyl radical substituted by halogen having an atomic number of up to and including 35, $C_1$-$C_7$alkyl, trifluoronethyl and/or by cyano, $R_1$ represents amino, N-mono-$C_1$-$C_7$alkylamino, N,N-di-$C_1$-$C_7$alkylamino wherein the two N-alkyl groups may be the same or different, N-($C_1$-$C_4$alkoxy-$C_1$-$C_7$alkyl)amino, N-(hydroxy-$C_1$-$C_7$alkyl)amino, N-(hydroxy-$C_1$-$C_7$alkyl)-N-$C_1$-$C_7$alkylamino, N-mono-$C_3$-$C_8$cycloalkylamino, N,N-di-$C_3$-$C_8$cycloalkylamino wherein the two N-cycloalkyl groups may be the sane or different, N-$C_3$-$C_8$cycloalkyl-N-$C_1$-$C_7$alkylamino, N-mono($C_3$-$C_8$cycloalkyl-$C_1$-$C_7$alkyl)amino, N,N-di($C_3$-$C_8$cycloalkyl-$C_1$-$C_7$alkyl)amino wherein the two N-cyclo-alkylalkyl groups may be the same or different, N-($C_3$-$C_8$cycloalkyl-$C_1$-$C_7$alkyl)-N-$C_1$-$C_7$alkylamino, N-$C_2$-$C_5$-alkanoylamino or N-$C_2$-$C_5$alkanoyl-N-$C_1$-$C_7$alkylamino and $R_2$ represents hydrogen, $C_1$-$C_7$alkyl, amino, N-nono-$C_1$-$C_7$alkylamino, N,N-di-$C_1$-$C_7$alkylanino wherein the two N-alkyl groups may be the same or different, N-($C_1$-$C_4$-alkoxy-$C_1$-$C_7$alkyl)amino, N-(hydroxy-$C_1$-$C_7$alkyl)amino, N-(hydroxy-$C_1$-$C_7$alkyl)-N-$C_1$-$C_7$alkylamino, N-nono-$C_3$-$C_8$cycloalkylamino, N,N-di-$C_3$-$C_8$cycloalkylanino wherein the two N-cycloalkyl groups may be the same or different, N-$C_3$-$C_8$cycloalkyl-N-$C_1$-$C_7$alkylamino, N-mono($C_3$-$C_8$ cycloalkyl-$C_1$-$C_7$alkyl)amino, N,N-di($C_3$-$C_8$cycloalkyl-$C_1$-$C_7$alkyl)amino wherein the two N-cycloalkylalkyl groups may be the same or different, N-($C_3$-$C_8$cycloalkyl-$C_1$-$C_7$alkyl)-N-$C_1$-$C_7$alkylamino, N-$C_2$-$C_5$alkanoylamino or N-$C_2$-$C_5$alkanoyl-N-$C_1$-$C_7$alkylamino, in free form or in salt form, with the proviso that in a compound of

formula I in free form in which $R_1$ represents N,N-di-$C_1$-$C_6$alkylamino wherein the two N-$C_1$-$C_6$alkyl groups are the same or different, N-monos-$C_1$-$C_6$alkylamino or amino, $R_2$ is other than hydrogen or $C_1$-$C_6$alkyl when Ph represents phenyl monosubstituted by halogen having an atomic number of up to and including 35 or by trifluoromethyl, and a compound of formula I in free form wherein Ph is o-fluorophenyl, $R_1$ is n-monomethylamino or amino and $R_2$ is hydrogen or methyl, or Ph is o-fluorophenyl, o-chlorophenyl or m-trifluoromethylphenyl, $R_1$ is N,N-dimethylamino and $R_2$ is hydrogen, or Ph is m-fluorophenyl, p-fluorophenyl, o-chlorophenyl, o-trifluoromethylphenyl, m-trifluoromethylphenyl or p-trifluoromethylphenyl, $R_1$ is N-monomethylamino and $R_2$ is hydrogen, or a salt thereof, characterised in that

a) in a compound of formula

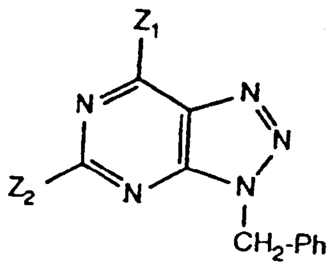

(II),

wherein $Z_1$ represents a nucleofugal leaving group $X_1$ and $Z_2$ represents a nucleofugal leaving group $X_2$ or a radical $R_2$, or wherein $Z_1$ is a radical $R_1$ and $Z_2$ represents a nucleofugal leaving group $X_2$, or in a tautomer and/or salt thereof, $X_1$ is converted into $R_1$ by reaction with a compound of formula H-$R_1$ (IIb) with the removal of a compound $X_1$-H, and/or $X_2$ is converted into $R_2$ by reaction with a compound of formula H-$R_2$ (IIj) with the removal of a compound $X_2$-H , or

b) the compound Y-H is eliminated from a compound of formula

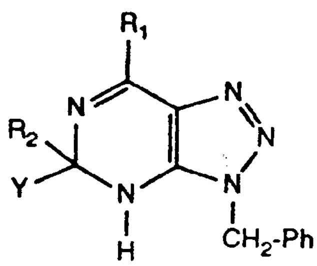

(III)

wherein Y represents hydroxy, mercapto or optionally aliphatically substituted amino, or from a tautomer and/or salt thereof, or

c) a salt of formula

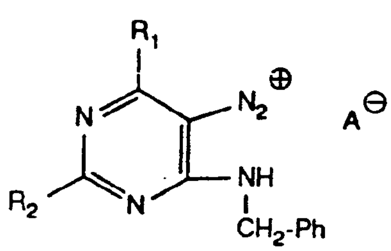

(V),

(V),

wherein A represents the anion of a protonic acid, is cyclised, or

d) a compound of formula

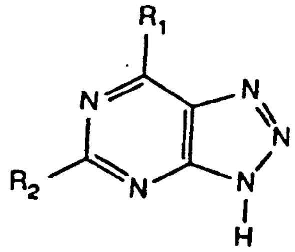

(VI)

or a salt thereof is reacted with a compound of formula $X_1$-$CH_2$-Ph (VII), wherein $X_1$ represents a nucleofugal leaving group, and

e) if desired, in each case an isomeric mixture that may be obtainable according to the process is separated into the components and the isomer of formula I is isolated and,

f) if desired, in each case a compound I obtainable according to the process or by another method is converted into a different compound I and,

g) if desired, in each case a free compound I obtainable according to the process is converted into a salt or a salt of a compound I obtainable according to the process is converted into the free compound I or into a different salt of compound I and,

h) if desired, in each case a stereoisomeric mixture that may be obtainable according to the process is separated into the stereoisomer and the desired stereoisomer is isolated.

2. A process according to claim 1 for the manufacture of a compound of formula I wherein, taking into consideration the proviso mentioned in claim 1, Ph represents a phenyl radical substituted by at least one halogen atom having an atomic number of up to and including 35, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

3. A process according to claim 1 for the manufacture of a compound of formula I wherein, taking into consideration the proviso mentioned in claim 1, Ph is 2-, 3- or 4-halophenyl, 2,3-, 2,5- or 2,6-dihalophenyl, 2-, 3- or 4-$C_1$-$C_4$alkylphenyl, 2-, 3- or 4-trifluoromethylphenyl or 2-, 3- or 4-cyanophenyl, it being possible for halogen in each case to be halogen having an atomic number of up to and including 35, $R_1$ is amino, N-mono-$C_1$-$C_4$alkylamino, N,N-di-$C_1$-$C_4$alkylamino, N-$C_3$-$C_6$cycloalkylamino or N-$C_2$-$C_5$alkanoylamino, and $R_2$ is hydrogen, $C_1$-$C_4$alkyl, amino, N-mono-$C_1$-$C_4$alkylamino, N,N-di-$C_1$-$C_4$alkylamino, N-$C_3$-$C_6$cycloalkylamino or N-$C_2$-$C_5$alkanoylamino, in free form or in salt form, and a compound of formula I in free form wherein Ph is o-fluorophenyl, $R_1$ is N-monomethylamino or amino and $R_2$ is hydrogen or methyl, or Ph is o-fluorophenyl, o-chlorophenyl or m-trifluoromethylphenyl, $R_1$ is N,N-dimethylanino and $R_2$ is hydrogen, or Ph is m-fluorophenyl, p-fluorophenyl, o-chlorophenyl, o-trifluoromethylphenyl, m-trifluoromethylphenyl or p-trifluoromethylphenyl, $R_1$ is N-monomethylamino and $R_2$ is hydrogen.

4. A process according to claim 3 for the manufacture of a compound of formula I wherein, taking into consideration the proviso mentioned in claim 1, Ph is 2-halophenyl which, in addition, may be substituted in the 3-, 5- or 6-position by halogen, it being possible for halogen in each case to be halogen having an atomic number of up to and including 35, in free form or in salt form, and a compound of formula I in free form wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

5. A process according to claim 3 for the manufacture of a compound of formula I wherein, taking into consideration the proviso mentioned in claim 1, Ph is 2- or 3-fluorophenyl, 2-chlorophenyl, 2,6-difluorophenyl or 2-$C_1$-$C_4$alkylphenyl, $R_1$ is N-mono-$C_1$-$C_4$alkylamino, N,N-di-$C_1$-$C_4$alkylamino or N-$C_3$-$C_6$cycloalkylamino and $R_2$ is hydrogen, $C_1$-$C_4$alkyl or amino, in free form or in salt form, and a compound of formula I in free form, wherein Ph is o-fluorophenyl, $R_1$ is N-monomethylamino and $R_2$ is hydrogen or methyl, or Ph is o-fluorophenyl or o-chlorophenyl, $R_1$ is N,N-dimethylamino and $R_2$ is hydrogen, or Ph is m-fluorophenyl or o-chlorophenyl, $R_1$ is N-monomethylamino and $R_2$ is hydrogen.

6. A process according to claim 4 for the manufacture of a compound of formula I wherein, taking into consideration the proviso mentioned in claim 1, Ph is 2-fluorophenyl, which in addition may be substituted in the 5- or 6-position by halogen having an atomic number of up to and including 35, $R_1$ is amino, N-mono-$C_1$-$C_4$alkylamino, N,N-di-$C_1$-$C_4$alkylamino or N-$C_3$-$C_6$cycloalkylamino and $R_2$ is hydrogen, $C_1$-$C_4$alkyl or amino, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

7. A process according to claim 6 for the manufacture of a compound of formula I wherein, taking into consideration the proviso mentioned in claim 1, Ph is 2-fluoro- or 2,6-difluorophenyl, $R_1$ is amino, N-mono-$C_1$-$C_4$alkylamino or N,N-di-$C_1$-$C_4$alkylamino and $R_2$ is hydrogen or $C_1$-$C_4$alkyl, in free form or in salt form, and a compound of formula I in free form, wherein either Ph is o-fluorophenyl, $R_1$ is N-monomethylamino or amino and $R_2$ is hydrogen or methyl, or Ph is o-fluorophenyl, $R_1$ is N,N-dimethylamino and $R_2$ is hydrogen.

8. A process according to claim 6 for the manufacture of a compound of formula I wherein, taking into consideration the proviso mentioned in claim 1, Ph is 2-fluorophenyl, $R_1$ is N-mono-$C_1$-$C_4$alkylamino or N,N-di-$C_1$-$C_4$alkylamino and $R_2$ is hydrogen or amino, in free form or in salt form, and a compound of formula I in free form, wherein either Ph is o-fluorophenyl, $R_1$ is N-monomethylamino and $R_2$ is hydrogen, or Ph is o-fluorophenyl, $R_1$ is N,N-dimethylamino and $R_2$ is hydrogen.

9. A process for the manufacture of a compound of formula (I) according to any one of claims 1 to 8 wherein $R_2$ represents amino, or a salt thereof characterised in that

a) a compound of formula

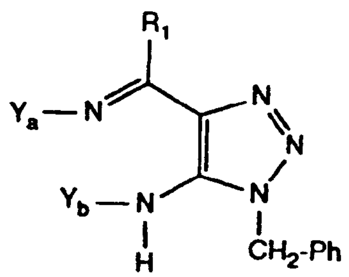

(IV)

wherein one of the radicals $Y_a$ and $Y_b$ is cyano and the other is hydrogen, or a tautomer and/or salt thereof, is cyclised and,

b) if desired, in each case an isomeric mixture that may be obtainable according to the process is separated into the components and the isomer of formula I is isolated and,

c) if desired, in each case a compound I obtainable according to the process or in some other manner is converted into a different compound I and,

d) if desired, in each case a free compound I obtainable according to the process is converted into a salt or a salt of a compound I obtainable according to the process is converted into the free compound I or into a different salt of compound I and,

e) if desired, in each case a stereoisomeric mixture that may be obtainable according to the process is separated into the stereoisomers and the desired stereoisomer is isolated.

10. A process according to claim 1 or 9 for the manufacture of 3-(2-fluorobenzyl)-7-(N-methylamino)-3H-1,2,3-triazolo[4,5-d]pyrimidine or a salt thereof.

11. A process according to claim 1 or 9 for the manufacture of 7-(N,N-dimethylamino)-3-(2-fluorobenzyl)-3H-1,2,3-triazolo[4,5-d]pyrimidine, 3-(2,6-difluorobenzyl)-7-(N-methylamino)-3H-1,2,3-triazolo[4,5-d]pyrimidine, 3-(2-fluorobenzyl)-5-methyl-7-(N-methylamino)-3H-1,2,3-triazolo[4,5-d]pyrimidine, or a salt thereof in each case.

12. A process according to claim 1 or 9 for the manufacture of 3-(2-chlorobenzyl)-7-(N-methylamino)-3H-1,2,3-triazolo[4,5-d]pyrimidine, 3-(2-chlorobenzyl)-7-(N,N-dimethylamino)-3H-1,2,3-triazolo[4,5-d]pyrimidine, 7-(N-methylamino)-3-(2-methylbenzyl)-3H-1,2,3-triazolo[4,5-d]pyrimidine, 3-(3-fluorobenzyl)-7-(N-methylamino)-3H-1,2,3-triazolo[4,5-d]pyrimidine or 5-amino-7-(N,N-dimethylamino)-3-(2-fluorobenzyl)-3H-1,2,3-triazolo[4,5-d]pyrimidine, or a salt thereof in each case.

13. A process according to claim 1 or 9 for the manufacture of 7-amino-3-(2-fluorobenzyl)-3H-1,2,3-triazolo[4,5-d]pyrimidine, 7-amino-3-(2-fluorobenzyl)-5-methyl-3H-1,2,3-triazolo[4,5-d]pyrimidine, 7-(N-acetylamino)-3-(2-fluorobenzyl)-3H-1,2,3-triazolo[4,5-d]pyrimidine or 7-(N-acetyl-N-methylamino)-3-(2-fluorobenzyl)-3H-1,2,3-triazolo[4,5-d]pyrimidine, or a salt thereof in each case.

14. A process according to claim 1 or 9 for the manufacture of 3-(4-fluorobenzyl)-7-(N-methylamino)-3H-1,2,3-triazolo[4,5-d]pyrimidine, 5-amino-3-(2-fluorobenzyl)-7-(N-methylamino)-3H-1,2,3-triazolo[4,5-d]-pyrimidine, 7-(N-methylamino)-3-(3-trifluoromethylbenzyl)-3H-1,2,3-triazolo[4,5-d]pyrimidine, 7-(N,N-dimethylamino)-3-(3-trifluoromethylbenzyl)-3H-1,2,3-triazolo[4,5-d]pyrimidine, 5,7-bis(N,N-dimethylamino)-3-(2-fluorobenzyl) 3H-1,2,3-triazolo[4,5-d]pyrimidine, 5-(N,N-dimethylamino)-3-(2-fluorobenzyl)-7-(N-methylanino)-3H-1,2,3-triazolo[4,5-d]pyrimidine, 7-(N-methylamino)-3-(2-trifluoromethylbenzyl)-3H-1,2,3-triazolo[4,5-d]pyrimidine, 7-(N-methylamino)-3-(4-trifluoromethylbenzyl)3H-1,2,3-triazolo[4,5-d]pyrimidine, 3-(2-cyanobenzyl)-7-(N-methylamino)-3H-1,2,3-triazolo[4,5-d]pyrimidine, 3-(3-cyanobenzyl)-7-(N-methylamino)-3H-1,2,3-triazolo[4,5-d]pyrimidine, 3-(4-cyanobenzyl)-7-(N-methylamino)-3H-1,2,3-triazolo[4,5-d]pyrimidine, or a salt thereof in each case.

15. A process for the manufacture of a pharmaceutical preparation, characterised in that a compound

obtainable according to any one of claims 1 to 14, or a pharmaceutically acceptable salt thereof, is mixed with customary pharmaceutical adjuncts.

16. A process for the manufacture of a pharmaceutical preparation, characterised in that a compound obtainable according to any one of claims 2, 4, 6, 7, 9, 10 and 12, or a pharmaceutically acceptable salt thereof, is mixed with customary pharmaceutical adjuncts.

17. The use of a compound obtainable according to any one of claims 1 to 14, or of a pharmaceutically acceptable salt thereof, for the manufacture of a pharmaceutical preparation.

18. The use of a compound according to claim 17 for the manufacture of an anticonvulsive.

## Revendications

### Revendications pour les Etats contractants suivants: AT, BE, CH, DE, FR, GB, IT, LI, LU, NL, SE

1. Composé de formule :

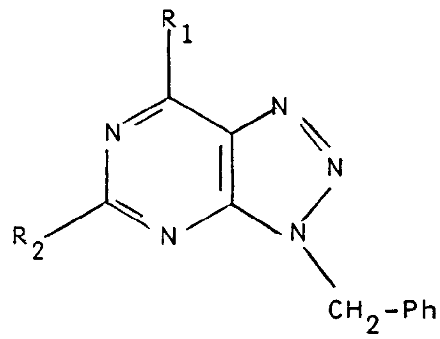

(I)

où Ph représente un reste phényle substitué par un halogène ayant un numéro atomique allant jusqu'à 35, par un alkyle en $C_1$-$C_7$, par le trifluorométhyle et/ou par le cyano, $R_1$ représente un amino, un N-monoalkyle en $C_1$-$C_7$ amino, un N,N-dialkyle en $C_1$-$C_7$ amino, les deux groupes N-alkyle pouvant être identiques ou différents, un N-(alcoxy en $C_1$-$C_4$ alkyle en $C_1$-$C_7$) amino, un N-(hydroxyalkyle en $C_1$-$C_7$)amino, un N-(hydroxyalkyle en $C_1$-$C_7$)-N-alkyle en $C_1$-$C_7$ amino, un N-monocycloalkyle en $C_3$-$C_8$ amino, un N,N-dicycloalkyle en $C_3$-$C_8$ amino, les deux groupes N-cycloalkyle pouvant être identiques ou différents, un N-cycloalkyle en $C_3$-$C_8$ N-alkyle en $C_1$-$C_7$ amino, un N-mono(cycloalkyle en $C_3$-$C_8$ alkyle en $C_1$-$C_7$)amino, un N,N-di(cycloalkyle en $C_3$-$C_8$ alkyle en $C_1$-$C_7$)amino, les deux groupes N-cycloalkylalkyle pouvant être identiques ou différents, un N-(cycloalkyle en $C_3$-$C_8$ alkyle en $C_1$-$C_7$)-N-alkyle en $C_1$-$C_7$ amino, un N-alcanoyle en $C_2$-$C_5$ amino ou un N-alcanoyle en $C_2$-$C_5$ N-alkyle en $C_1$-$C_7$ amino et $R_2$ représente l'hydrogène, un alkyle en $C_1$-$C_7$, un amino, un N-monoalkyle en $C_1$-$C_7$ amino, un N,N-dialkyle en $C_1$-$C_7$ amino, les deux groupes N-alkyle pouvant être identiques ou différents, un N-(alcoxy en $C_1$-$C_4$ alkyle en $C_1$-$C_7$) amino, un N-(hydroxyalkyle en $C_1$-$C_7$) amino, un N-(hydroxyalkyle en $C_1$-$C_7$)-N-alkyle en $C_1$-$C_7$ amino, un N-monocycloalkyle en $C_3$-$C_8$ amino, un N,N-dicycloalkyle en $C_3$-$C_8$ amino, les deux groupes N-cycloalkyle pouvant être identiques ou différents, un N-cycloalkyle en $C_3$-$C_8$ N-alkyle en $C_1$-$C_7$ amino, un N-mono(cycloalkyle en $C_3$-$C_8$ alkyle en $C_1$-$C_7$)amino, un N,N-di(cycloalkyle en $C_3$-$C_8$ alkyle en $C_1$-$C_7$)amino, les deux groupes N-cycloalkylalkyle pouvant être identiques ou différents, un N-(cycloalkyle en $C_3$-$C_8$ alkyle en $C_1$-$C_7$)-N-alkyle en $C_1$-$C_7$ amino, un N-alcanoyle en $C_2$-$C_5$ amino ou un N-alcanoyle en $C_2$-$C_5$ N-alkyle en $C_1$-$C_7$ amino, sous forme libre ou sous forme de sel, sous réserve que dans un composé de formule I sous forme libre où $R_1$ représente un N,N-dialkyle en $C_1$-$C_6$ amino, les deux groupes N-alkyles en $C_1$-$C_6$ étant identiques ou différents, un N-monoalkyle en $C_1$-$C_6$ amino ou un amino, $R_2$ est différent de l'hydrogène et d'un alkyle en $C_1$-$C_6$, lorsque Ph représente un phényle simplement substitué par un halogène ayant un numéro atomique allant jusqu'à 35 ou par le trifluorométhyle, ainsi qu'un composé de formule I sous forme libre où Ph est l'o-fluorophényle, $R_1$ le N-monométhylamino ou un amino et $R_2$ l'hydrogène ou le méthyle ou où Ph est l'o-fluorophényle, l'o-chlorophényle ou le m-trifluorométhylphényle, $R_1$ est le N,N-diméthylamino et $R_2$ est l'hydrogène ou où Ph est le m-fluorophényle, le p-fluorophényle, l'o-chlorophényle, l'o-trifluorométhylphényle, le m-trifluorométhylphényle ou le p-trifluorométhylphényle, $R_1$ est le N-monométhylamino et $R_2$ est l'hydrogène.

2. Composé de formule I selon la revendication 1 où, en tenant compte de la réserve mentionnée dans la revendication 1, Ph représente un reste phényle substitué par au moins un atome d'halogène ayant un numéro atomique allant jusqu'à 35, sous forme libre ou sous forme de sel, ainsi qu'un composé de formule I sous forme

libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamino et $R_2$ est l'hydrogène.

3. Composé de formule I selon la revendication 1 où, en tenant compte de la réserve mentionnée dans la revendication 1, Ph représente un 2-, 3- ou 4-halogénophényle, un 2,3-, 2,5- ou 2,6-dihalogénophényle, un 2-, 3- ou 4-alkyle en $C_1$-$C_4$ phényle, le 2-, 3- ou 4-trifluorométhylphényle ou le 2-, 3- ou 4-cyanophényle, l'halogène pouvant être un halogène ayant un numéro atomique pouvant aller jusqu'à 35, $R_1$ représente un amino, un N-monoalkyle en $C_1$-$C_4$ amino, un N,N-dialkyle en $C_1$-$C_4$ amino, un N-cycloalkyle en $C_3$-$C_6$ amino ou un N-alcanoyle en $C_2$-$C_5$ amino et $R_2$ représente l'hydrogène, un alkyle en $C_1$-$C_4$, un amino, un N-monoalkyle en $C_1$-$C_4$ amino, un N,N-dialkyle en $C_1$-$C_4$ amino, un N-cycloalkyle en $C_3$-$C_6$ amino ou un N-alcanoyle en $C_2$-$C_5$ amino, sous forme libre ou sous forme de sel, ainsi qu'un composé de formule I sous forme libre, où ou Ph est l'o-fluorophényle, $R_1$ est le N-monométhylamino ou l'amino et $R_2$ est l'hydrogène ou le méthyle ou où Ph est l'o-fluorophényle, l'o-chlorophényle ou le m-trifluorométhylphényle, $R_1$ est le N,N-diméthylamino et $R_2$ est l'hydrogène ou où Ph est le m-fluorophényle, le p-fluorophényle, l'o-chlorophényle, l'o-trifluorométhylphényle, le m-trifluorométhylphényle ou le p-trifluorométhylphényle, $R_1$ est le N-monométhylamino et $R_2$ est l'hydrogène.

4. Composé de formule I selon la revendication 3 où, en tenant compte de la réserve mentionnée dans la revendication 1, Ph représente un 2-halogénophényle pouvant être, en plus substitué en position 3, 5 ou 6 par un halogène, l'halogène pouvant être un halogène ayant un numéro atomique allant jusqu'à 35, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamino et $R_2$ est l'hydrogène.

5. Composé de formule I selon la revendication 3 où, en tenant compte de la réserve mentionnée dans la revendication 1, Ph représente le 2- ou 3-fluorophényle, le 2-chlorophényle, le 2,6-difluorophényle ou un 2-alkyle en $C_1$- $C_4$ phényle, $R_1$ représente un N-monoalkyle en $C_1$-$C_4$ amino, un N,N-dialkyle en $C_1$-$C_4$ amino ou un N-cycloalkyle en $C_3$-$C_6$ amino et $R_2$ représente l'hydrogène, un alkyle en $C_1$-$C_4$ ou un amino, sous forme libre ou sous forme de sel, ainsi qu'un composé de formule I sous forme libre où ou Ph est l'o-fluorophényle, $R_1$ est le N-monométhylamino et $R_2$ est l'hydrogène ou le méthyle ou où Ph est l'o-fluorophényle ou l'o-chlorophényle, $R_1$ est le N,N-diméthylamino et $R_2$ est l'hydrogène ou où Ph est le m-fluorophényle ou l'o-chlorophényle, $R_1$ est le N-monométhylamino et $R_2$ est l'hydrogène.

6. Composé de formule I selon la revendication 4 où, en tenant compte de la réserve mentionnée dans la revendication 1, Ph est le 2-fluorophényle pouvant être substitué en plus en position 5 ou 6 par un halogène ayant un numéro atomique allant jusqu'à 35, $R_1$ représente un amino, un N-monoalkyle en $C_1$-$C_4$ amino, un N,N-dialkyle en $C_1$-$C_4$ amino ou un N-cycloalkyle en $C_3$-$C_6$ amino et $R_2$ représente l'hydrogène, un alkyle en $C_1$-$C_4$ ou un amino, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle, ou où Ph est l'o-fluorophényle, $R_1$ est le N,N-diméthylamino et $R_2$ est l'hydrogène.

7. Composé de formule I selon la revendication 6 où, en tenant compte de la réserve mentionnée dans la revendication 1, Ph représente le 2-fluoro- ou le 2,6-difluorophényle, $R_1$ représente l'amino, un N-monoalkyle en $C_1$-$C_4$ amino ou un N,N-dialkyle en $C_1$-$C_4$ amino et $R_2$ représente l'hydrogène ou un alkyle en $C_1$-$C_4$, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamino et $R_2$ est l'hydrogène.

8. Composé de formule I selon la revendication 6 où, en tenant compte de la réserve mentionnée dans la revendication 1, Ph représente le 2-fluorophényle, $R_1$ représente un N-monoalkyle en $C_1$-$C_4$ amino ou un N,N-dialkyle en $C_1$-$C_4$ amino et $R_2$ représente l'hydrogène ou un amino, sous forme libre ou sous forme de sel, ainsi qu'un composé de formule I sous forme libre, où ou Ph est l'o-fluorophényle,$R_1$ est le N-monométhylamino et $R_2$ est l'hydrogène ou où Ph est l'o-fluorophényle, $R_1$ est le N,N-diméthylamino et $R_2$ est l'hydrogène.

9. La 3-(2-fluorobenzyl)-7-(N-méthylamino)-3H-1,2,3-triazolo[4,5-d]pyrimidine ou l'un de ses sels.

10. La 7-(N,N-diméthylamino)-3-(2-fluorobenzyl)3H-1,2,3-triazolo[4,5-d]pyrimidine, la 3-(2,6-difluorobenzyl)-7-(N-méthylamino)-3H-1,2,3-triazolo[4,5-d]pyrimidine ou la 3-(2-fluorobenzyl)-5-méthyl-7-(N-méthylamino)-3H-1,2,3-triazolo [4,5-d]pyrimidine ou l'un de leurs sels.

11. La 3-(2-chlorobenzyl)-7-(N-méthylamino)3H-1,2,3-triazolo[4,5-d]pyrimidine, la 3-(2-chlorobenzyl)-7-(N,N-diméthylamino)-3H-1,2,3-triazolo[4,5-d] pyrimidine, la 7-(N-méthylamino)-3-(2-méthylbenzyl)-3H-1,2,3-triazolo[4,5-d]pyrimidine, la 3-(3-fluorobenzyl)7-(N-méthylamino)-3H-1,2,3-triazolo[4,5-d]pyrimidine ou la 5-amino-7-(N,N-diméthylamino)-3-(2-fluorobenzyl)3H-1,2,3-triazolo[4,5-d]pyrimidine ou l'un de leurs sels.

12. La 7-amino-3-(2-fluorobenzyl)-3H-1,2,3-triazolo[4,5-d]pyrimidine, la 7-amino-3-(2-fluorobenzyl)-5-méthyl-3H-1,2,3-triazolo[4,5-d]pyrimidine, la 7-(N-acétylamino)-3-(2-fluorobenzyl)-3H-1,2,3-triazolo[4,5-d]pyrimidine ou la 7-(N-acétyl-N-méthylamino)-3-(2-fluorobenzyl)-3H-1,2,3-triazolo[4,5-d]pyrimidine ou l'un-de leurs sels.

13. La 3-(4-fluorobenzyl)-7-(N-méthylamino)-3H-1,2,3-triazolo[4,5-d]pyrimidine, la 5-amino-3-(2-fluorobenzyl)-7-(N-méthylamino)-3H-1,2,3-triazolo[4,5-d]pyrimidine, la 7-(N-méthylamino)-3-(3-trifluorométhylbenzyl)-3H-1,2,3-triazolo[4,5-d]pyrimidine, la 7-(N,N-diméthylamino)-3-(3-trifluorométhylbenzyl)-3H-1,2,3-triazolo [4,5-d] pyrimidine, la 5,7-bis-(N,N-diméthylamino)-3-(2-fluorobenzyl)-3H-1,2,3-triazolo [4,5-d] pyrimidine, la 5-(N,N-diméthylamino)-3-(2-fluorobenzyl)-7-(N-méthylamino)-3H-1,2,3-triazolo[4,5-d]pyrimidine, la 7-(N-méthylamino)-3-(2-trifluorométhylbenzyl)-3H-1, 2,3-triazolo[4,5-d]pyrimidine, la 7-(N-méthylamino)-3-(4-trifluorométhylbenzyl)-3H-1,2,3-triazolo[4,5-d]pyrimidine, la 3-(2-cyanobenzyl)-7-(N-méthylamino)-3H-1,2,3-triazolo[4,5-d] pyrimidine, la 3-(3-cyanobenzyl)-7-(N-méthylamino)-3H-1,2,3-triazolo[4,5-d]pyrimidine ou la 3-(4-cyanobenzyl)-7-(N-méthylamino)-3H-1,2,3-triazolo[4,5-d) pyrimidine ou l'un de leurs sels.

14. Composé selon l'une des revendications 1 à 13 ou l'un de ses sels pharmaceutiquement utilisables destiné à être utilisé dans un procédé pour le traitemnet prophylactique et/ou thérapeutique du corps animal ou humain.

15. Composé selon l'une des revendications 2, 4, 6, 7, 9, 10 et 12 ou l'un de ses sels pharmaceutiquement utilisables destiné à être utilisé dans un procédé pour le traitement prophylactique et/ou thérapeutique du corps animal ou humain.

16. Composé selon l'une des revendications 1 à 13 destiné à être utilisé comme anticonvulsivant.

17. Composé selon l'une des revendications 2, 4, 6, 7, 9, 10 et 12 destiné à être utilisé comme anticonvulsivant.

18. Préparation pharmaceutique contenant, comme substance active, un composé selon l'une des revendications 1 à 13 ou l'un de ses sels pharmaceutiquement utilisables, éventuellement à côté d'adjuvants pharmaceutiques usuels.

19. Préparation pharmaceutique contenant, comme substance active, un composé selon l'une des revendications 2, 4, 6, 7, 9, 10 et 12 ou l'un de ses sels pharmaceutiquement utilisables, éventuellement à côté d'adjuvants pharmaceutiques usuels.

20. Préparation pharmaceutique ayant une action anticonvulsivante selon la revendication 18, caractérisée en ce que l'on choisit une substance active anticonvulsivante.

21. Préparation pharmaceutique ayant une action anticonvulsivante selon la revendication 19, caractérisée en ce que l'on choisit une substance active anticonvulsivante.

22. Procédé pour la préparation d'un composé de formule I ou de l'un de ses sels selon l'une des revendications 1 à 13, caractérisé

a) en ce que dans un composé de formule

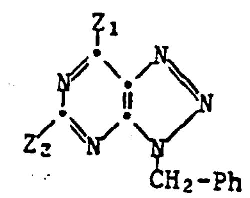

(II)

où $Z_1$ est un groupe partant nucléofuge $X_1$ et $Z_2$ représente un groupe partant nucléofuge $X_2$ ou un reste $R_2$ ou où $Z_1$ est un reste $R_1$ et $Z_2$ représente un groupe partant nucléofuge $X_2$ ou dans l'un de ses tautomères et/ou dans l'un de ses sels, on transforme $X_1$ en $R_1$ par réaction avec un composé de formule $H-R_1$ (IIb) avec élimination d'un composé $X_1$-H et/ou $X_2$ en $R_2$ par réaction avec un composé de formule $H-R_2$ (IIj) avec élimination d'un composé $X_2$-H ou

b) en ce que l'on élimine dans un composé de formule :

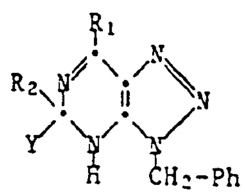

(III)

où Y représente l'hydroxy, un mercapto ou un amino éventuellement aliphatiquement substitué ou dans l'un de ses tautomères et/ou dans l'un de ses sels le composé Y-H ou

c) en ce que l'on cyclise un sel de formule:

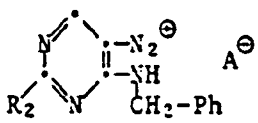

(V)

où A représente l'agrion d'un acide protonique ou

d) en ce que l'on fait réagir un composé de formule :

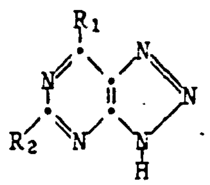

(VI)

ou l'un de ses sels avec un composé de formule $X_1$-$CH_2$-Ph (VII) où $X_1$ représente un groupe partant nucléofuge et,

e) si désiré, en ce que l'on sépare le mélange d'isomères éventuellement obtenu par le procédé conforme à l'invention en ses composants et en ce que l'on isole l'isomère de formule I et,

f) si désiré, en ce que l'on transforme le composé I pouvant être obtenu par le procédé conforme à l'invention ou d'une autre façon en un autre composé I et,

g) si désiré, en ce que l'on transforme le composé I libre pouvant être obtenu par le procédé conforme à l'invention en un sel ou un sel du composé I pouvant être obtenu par le procédé conforme à l'invention en un composé I libre ou en un autre sel du composé I et,

h) si désiré, en ce que l'on sépare le mélange de stéréoisomères éventuellement obtenu par le procédé conforme à l'invention en ses stéréoisomères et en ce que l'on isole le stéréoisomère désiré.

23. Procédé pour la préparation d'un composé de formule I où $R_2$ représente un amino ou de l'un de ses sels selon l'une des revendications 1 à 6, 8, 11 et 13, caractérisé :

a) en ce que l'on cyclise un composé de formule:

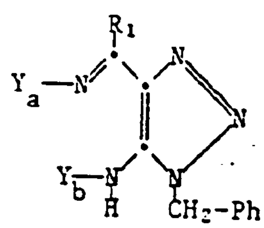

(IV)

où l'un des restes Ya et Yb représente le cyano et l'autre reste l'hydrogène ou l'un de ses tautomères et/ou l'un de ses sels et,

b) si désiré, en ce que l'on sépare le mélange d'isomères éventuellement obtenu par le procédé conforme à l'invention en ses composants et en ce que l'on isole l'isomère de formule I et,

c) si désiré, en ce que l'on transforme le composé I pouvant être obtenu par le procédé conforme à l'invention en un autre composé I et,

d) si désiré, en ce que l'on transforme le composé I libre pouvant être obtenu par le procédé conforme à l'invention en un sel ou le sel d'un composé I pouvant être obtenu par le procédé conforme à l'invention en un composé I libre ou en un autre sel du composé I, et

e) si désiré, en ce que l'on sépare le mélange de stéréoisomères éventuellement obtenu par le procédé conforme à l'invention en ses stéréoisomères et en ce quel'on isole le stéréoisomère désiré.

24. Utilisation d'un composé selon l'une des revendications 1 à 13 ou de l'un de ses sels pharmaceutiquement utilisables pour l'obtention d'une préparation pharmaceutique.

25. Utilisation d'un composé selon la revendication 24 pour la préparation d'un anticonvulsivant.

**Revendications pour les Etats contractants suivants : ES, GR**

1. Procédé pour la préparation d'un composé de formule :

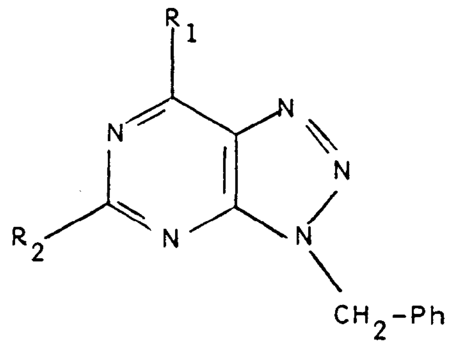

(I)

où Ph représente un reste phényle substitué par un halogène ayant un numéro atomique allant jusqu'à 35, par un alkyle en $C_1$-$C_7$, par le trifluorométhyle et/ou par le cyano, $R_1$ représente un amino, un N-monoalkyle en $C_1$-$C_7$ amino, un N,N-dialkyle en $C_1$-$C_7$ amino, les deux groupes N-alkyle pouvant être identiques ou différents, un N-(alcoxy en $C_1$-$C_4$ alkyle en $C_1$-$C_7$) amino, un N-(hydroxyalkyle en $C_1$-$C_7$)amino, un N-(hydroxyalkyle en $C_1$-$C_7$)-N-alkyle en $C_1$-$C_7$ amino, un N-monocycloalkyle en $C_3$-$C_8$ amino, un- N,N-dicycloalkyle en $C_3$-$C_8$ amino, les deux groupes N-cycloalkyle pouvant être identiques ou différents, un N-cycloalkyle en $C_3$-$C_8$ N-alkyle en $C_1$-$C_7$ amino, un N-mono(cycloalkyle en $C_3$ $C_8$ alkyle en $C_1$-$C_7$) amino, un N,N-di(cycloalkyle en $C_3$-$C_8$ alkyle en $C_1$-$C_7$)amino, les deux groupes N-cycloalkylalkyle pouvant être identiques ou différents, un N-(cycloalkyle en $C_3$-$C_8$ alkyle en $C_1$-$C_7$)-N-alkyle en $C_1$-$C_7$ amino, un N-alcanoyle en $C_2$-$C_5$ amino ou un N-alcanoyle en $C_2$-$C_5$ N-alkyle en $C_1$-$C_7$ amino et $R_2$ représente l'hydrogène, un alkyle en $C_1$-$C_7$, un amino, un N-monoalkyle en $C_1$-$C_7$ amino, un N,N-dialkyle en $C_1$-$C_7$ amino, les deux groupes N-alkyle pouvant être identiques ou différents, un N-(alcoxy en $C_1$-$C_4$ alkyle en $C_1$-$C_7$) amino, un N-(hydroxyalkyle en $C_1$-$C_7$) amino, un N-(hydroxyalkyle en $C_1$-$C_7$)-N-alkyle en $C_1$-$C_7$ amino, un N-monocycloalkyle en $C_3$-$C_8$ amino, un N,N-dicycloalkyle en $C_3$-$C_8$ amino, les deux groupes N-cycloalkyle pouvant être identiques ou différents, un N-cycloalkyle en $C_3$-$C_8$ N-alkyle en $C_1$-$C_7$ amino, un N-mono(cycloalkyle en $C_3$-$C_8$ alkyle en $C_1$-$C_7$)amino, un N,N-di(cycloalkyle en $C_3$-$C_8$ alkyle en $C_1$-$C_7$)amino, les deux groupes N-cycloalkylalkyle pouvant être identiques ou différents, un N-(cycloalkyle en $C_3$-$C_8$ alkyle en $C_1$-$C_7$)-N-alkyle en $C_1$-$C_7$ amino, un Nalcanoyle en $C_2$-$C_5$ amino ou un N-alcanoyle en $C_2$-$C_5$ N-alkyle en $C_1$-$C_7$ amino, sous forme libre ou sous forme de sel, sous réserve que dans un composé de formule I sous forme libre où $R_1$ représente un N,N-dialkyle en $C_1$-$C_6$ amino, les deux groupes N-alkyles en $C_1$-$C_6$ étant identiques ou différents, un N-monoalkyle en $C_1$-$C_6$ amino ou un amino, $R_2$ est différent de l'hydrogène et d'un alkyle en $C_1$-$C_6$, lorsque Ph représente un phényle simplement substitué par un halogène ayant un numéro atomique allant jusqu'à 35 ou par le trifluorométhyle, ainsi qu'un composé de formule I sous forme libre où Ph est l'o-fluorophényle, $R_1$ le N-monométhylamino ou un amino et $R_2$ l'hydrogène ou le méthyle ou où Ph est l'o-fluorophényle, l'o-chlorophényle ou le m-trifluorométhylphényle, $R_1$ est le N,N-diméthylamino et $R_2$ est l'hydrogène ou où Ph est le m-fluorophényle, le p-fluorophényle, l'o-chlorophényle, l'o-trifluorométhylphényle, le m-trifluorométhylphényle ou le p-trifluorométhylphényle, $R_1$ est le N-monométhylamino et $R_2$ est l'hydrogène, ou de l'un de ses sels, caractérisé

a) en ce que dans un composé de formule

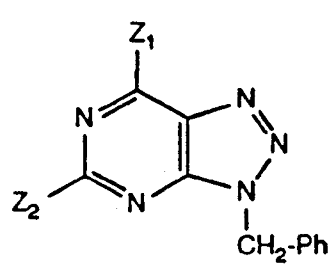

(II)

où $Z_1$ est un groupe partant nucléofuge $X_1$ et $Z_2$ représente un groupe partant nucléofuge $X_2$ ou un reste $R_2$ ou où $Z_1$ est un reste $R_1$ et $Z_2$ représente un groupe partant nucléofuge $X_2$ ou dans l'un de ses tautomères et/ou dans l'un de ses sels, on transforme $X_1$ en R $_1$ par réaction avec un composé de formule H-$R_1$ (IIb) avec élimination d'un composé $X_1$-H et/ou $X_2$ en $R_2$ par réaction avec un composé de formule H-$R_2$ (IIj) avec élimination d'un composé $X_2$-H ou

b) en ce que l'on élimine dans un composé de formule :

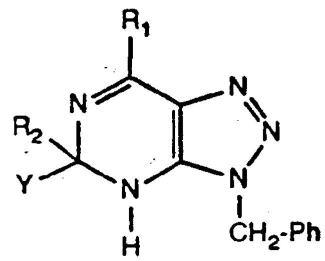

(III)

où Y représente l'hydroxy, un mercapto ou un amino éventuellement aliphatiquement substitué ou dans l'un de ses tautomères et/ou dans l'un de ses sels le composé Y-H ou

c) en ce que l'on cyclise un sel de formule

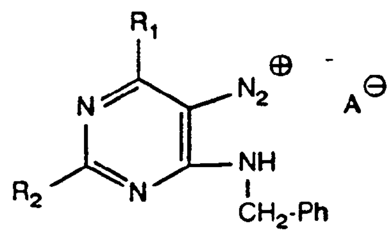

(V)

où A représente l'anion d'un acide protonique ou

d) en ce que l'on fait réagir un composé de formule :

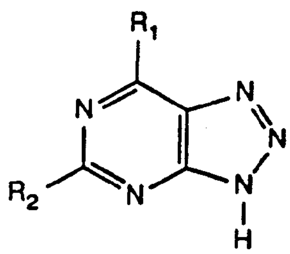

(VI)

ou l'un de ses sels avec un composé de formule $X_1$-$CH_2$-Ph (VII) où $X_1$, représente un groupe partant nucléofuge et,

e) si désiré, en ce que l'on sépare le mélange d'isomères, éventuellement obtenu par le procédé conforme à l'invention en ses composants et en ce que l'on isole l'isomère de formule I et,

f) si désiré, en ce que l'on transforme le composé I pouvant être obtenu par le procédé conforme à l'invention ou d'une autre façon en un autre composé I, et

g) si désiré, en ce que l'on transforme le composé I libre pouvant être obtenu par le procédé conforme à l'invention en un sel ou un sel du composé I pouvant être obtenu par le porcédé conforme à l'invention en un composé I libre ou en un autre sel du composé I, et

h) si désiré, en ce que l'on sépare le mélange de stéréoisomères éventuellement obtenu par le procédé conforme à l'invention en ses stéréoisomères et en ce que l'on isole le stéréoisomère désiré.

2. Procédé selon la revendication 1 pour la préparation d'un composé de formule I où, en tenant compte de la réserve mentionnée dans la revendication 1, Ph représente un reste phényle substitué par au moins un atome d'halogène, ayant un numéro atomique allant jusqu'à 35, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamino et $R_2$ est l'hydrogène.

3. Procédé selon la revendication 1 pour la préparation d'un composé de formule I où, en tenant compte de la réserve mentionnée dans la revendication 1, Ph représente un 2-, 3- ou 4-halogénophényle, un 2,3-, 2,5- ou 2,6-dihalogénophényle, un 2-, 3- ou 4-alkyle en $C_1$-$C_4$ phényle, le 2-, 3- ou 4-trifluorométhylphényle ou le 2-, 3- ou 4-cyanophényle, l'halogène pouvant être un halogène ayant un numéro atomique pouvant aller jusqu'à 35, $R_1$ représente un amino, un N-monoalkyle en $C_1$-$C_4$ amino, un N,N-di-alkyle en $C_1$-$C_4$ amino, un N-cyclo-

alkyle en $C_3$-$C_6$ amino ou un N-alcanoyle en $C_2$-$C_5$ amino et $R_2$ représente l'hydrogène, un alkyle en $C_1$-$C_4$, un amino, un N-monoalkyle en $C_1$-$C_4$ amino, un N,N-dialkyle en $C_1$-$C_4$ amino, un N-cycloalkyle en $C_3$-$C_6$ amino ou un N-alcanoyle en $C_2$-$C_5$ amino, sous forme libre ou sous forme de sel, ainsi qu'un composé de formule I sous forme libre, où ou Ph est l'o-fluorophényle, $R_1$ est le N-monométhylamlno ou l'amino et $R_2$ est l'hydrogène ou le méthyle ou où Ph est l'o-fluorophényle, l'o-chlorophényle ou le m-trifluorométhylphényle, $R_1$ est le N,N-diméthylamino et $R_2$ est l'hydrogène ou où Ph est le m-fluorophényle, le p-fluorophényle, l'o-chlorophényle, l'o-trifluorométhylphényle, le m-trifluorométhylphényle ou le p-trifluorométhylphényle, $R_1$ est le N-monométhylamino et $R_2$ est l'hydrogène.

4. Procédé selon la revendication 3 pour la préparation d'un composé de formule I où, en tenant compte de la réserve mentionnée dans la revendication 1, Ph représente un 2-halogénophényle pouvant être, en plus substitué en position 3, 5 ou 6 par un halogène, l'halogène pouvant être un halogène ayant un numéro atomique allant jusqu'à 35, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamlno et $R_2$ est l'hydrogène.

5. Procédé selon la revendication 3, pour la préparation d'un composé de formule I où en tenant compte de la réserve mentionnée dans la revendication 1, Ph représente le 2- ou 3-fluorophényle, le 2-chlorophényle, le 2,6-difluorophényle ou un 2-alkyle en $C_1$-$C_4$ phényle, $R_1$ représente un N-monoalkyle en $C_1$-$C_4$ amino,un N,N-dialkyle en $C_1$-$C_4$ amino ou un N-cycloalkyle en $C_3$-$C_6$ amino et $R_2$ représente l'hydrogène, un alkyle en $C_1$-$C_4$ ou un amino, sous forme libre ou sous forme de sel, ainsi qu'un composé de formule I sous forme libre où ou Ph est l'o-fluorophényle, $R_1$ est le N-monométhylamino et $R_2$ est l'hydrogène ou le méthyle ou où Ph est l'o-fluorophényle ou l'o-chlorophényle, $R_1$ est le N,N-diméthylamino et $R_2$ est l'hydrogène ou où Ph est le m-fluorophényle ou l'o-chlorophényle, $R_1$ est le N-monométhylamino et $R_2$ est l'hydrogène.

6. Procédé selon la revendication 4 pour la préparation d'un composé de formule I où, en tenant compte de la réserve mentionnée dans la revendication 1, Ph est le 2-fluorophényle pouvant être substitué en plus en positon 5 ou 6 par un halogène ayant un numéro atomique allant jusqu'à 35, $R_1$ représente un amino, un N-monoalkyle en $C_1$-$C_4$ amino, un N,N-dialkyle en $C_1$-$C_4$ amino ou un N-cycloalkyle en $C_3$-$C_6$ amino et $R_2$ représente l'hydrogène, un alkyle en $C_1$-$C_4$ ou un amino, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle, ou où Ph est l'o-fluorophényle, $R_1$ est le N,N-diméthylamino et $R_2$ est l'hydrogène.

7. Procédé selon la revendication 6 pour la préparation d'un composé de formule 1, où, en tenant compte de la réserve mentionnée dans la revendication 1, Ph est le 2-fluoro- ou le 2,6-difluorophényle, $R_1$ représente l'amino, un N-monoalkyle en $C_1$-$C_4$ amino ou un N,N-dialkyle en $C_1$-$C_4$ amino et $R_2$ représente l'hydrogène ou un alkyle en $C_1$-$C_4$, sous forme libre ou sous forme de sel, ainsi qu'un composé de formule I sous forme libre où ou Ph est l'o-fluorophényle, $R_1$ est le N-monométhylamino ou l'amino et $R_2$ est l'hydrogène ou le méthyle ou où Ph est l'o-fluorophényle, $R_1$ est le N,N-diméthylamino et $R_2$ est l'hydrogène.

8. Procédé selon la revendication 6 pour la préparation d'un composé de formule I où, en tenant compte de la réserve mentionnée dans la revendication 1, Ph représente le 2-fluorophényle, $R_1$ représente un N-monoalkyle en $C_1$-$C_4$ amino ou un N,N-dialkyle en $C_1$-$C_4$ amino et $R_2$ représente l'hydrogène ou un amino, sous forme libre ou sous forme de sel, ainsi qu'un composé de formule I sous forme libre, où ou Ph est l'o-fluorophényle, $R_1$ est le N-monométhylamino et $R_2$ est l'hydrogène ou où Ph est l'o-fluorophényle, $R_1$ est le N,N-diméthylamino et $R_2$ est l'hydrogène.

9. Procédé pour la préparation d'un composé de formule I selon l'une des revendications 1 à 8 où $R_2$ représente un amino ou de l'un de ses sels, caractérisé

a) en ce que l'on cyclise un composé de formule :

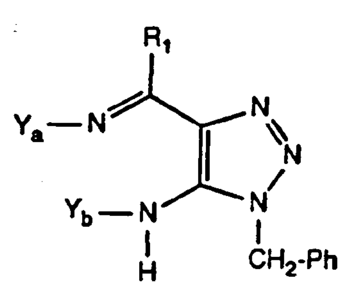

(IV)

où l'un des restes Ya et Yb représente le cyano et l'autre reste l'hydrogène ou l'un de ses tautomères et/ou l'un de ses sels et,

b) si désiré, en ce que l'on sépare le mélange d'isomères éventuellement obtenu par le procédé conforme à l'invention en ses composants et en ce que l'on isole l'isomère de formule I et,

c) en ce que, si désiré, on transforme le composé I pouvant être obtenu par le procédé conforme à l'invention ou d'une autre façon en un autre composé I et,

d) si désiré, en ce que l'on transforme le composé I libre pouvant être obtenu par le procédé conforme à l'invention en un sel ou le sel d'un composé I pouvant être obtenu par le procédé conforme à l'invention en un composé I libre ou en un autre sel du composé I et

e) si désiré, en ce que l'on sépare le mélange de stéréoisomères éventuellement obtenu par le procédé conforme à l'invention en ses stéréoisomères et en ce que l'on isole le stéréoisomère désiré.

10. Procédé selon la revendication 1 ou 9 pour la préparation de la 3-(2-fluorobenzyl)-7-(N-méthylamino)-3H-1,2,3-triazolo[4,5-d]pyrimidine ou de l'un de ses sels.

11. Procédé selon la revendication 1 ou 9 pour la préparation de la 7-(N,N-diméthylamino)-3-(2-fluorobenzyl)-3H-1,2,3-triazolo[4,5-d]pyrimidine, de la 3-(2,6-difluorobenzyl)-7-(N-méthylamino)-3H-1,2,3-tiiazolo[4,5-d]pyrimidine, de la 3-(2-fluorobenzyl)-5-méthyl-7-(N-méthylamino)-3H-1,2,3-triazolo[4,5-d]pyrimidine ou de l'un de leurs sels.

12. Procédé selon la revendication 1 ou 9 pour la préparation de la 3-(2-chlorobenzyl)-7-(N-méthylamino)-3H-1,2,3-triazolo[4,5-d]pyrimidine, de la 3-(2-chlorobenzyl)-7-(N,N-diméthylamino)-3H-1,2,3-triazolo[4,5-d]pyrimidlne, de la 7-(N-méthylamino)-3-(2-méthylbenzyl)-3H-1,2,3-triazolo[4,5-d]pyrimidine, de la 3-(3-fluorobenzyl)-7-(N-méthylamino)-3H-1,2,3-triazolo [4,5-d]pyrimidine ou de la 5-amino-7-(N,N-diméthylamino)-3-(2-fluorobenzyl)-3H-1,2,3-triazolo[4,5-d] pyrimidine ou de l'un de leurs sels.

13. Procédé selon la revendication 1 ou 9 pour la préparation de la 7-amino-3-(2-fluorobenzyl)-3H-1,2,3-triazolo[4,5-d]pyrimidine, de la 7-amino-3-(2-fluorobenzyl)-5-méthyl-3H-1,2,3-triazolo[4,5-d]pyrimidine, de la 7-(N-acétylamino)-3-(2-fluorobenzyl)-3H-1,2,3-triazolo[4,5-d]pyrimidine ou de la 7-(N-acétyl-N-méthylamino)-3-(2-fluorobenzyl)-3H-1,2,3-triazolo[4,5-d] pyrimidine ou de l'un de leurs sels.

14. Procédé selon la revendication 1 ou 9 pour la préparation de la 3-(4-fluorobenzyl)-7-(N-méthylamino)-3H-1,2,3-triazolo[4,5-d]pyrimidine, de la 5-amino-3-(2-fluorobenzyl)-7-(N-méthylamino)-3H-1,2,3-triazolo[4,5-d]pyrimidine, de la 7-(N-méthylamino)-3-(3-trifluorométhylbenzyl)-3H-1,2,3-triazolo[4,5-d]pyrimidine, de la 7-(N,N-diméthylamino)-3-(3-trifluorométhylbenzyl)-3H-1,2,3-triazolo[4,5-d]pyrimidine, de la 5,7-bis-(N,N-diméthylamino)-3-(2-fluorobenzyl)-3H-1,2,3-triazolo[4,5-d]pyrimidine, de la 5-(N,N-diméthylamino)-3-(2-fluorobenzyl)-7-(N-méthylamino)-3H-1,2,3-triazolo[4,5-d]pyrimidine, de la 7-(méthylamino)-3-(2-trifluorométhylbenzyl)-3H-1,2,3-triazolo[4,5-d]pyrimidine, de la 7-(N-méthylamino)-3-(4-trifluorométhylbenzyl)-3H-1,2,3-triazolo[4,5-d]pyrimidine, de la 3-(2-cyanobenzyl)-7-(N-méthylamino)-3H-1,2,3-triazolo[4,5-d]pyrimidine, de la 3-(3-cyanobenzyl)-7-(N-méthylamino)-3H-1,2,3-triazolo[4,5-d]pyrimidine, de la 3-(4-cyanobenzyl)-7-(N-méthylamino)-3H-1,2,3-triazolo[4,5-d]pyrimidine ou de l'un de leurs sels.

15. Procédé pour l'obtention d'une préparation pharmaceutique, caractérisé en ce que l'on mélange un composé pouvant être obtenu conformément à l'une des revendications 1 à 14 ou l'un de ses sels pharmaceutiquement utilisables avec des adjuvants pharmaceutiques usuels.

16. Procédé pour l'obtention d'une préparation pharmaceutique, caractérisé en ce que l'on mélange un composé pouvant être obtenu conformément à l'une des revendications 2, 4, 6, 7, 9, 10 et 12 ou l'un de ses sels pharmaceutiquement utilisables avec des adjuvants pharmaceutiques usuels.

17. Utilisation d'un composé pouvant être obtenu conformément à l'une des revendications 1 à 14 ou de l'un de ses sels pharmaceutiquement utilisables pour l'obtention d'une préparation pharmaceutique.

18. Utilisation d'un composé selon la revendication 17 pour la préparation d'un anticonvulsivant.